# EUROPEAN PATENT SPECIFICATION

(11) **EP 1 565 473 B1**
(45) Date of publication and mention of the grant of the patent: **02.08.2006**
(21) Application number: 03780067.9
(22) Date of filing: 26.11.2003
(51) Int. Cl.: C07D 487/16, C07D 487/22, A61K 31/407, C07D 209/00, C07D 243/00

(54) **N,N-BRIDGED, NITROGEN-SUBSTITUTED CARBACYCLIC INDOLOCARBAZOLES AS PROTEIN KINASE INHIBITORS**
N,N'-VERBRÜCKTE, N-SUBSTITUIERTE CARBACYCLISCHE INDOLCARBAZOLE ALS PROTEIN-KINASE HEMMER
INDOLOCARBAZOLES CARBACYCLIQUES A PONT N,N ET SUBSTITUTION AZOTE, UTILES COMME INHIBITEURS DE PROTEINE KINASE

(30) Priority: 27.11.2002 DE 10255343
(43) Date of publication of application: 24.08.2005
(73) Proprietor: Nad AG, 80339 München (DE)
(72) Inventor: MONSE, Barbara, 85258 Weichs (DE); BRAXMEIER, Tobias, 01069 Dresden (DE); FERRAND, Sandrine, F-68330 Huningue (FR); GORDON, Sandra, 21742 Malmö (SE); KLAFKI, Hans, 91052 Erlangen (DE); LAHU, Gezim, 78467 Konstanz (DE); RODER, Hanno, 80997 München (DE); SAHAGUN-KRAUSE, Heidi, 80687 München (DE); SENECI, Pierfausto, 80803 München (DE); THILLAYE DU BOULLAY, Olivier, F-81600 Montans (FR)
(74) Representative: Schnappauf, Georg
(86) International application number: PCT/EP2003/013322
(87) International publication number: WO 2004/048384

(56) References cited:
- WO-A-00/01699
- WO-A-94/02488
- RILEY D A ET AL: "Synthesis of Novel Carbocyclic Analogues of Indolocarbazole Natural Products" TETRAHEDRON LETTERS, ELSEVIER SCIENCE PUBLISHERS, AMSTERDAM, NL, vol. 40, no. 20, 14 May 1999 (1999-05-14), pages 3929-3932, XP004163783 ISSN: 0040-4039
- MCCOMBIE S W ET AL: "INDOLOCARBAZOLES. 1. TOTAL SYNTHESIS AND PROTEIN KINASE INHIBITING CHARACTERISTICS OF COMPOUNDS RELATED TO K-252C" BIOORGANIC & MEDICINAL CHEMISTRY LETTERS, OXFORD, GB, vol. 3, no. 8, 1993, pages 1537-1542, XP008003693 ISSN: 0960-894X

## Description

### Field of the Invention

The present invention relates to novel protein kinase inhibitors with advantageous pharmaceutical properties, methods for their preparation, intermediates thereof and pharmaceutical compositions comprising the same, reagents containing the same, and methods of using the same as therapeutics, particularly in CNS diseases.

### Background of the Invention

Protein kinases constitute a large class of enzymes that catalyze the transfer of a phosphate group to a hydroxyl group located on a protein substrate. Aberrant regulation of protein kinases is now generally recognized to be critically involved in disorders of cell proliferation, aberrant cellular responses to stimuli, differentiation, and degeneration in bone diseases, metabolic diseases, inflammatory disorders, infectious diseases and diseases of the central nervous system. On the background of an ever increasing understanding of the function of protein kinases in physiology and disease inhibition of such enzymes promises to deliver more causal and thus more effective therapies.

In disorders related to receptor signaltransduction tyrosine kinases are well established pharmacological targets, especially in various tumor types, where somatic mutations are often causally oncogenic by virtue of inducing constitutive activity in the normally strictly controlled receptor kinase domains. Kinases of this class are exemplified by abl, kit, met, src, EGFR, various homologs of the ErbB receptor and FGFR, Fak, fes, Fyn, IGF-1R, Ins-R, Jak, Lck, Lyn, PDGFR, trk.

Ser/Thr-kinases are more frequently involved in intracellular regulation. Although usually not associated with disease by virtue of mutations (with the notable exception of raf-kinase in tumors), hyperactivities related to aberrant signalling render them attractive pharmacological targets as well. The class is exemplified by various members of the CDK family as central control points of the cell cycle, and members of signal transduction cascades, particularly of the extended MAP-kinase family, like raf kinase, MLKs, MEKKs, MEKs, ERKs, JNK, SAPK2 (p38), and GSK-3. The above all belong to the superfamily of the socalled "proline-directed" kinases due to their specificity for various Ser/Thr-Pro sequence motifs. By contrast, non-proline directed kinases, like PKA, PKC, CaMK, are transducing second messenger signals related to and regulating membrane- and ionic current activity.

While the function of the above kinases is now well-appreciated in tumor biology (e.g. CDKs or MAP-kinase pathway related to ras mutations), inflammatory (e.g. p38 in TNF signalling) or metabolic diseases (GSK3 in regulation of glucose availability), their role in CNS diseases is less established. However, it is increasingly appreciated that they play crucial roles in memory formation, neuronal degeneration related to chronic exposure to oxygen radicals, and derangements of the cytoskeleton in Alzheimer's disease (AD), Parkinson's disease (PD), stroke, and head trauma.

Aberrant protein phosphorylation is best documented in pathologies related to the microtubule- associated protein tau (tauopathies), which is an invariable feature of AD and most likely decisive for the development of the clinical symptoms of progressive dementia. ERK2 is a prime candidate kinase for performing the pathological hyperphosphorylation of tau (PHF-tau) due to its unique ability to phosphorylate_virtually all 17 available Ser/Thr-Pro sites of tau [Roder, H.M. and Ingram, V.M., *J. Neurosci.* 1991, *11,* 3325-3343; Drewes, G. et al., *EMBO J*., 1992, *11,* 2131-2138; Roder, H.M. et al., *BBRC,* 1993, *193,* 639-647]. Inhibition of ERK2 could thus prevent tau hyperphosphorylation [WO 00/01699] and its functional sequelae, i.e. destabilization of microtubules and disturbance of transport, and aggregation of hyperphosphorylated tau in form of PHF (neurofibrillary tangles).

Inhibitors of kinases provide novel therapies for disorders caused by the metabolic processes in which protein kinases are involved. Some potent and selective kinase inhibitors have already been identified both from natural sources and as results of synthetic efforts. The protein kinase inhibitors known in the prior art cover very diverse structures for example pyrimidines, indolinones, pyridinylimidazoles, aminopurines, flavonoids and glycosylated indolocarbazoles. These protein kinase inhibitors are described for example in Adams, J.L. and Lee, D., *Curr. Opin. Drug Disc. Dev.,* 1999, 2, 96-109, Stover, D.R et al., *Curr. Opin. Drug Disc. Dev.,* 1999, 2, 274-285, Dumas, J., *Exp. Opin. Ther. Pat.,* 2000, 11, 405-429, and Davies, S.P. et al., *Biochem. J.,* 2000, *351, 95-105.*

The group of glycosylated indolocarbazole kinase inhibitors has been the subject of intense scrutiny. Most of the prior art in this area has been derived from a natural product class exemplified by staurosporine and K252a. Use of a variety of compounds derived from these natural product precursors has been claimed in treatments of various forms of cancer and CNS disorders, e.g. WO 93/08809, WO 94/02488, WO 94/27982, WO 94/04541, WO 95/07911, EP 0 508 792. Several compounds from the class are under clinical evaluation for various types of cancer. One compound is in clinical phase III for PD. However, the structural versatility of these previously disclosed compound series has been limited due to the restrictions imposed by the merely semi-synthetic access from natural product starting materials.

More versatile are the synthetically accessible carbacycle derivatives disclosed in US 6,013,646. However, some of the pharmaceutical issues generally associated with compounds of the above type have not been addressed satisfactorily.

One object of the present invention is to provide compounds with protein kinase inhibitory activity.

A further object of the present invention is to provide compounds allowing full synthetic access to a large structural diversity as needed for cooptimization of potency and selectivity, especially in the target class of protein kinases.

Another object of the present invention is to provide compounds which are distinguished over prior art compounds by an exceptional ability to cross the blood/brain barrier (BBB), which renders them particularly suited for CNS indications.

It is a further object of the present invention to provide compounds that are suitable for being formulated as salts, improving solubility characteristics and providing easier means of administration. Salt formation can also be a convenient feature in chiral separations after forming salts with enantiomerically pure acids.

### Summary of the Invention

This invention relates to N,N-bridged, nitrogen-substituted carbacyclic indolocarbazole compounds. This invention also relates to N,N-bridged, nitrogen-substituted carbacyclic indolocarbazole compounds for inhibiting the activity of protein kinases. In further embodiments this invention relates to N,N-bridged, nitrogen-substituted carbacyclic indolocarbazole compounds for treating non-insulin dependent diabetes mellitus, acute stroke and other neurotraumatic injuries, for treating diabetes mellitus, as a chemotherapeutic for the treatment of various malignant diseases, for treating diseases caused by malfunctioning of specific signaling pathways, and for treating neumdegenerative diseases such as for example Alzheimer's disease.

### Brief Description of the Figures

**Fig. 1** shows the structures of compounds **Ia-h** of the present invention whose synthesis is described in detail below.
**Fig. 2** shows the synthesis of the dibromo intermediate **II** which has been used for the synthesis of the compounds of the general formula I.
**Fig. 3** shows the synthesis of the bis-halo maleimide intermediates **IVa,b** which have been used for the synthesis of the compounds of the general formula **I.**
**Fig. 4** shows an alternative detailed pathway for the synthesis of the key N-protected, symmetrical bridged indolocarbazole imide aglycones **VIIIa** which have been used for the synthesis of the compounds of the general formula I (R₈,R₉ and R₁₀,R₁₁ = carbonyl).
**Fig. 5** shows an alternative detailed pathway for the synthesis of the key N-protected, unsymmetrical bridged indolocarbazole imide aglycones **VIIIb** which have been used for the synthesis of the compounds of the general formula I (R₈,R₉ and R₁₀,R₁₁ = carbonyl).
**Fig. 6** shows an alternative detailed pathway for the synthesis of the key N-protected, bridged indolocarbazole lactam aglycones **XIIIa,b** which have been used for the synthesis of the compounds of the general formula I (only one of R₈R₉ and R₁₀,R₁₁= carbonyl).
**Fig. 7** shows an alternative detailed pathway for the synthesis of the key bridged indolocarbazole imide intermediates **XVa,b** and **XVIa,b** which have been used for the synthesis of the compounds of the general formula I (R₈,R₉ and R₁₀,R₁₁ = carbonyl).
**Fig. 8** shows an alternative detailed pathway for the synthesis of compounds Ia as examples for the preparation of the members of the general class of compounds depicted in the general formula I.
**Fig. 9** shows an alternative detailed pathway for the synthesis of compounds Ib as examples for the preparation of the members of the general class of compounds depicted in the general formula I.
**Fig. 10** shows an alternative detailed pathway for the synthesis of compounds **Ic/1-3** as examples for the preparation of the members of the general class of compounds depicted in the general formula I.
**Fig. 11** shows an alternative detailed pathway for the synthesis of compounds **Id** and **Id/1** as examples for the preparation of the members of the general class of compounds depicted in the general formula I.
**Fig. 12** shows an alternative detailed pathway for the synthesis of compounds **Ie** and **Ie/1** as examples for the preparation of the members of the general class of compounds depicted in the general formula I.
**Fig. 13** shows an alternative detailed pathway for the synthesis of compounds **Ie** and **Ie/2** as examples for the preparation of the members of the general class of compounds depicted in the general formula I.
**Fig. 14** shows an alternative detailed pathway for the synthesis of compounds **Ie/3** and **Ie/4** as examples for the preparation of the members of the general class of compounds depicted in the general formula I.
**Fig. 15** shows an alternative detailed pathway for the synthesis of compounds **Ie/5** and **Ie/6** as examples for the preparation of the members of the general class of compounds depicted in the general formula I.
**Fig. 16** shows an alternative detailed pathway for the synthesis of compounds **If** and **If/1** as examples for the preparation of the members of the general class of compounds depicted in the general formula I.
**Fig. 17** shows an alternative detailed pathway for the synthesis of the key bridged indolocarbazole lactam intermediates **XXVIa,b** and **XXVIIa-d** which have been used for the synthesis of the compounds of the general formula I (only one of R₈,R₉ and R₁₀,R₁₁ > = carbonyl).
**Fig. 18** shows an alternative detailed pathway for the synthesis of the key E- and Z-regioisomeric couples of keto bridged indolocarbazole lactam intermediates **XXVIIa,b** and **XXVIIc,d** which have been used for the synthesis of the compounds of the general formula I (only one of R₈,R₉ and R₁₀,R₁₁ = carbonyl).
**Fig.19** shows the structures of compounds **Ig/1-3** and **Ih/1-3** as examples for the preparation of the members of the general class of compounds depicted in the general formula I (only one of R₈, R₉ and R₁₀,R₁₁ = carbonyl).
**Fig. 20** shows brain and plasma concentration of NAD0241 (= compound of example 5) treated rats in comparison with the brain and plasma concentration of NAD0180 (comparative compound) treated rats after 1 hour post-administration. There is a significant difference between the concentrations of NAD0241 and NAD0180 in brain showing the ability of NAD0241 to pass more readily the BBB.
**Fig. 21** shows ratios between brain and plasma for NAD0241 and NAD0180 at the conditions of application. There is an over 30 fold difference in this ratio showing better kinetic properties of penetration for NAD0241 compared with NAD0180.
**Fig. 22** shows brain and plasma concentrations of NAD0241, NAD002 (= compound CII described in WO 00/01699) and K252a after 1 hour post-administration applied under conditions given in examples 56E and 56F. Significantly higher concentrations of NAD0241 compared with NAD002 were found in brain. There is also significant difference between the plasma levels of NAD002 and K252a compared to NAD0241.
**Fig. 23** shows brain/plasma ratios at 1 hour for NAD0241, NAD002 and K252a. Brain/plasma ratios of 1 are achieved under the conditions of application, resulting in lower central compartment exposure of equal or higher brain levels. Lower plasma levels of NAD0241 may point to better distribution in the body as well. Brain to plasma ratios also suggest that by determining the plasma levels at 1h the brain levels of NAD0241 are easily predicted which simplifies the design of therapeutic dosage regimen.
**Fig. 24** shows the solubility of NAD0241 (free base) and of NAD0241-hydrochloride salt in water/DMSO 99/1.

### Detailed Description of the Invention

The following definitions will be useful in describing the invention and will eliminate the need for repetitive explanations.

"Alkoxy" denotes unsubstitued and substituted straight-chain and branched chain aromatic or non aromatic alkoxy radicals derived from the removal of a single hydrogen atom from the hydroxyl group of a parent alcohol or phenol.

"Alkyl" denotes unsubstitued and substituted straight-chain and branched chain hydrocarbon radicals derived from the removal of a single hydrogen atom from a parent alkane.

"Alkylene" denotes unsubstitued and substituted straight-chain and branched chain hydrocarbon radicals derived from the removal of a hydrogen atom from two terminal carbon atoms of a parent alkane.

"Alkenyl" denotes unsubstitued and substituted straight-chain and branched chain hydrocarbon radicals derived from the removal of a single hydrogen atom from a parent alkene.

"Alkynyl" denotes unsubstitued and substituted straight-chain and branched chain hydrocarbon radicals derived from the removal of a single hydrogen atom from a parent alkyne.

"Aryl" denotes unsubstituted and substituted aromatic radicals derived from the removal of a single hydrogen atom from a parent aromatic ring system.

"Carbacycle" means 5 unsubstituted or substituted cyclic, non-aromatic hydrocarbon radicals derived from the removal of a single hydrogen atom from a parent cyclic compound, wherein the cyclic compound optionally comprises one or two carbon-carbon double bonds.

"Decoration" denotes the pattern of substitution which is especially useful for specific purposes such as to provide an increase of the inhibitory activity or to increase specificity towards a specific protein kinase.

"Decorating" denotes changing the substitution pattern in order to obtain compounds with the desired properties. Non-limiting examples for decorating e.g. of hydroxy, amino, carboxy, ester or amide function are acylation, oxidation, reduction (e.g. reductive amination) and alkylation.

"Heteroaryl" denotes unsubstituted and substituted radicals derived from the removal of a single hydrogen atom from a parent aromatic ring system in which one or more ring atoms are not carbon.

"Inhibitor" refers to a substance which, when added in a sufficient amount, is capable of reducing the catalytic activity of a given enzyme in at least one reaction which can be catalyzed by the said enzyme.

"Lower alkyl" denotes unsubstitued and substituted straight-chain and branched chain hydrocarbon, radicals derived from the removal of a single hydrogen atom from a parent alkane containing, 1-6 carbon atoms.

"Lower alkenyl" denotes unsubstitued and substituted straight-chain and branched chain hydrocarbon radicals derived from the removal of a single hydrogen atom from a parent alkene containing 2-6 carbon atoms.

"Lower alkynyl" denotes unsubstitued and substituted straight-chain and branched chain hydrocarbon radicals derived from the removal of a single hydrogen atom from a parent alkyne containing 2-6 carbon atoms.

"Substituted" as used herein refers to a molecule or a molecular residue, wherein one or more hydrogen atoms are replaced with one or more non-hydrogen atoms, functional groups or moieties including but not limited to alkyl, substituted alkyl, hydroxyl, thiol, alkylthiol, halogen (such as fluorine, chlorine, bromine, iodine), alkoxy, amino, substituted amino, amido, carboxyl, alkylcarboxylate, cycloalkyl, substituted cycloalkyl, heterocycle, cycloheteroalkyl, substituted cycloheteroalkyl, acyl, oxo, aryl, substituted aryl, aryloxy, heteroaryl, substituted heteroaryl, aralkyl, alkyl alkenyl, alkyl alkynyl, alkyl cycloalkyl, alkyl cycloheteroalkyl, and cyano.

The invention will now be explained in greater detail with reference to the preferred non-limiting embodiments of the invention, examples of which are illustrated in the accompanying drawings.

It has been found that molecules of the general formula I are very potent inhibitors of protein kinases. Surprisingly, their physico-chemical properties and their BBB penetration is significantly improved over prior art compounds; e.g. those described in WO 00/01699.

This invention relates to a class of compounds having a structure according to the general formula (I) 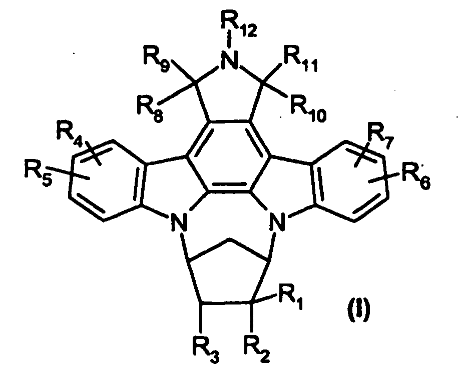 wherein
- R₁: is NR₁₃R₁₄ or may join together with R₂ to form an optionally substituted saturated or unsaturated N-heterocycle (e.g. spiro-hydantoyl) or may join together with R₃ to form an optionally substituted saturated or unsaturated N-heterocycle (e.g. oxazolin-2-one-4,5-diyl),
- R₂: is selected from the group consisting of H, lower alkyl, aryl, heteroaryl, CN, COR₁₃, COOR₁₃, CONHR₁₃, and CONR₁₃R₁₄;
- R₃: is selected from the group consisting of H, OR₁₃, OCOR₁₃, OCONHR₁₃, and OCONR₁₃R₁₄;
- R_{4,}R₅,R₆,R₇: taken alone can be the same or different and are each independently selected from the group consisting of H, halogen, lower alkyl, lower alkenyl, lower alkynyl, aryl or heteroaryl, CN, COR₁₃, COOR₁₃, CONHR₁₃, CONR₁₃R₁₄, CSR₁₃, CSSR₁₃, NR₁₃R₁₄, NHCOR₁₃, NHCOOR₁₃, NHSO₂R₁₃, N₃, OR₁₃, OCOR₁₃, SR₁₃, SO₂R₁₃, and SiR₁₅R₁₆R₁₇; wherein R₁₅, R₁₆ and R₁₇ can be the same or different and are independently selected from the group consisting of H, lower alkyl, aryl and heteroaryl;
- R₈,R₉: when taken alone they are both H, or one of them is H and the other is OH, or when taken together they are the oxygen atom of a carbonyl group or the sulfur atom of a thiocarbonyl group; and with the proviso that when R₁₀,R₁₁ are different from carbonyl R₈,R₉ taken together are the oxygen atom of a carbonyl group or the sulfur atom of a thiocarbonyl group;
- R₁₀,R₁₁: when taken alone they are both H, or one of them is H and the other is OH, or when taken together they are the oxygen atom of a carbonyl group or the sulfur atom of a thiocarbonyl group; and with the proviso that when R₈,R₉ are different from carbonyl R₁₀,R₁₁ taken together are the oxygen atom of a carbonyl group or the sulfur atom of a thiocarbonyl group;
- R₁₂: is selected from the group consisting of H, lower alkyl, cycloalkyl, optionally substituted benzyl, aryl, hetemaryl, COR₁₃, COOR₁₃, NR₁₃R₁₄, and OR₁₃,
and wherein
- R₁₃ and R₁₄: can be the same or different and are independently selected from the group consisting of H, lower alkyl, cycloalkyl, optionally substituted acyl, aryl, optionally substituted benzyl and heteroaryl rest; or may join together to form N₃ or an optionally substituted saturated or unsaturated N-heterocyle (e.g. morpholino, piperidinyl, optionally substituted triazolyl such as a group of the formula 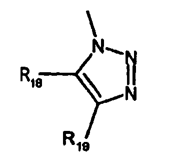
optionally substituted tetrazolyl such as a group of the formula 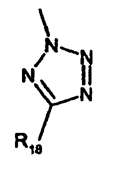 wherein R₁₈ and R₁₉ can be the same or different and are each independently selected from the group consisting of H, lower alkyl, COR₁₃, COOR₁₃, CONHR₁₃ and CONR₁₃R₁₄).

It will be appreciated by the person skilled in the art that the compounds of the present invention may contain one or more chiral centers, and may be isolated in optically active or racemic forms. Thus, all chiral, diastereomeric, racemic forms and all geometric isomeric forms of a structure are covered by the scope of the invention. Compounds falling within the scope of this invention also include the pharmaceutically acceptable salts of the above-identified compounds.

In a preferred embodiment the invention relates to a class of compounds having a structure according to the general formula (IA) 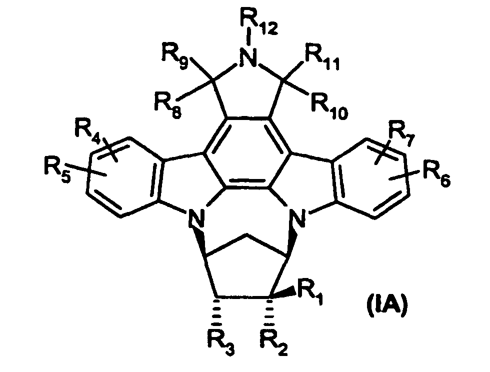 wherein R₁ to R₁₂ are as defined above for the general formula (I).

The compounds according to formulae (I) and (IA) of the present invention, herein named N,N-bridged, aminosubstituted carbacyclic indolocarbazoles, are very useful as kinase inhibitors, since their properties can be modulated by structural modifications to reach selectivity and potency against a variety of therapeutically relevant kinase targets as demonstrated by the results of Table 2. It has also been found that these compounds are a useful source of therapeutic agents for the specific and selective modulation of protein kinase activities in several disorders. More specifically, they are potent inhibitors of abnormal ERK2 activity which makes them useful for the treatment of neurodegenerative diseases, including Alzheimer's disease.

The preferred protein kinase inhibitors are those of the formulae I and IA in which
- R₁: is NR₁₃R₁₄;
- R₂: is selected from the group consisting of H, CN, COOR₁₃, CONHR₁₃, and CONR₁₃R₁₄;
- R₃: is selected from the group consisting of H and OH;
- R₄,R₅,R₆,R₇: taken alone can be the same or different and are each independently selected from the group consisting of H, CONHR₁₃, CONR₁₃R₁₄, NR₁₃R₁₄, NHCOR₁₃, NHCOOR₁₃, NHSO₂R₁₃, and OR₁₃;
- R₈,R₉: are both H, or one of them is H and the other is OH, or taken together they are the oxygen atom of a carbonyl group; and with the proviso that when R₁₀,R₁₁ are different from carbonyl R₈,R₉ taken together are the oxygen atom of a carbonyl group;
- R₁₀,R₁₁: are both H, or one of them is H and the other is OH, or taken together they are the oxygen atom of a carbonyl group; and with the proviso that when R₈,R₉ are different from carbonyl R₁₀,R₁₁ taken together are the oxygen atom of a carbonyl group;
- R₁₂: is selected from the group consisiting of H, substituted lower alkyl, NR₁₃R₁₄, and OR_{13,}
and wherein
- R₁₃ and R₁₄: can be the same or different and are independently selected from the group consisting of H and substituted lower alkyl.

The preferred compounds which show an improved in vivo activity against neurodegenerative diseases, including Alzheimer's disease are in accordance with the general formulae I and IA, wherein
- R₁: is NHR₁₃, wherein R₁₃ is selected from the group consisting of H and substituted lower alkyl;
- R₂: is selected from the group consisting of CN, COOR₁₃, and CONHR₁₃, wherein R₁₃ is selected from the group consisting of H and substituted lower alkyl;
- R₃: is selected from the group consisting ofH and OH;
- R₄,R₅,R₆,R₇: taken alone can be the same or different and are each independently selected from the group consisting of H, NHR₁₃, and OR₁₃, wherein R₁₃ is selected from the group consisting of H and substituted lower alkyl;
- R₈,R₉: are both H, or taken together they are the oxygen atom of a carbonyl group; with the proviso that when R₁₀,R₁₁ are different from carbonyl R₈,R₉ taken together are the oxygen atom of a carbonyl group.
- R₁₀,R₁₁: are both H, or taken together they are the oxygen atom of a carbonyl group; with the proviso that when R₈,R₉ are different from carbonyl R₁₀,R₁₁ taken together are the oxygen atom of a carbonyl group.
- R₁₂: is H.

In a further preferred embodiment the invention relates to a class of compounds having a structure according to the general formula (IB) 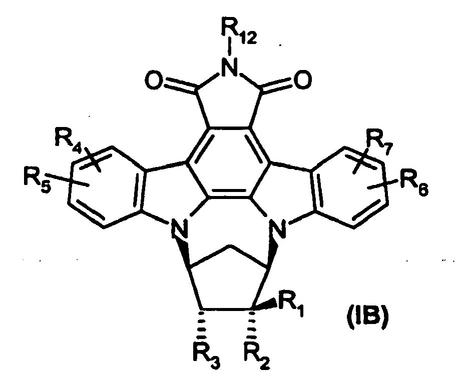 wherein R₁ to R₇ and R₁₂ are as defined above for the general formulae (I) and (IA).

Furthermore, the invention also relates to the use of the compounds according to the present invention for the preparation of a pharmaceutical composition for inhibiting the activity of one or more protein kinases. Preferably the protein kinase is selected from the group consisting of extracellular signal regulated kinase 2, protein kinase A, protein kinase C, glycogen synthase kinase 3β. In a further aspect the invention relates to the use of the compounds of the present invention for treating non-insulin dependent diabetes mellitus, acute stroke and other neurotraumatic injuries, for treating diabetes mellitus, as a chemotherapeutic for the treatment of various-malignant diseases, for treating diseases caused by malfunctioning of specific signaling pathways, and for treating neurodegenerative diseases such as for example Alzheimer's disease.

The treatment is accomplished using a therapeutically effective amount of at least one compound of this invention and/or a pharmaceutically acceptable salt thereof in admixture with a phannaceutically acceptable excipient.

A "therapeutically effective amount" relates to an amount which, either alone or in combination with additional doses, results in a desired reaction or physiological effect. Regarding treatment of a particular disease or condition, the desired reaction relates to the inhibition of the course of the disease comprising retardation, and particularly stop of the progress of the disease. The therapeutically effective amount can be chosen depending on the activity of the particular compound and other factors such as condition of the patient to be treated, severity of the disease, age, physiological condition, height and weight of the patient, duration of treatment and mode of administration.

Pharmaceutically acceptable salts comprise, but are not limited to, those salts which form with amino or carboxylate groups. Suitable acids for the preparation of acid addition salts are inorganic acids such as HCl, HBr, H₂SO₄, HNO₃, H₃PO₄, and organic acids such as acetic acid, propionic acid, oxalic acid, maleic acid, malonic acid, succinic acid, malic acid, fumaric acid, tartaric acid, citric acid, benzoic acid, cinnamic acid, methanesulfonic acid, p-toluenesulfonic acid and salicylic acid. Basic compounds which can form salts with carboxyl groups comprise, but are not limited to, NaOH, KOH, NH₃, Ca(OH)₂, isopropylamine, triethylamine, 2-ethylamino ethylamine, histidine and procaine.

The term "pharmaceutically acceptable excipient" relates to a compound which causes no or only a small significant irritation when administered to the patient and which does not abolish the activity of the compounds of the invention or interferes with the compounds of the invention.

The pharmaceutical compositions comprising at least one compound of the invention and/or a pharmaceutically acceptable salt thereof in admixture with a pharmaceutically acceptable excipient may be administered to the patient in any mode, e.g. by oral, buccal, sublingual, topic, enteral, nasal, ocular, topical, intraveneous, intramuscular, subcutaneous and rectal administration. The pharmaceutical compositions may be in form of tablets, lozenges, coated tablets, guttae, suppositories, solutions, suspensions, emulsions, syrups, sprays, capsules (preferably soft or hard gelatin capsules), grains or powders.

### Methods of preparation

The compounds of the present invention can be prepared by methods well known to those skilled in the art and by the methods described below, or variations thereon well known to those skilled in the art. All processes disclosed in association with the present invention are practiced on any scale from milligram to multi-kilogram commercial industrial scale.

Functional groups present in the compounds of the present invention may contain protecting groups during the course of the synthesis. For example, hydroxy substituents on the carbacycles in Formula I can be substituted with protecting groups such as t-butyldimethylsilyl or trimethylsilyl groups; amino substituents on the carbacycles in Formula I can be substituted with protecting groups such as benzyloxycarbonyl or t-butoxycarbonyl groups. Protecting groups including, but not limited to, the ones mentioned above are present in a chemical compound to render the substituted functionality inert to chemical reaction conditions to which the compound is exposed during the synthesis, but can also removed selectively from the substituted functionalities at any given synthetic step by methods known to the skilled artisan. Preferred protecting groups according to the invention include, but are not limited to, the above mentioned ones. Other preferred protecting groups can be found in Greene, T.W. and Wuts, P.G.M., *Protective Groups in Organic Synthesis,* 3^{rd} Ed., Wiley and Sons, 1999.

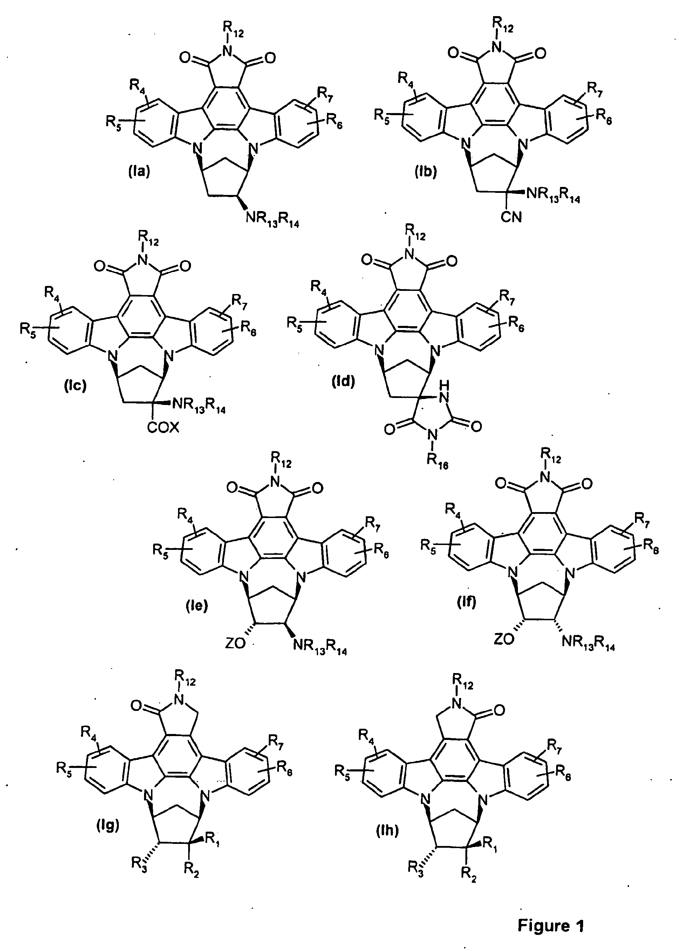

**Fig. 1** shows the structures of compounds of formula I having the formulae Ia-h whose synthesis is described in detail below as examples for the preparation of the members of the general class of compounds depicted in the general formula I.

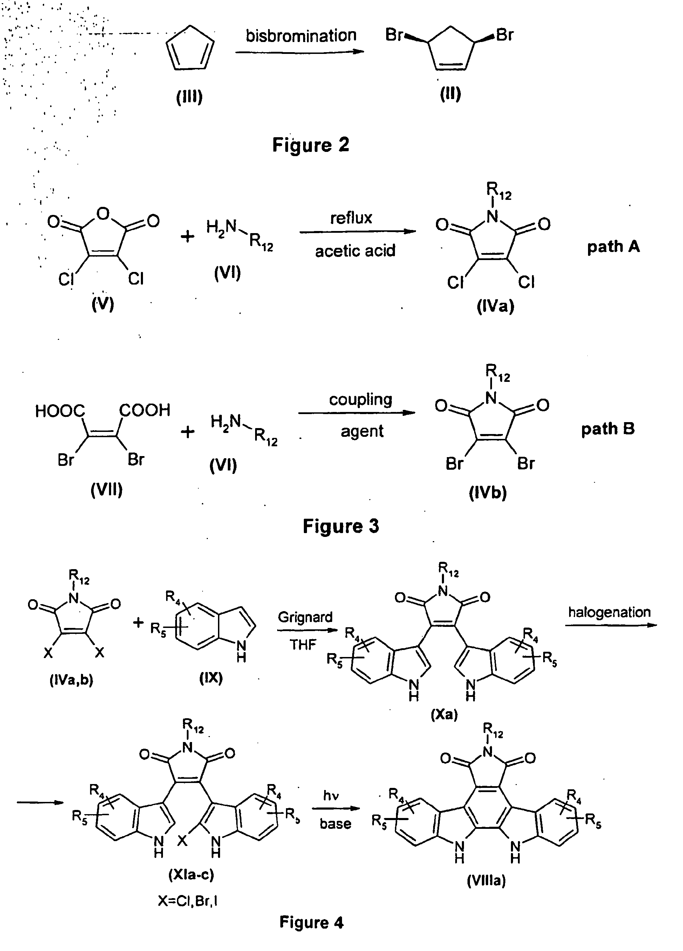

The compounds shown in **Fig. 1** may be prepared, for example, using key intermediates and synthetic strategies as described in **Fig. 2** through **Fig. 19.** X and Z denote a further decoration by a higher degree of functionalization.

**Fig. 2** shows the synthesis of the cyclopentene intermediate (II) which has been used for the synthesis of the compounds of the general formula I.

The compound (II) may be prepared by methods including, but not limited to, 1,4-addition of bromine on commercially available cyclopentadiene (III), using experimental protocols known to those skilled in the art such as, but not limited to, reaction in chloroform at -70°C for 1 hour.

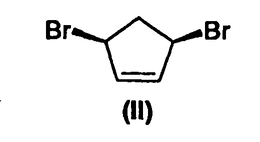

**Fig. 3** shows the synthesis of bis-halo maleimide intermediates (IVa,b) which have been used for the synthesis of the compounds of the general formula I.

Treatment of commercially available bis-chloromaleic anhydride (V) with a suitable primary amine (VI), mostly commercially available or prepared using precursors and pathways known to those skilled in the art, yields the corresponding bis-chloro compounds of formula (IVa) **(Fig. 3, path A).** Using commercially available 2,3-dibromomaleic acid (VII) and the same primary amines in the presence of coupling agents known to those skilled in the art the corresponding bis-bromo compounds of formula (IVb) are obtained **(Fig. 3, path B).**

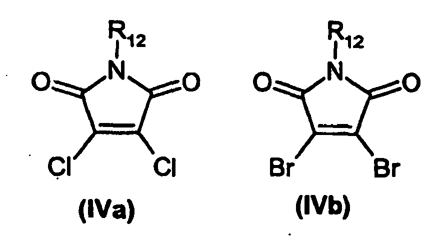

**Fig. 4** shows the synthesis of key N-protected, symmetrical indolocarbazole imide aglycones (VIIIa) which have been used for the synthesis of the compounds of the general formula I. N-protected bis-halomaleimides (IVa,b) and indoles (IX) are the intermediates for the specific synthetic strategy leading to compounds (VIIIa).

Compounds (IVa,b) may be condensed with slightly more than two equivalents of an activated derivative of indoles (IX) including, but not limited to, ethylmagnesium salts, using coupling conditions including, but not limited to, ethylmagnesium bromide in toluene/THF at reflux for 4 hours under nitrogen, affording N-protected symmetrical bisindolylmaleimides (Xa). Compounds (Xa) may be selectively monohalogenated using monohalogenating conditions and reagents, including, but not limited to, bromine and sulfuryl chloride, N-bromosuccinimide, N-chlorosuccinimide and elemental iodine, affording mono-2-halo N-protected bisindolylmaleimides (XIa-c). Compounds (XIa-c) may be cyclized using experimental conditions including, but not limited to, irradiation with a halogen lamp in refluxing ethyl acetate in the presence of diisopropylethylamine for 4 hours, affording the key N-protected, symmetrical indolocarbazole imide aglycones (VIIIa).

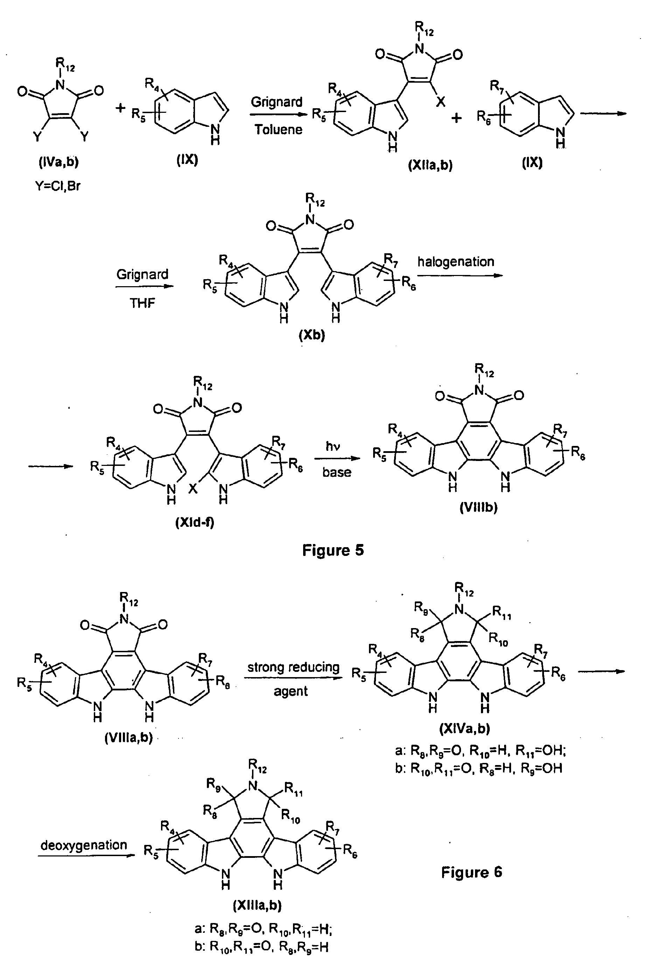

A niodification of the synthetic strategy shown in Fig. 4 is reported in Fig. 5 and leads to the key N-protected, unsymmetrical indolocarbazole imide aglycones (VIIIb) which have been used for the synthesis of the compounds of the general formula I. The same N-protected bis-halomaleimides (IVa,b) and indoles (IX) are the intermediates for the specific synthetic strategy leading to compounds (VMb).

Compounds (IVa,b) may be condensed with slightly more than one equivalent of an activated derivative of indoles (IX) including, but not limited to, ethylmagnesium salts, using coupling conditions including, but not limited to, ethylmagnesium bromide in toluene under nitrogen, affording N-protected mono-halo, monoindolylmaleimides (XIIa,b). Compounds (XIIa,b) may be condensed with slightly more than one equivalent of another activated derivative of indoles (IX) including, but not limited to, ethylmagnesium salts, using coupling conditions including, but not limited to, ethylmagnesium bromide in toluene or THF under nitrogen, affording N-protected unsymmetrical bisindolylmaleimides (Xb). Compounds (Xb) may be selectively monohalogenated using monohalogenating conditions and reagents, including, but not limited to, bromine and sulfuryl chloride, N-bromosuccinimide, N-chlorosuccinimide and elemental iodine, affording mono-2-halo N-protected bisindolylmaleimides (XId-f). Compounds (XId-f) may be cyclized using experimental conditions including, but not limited to, irradiation with a halogen lamp in refluxing ethyl acetate in the presence of DIPEA for 4 hours, affording the key N-protected, unsymmetrical indolocarbazole imide aglycones (VIIIb).

**Fig. 6** shows the synthesis of key N-protected indolocarbazole lactam aglycones (XIIIa,b) which have been used for the synthesis of the compounds of the general formula I, especially when only one among the groups R₈,R₉ and R₁₀,R₁₁ is a carbonyl group.

N-protected indolocarbazole imide aglycones (VIIIa,b) may be reduced using experimental conditions including, but not limited to, lithium aluminium hydride in tetrahydrofuran, affording the hydroxy lactams (XIVa,b). Compounds (XIVa,b) may be deoxygenated using experimental conditions including, but not limited to, triethylsilane in trifluoroacetic acid in presence of ammonium fluoride, affording key N-protected indolocarbazole lactam aglycones (XIIIa,b).

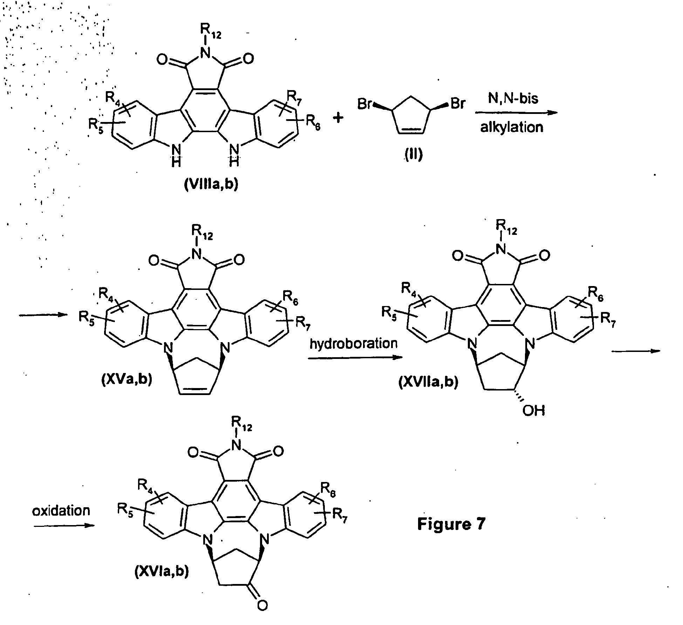

**Fig. 7** shows the synthesis of key N-imide-protected, N,N-bridged indolocarbazole imides (XVa,b) and (XVIa,b) which have been used for the synthesis of the compounds of the general formula I. N-protected indolocarbazole imide aglycones (VIIIa,b) and cis-1,4-dibromo cyclopentene (II) are the intermediates for the specific synthetic strategy leading to compounds (XVa,b and XVIa,b).

N-protected indolocarbazole imide aglycones (VIIIa,b) may be N,N-dialkylated with compound (II) using experimental conditions including, but not limited to, sodium hydride in THF at 0°C for 2 hours, affording the key alkene bridged indolocarbazole imides (XVa,b). Compounds (XVa,b) may be hydroxylated using experimental conditions including, but not limited to, boraneltetrahydrofuran complex in THF at rt **for** 16 hours, affording *trans*-hydroxy bridged indolocarbazole imides (XVIIa,b). Compounds (XVIIa,b) may be oxidized using experimental conditions including, but not limited to, pyridinium chlorochromate in 1,2-dichloroethane at rt for 4 hours, affording the key keto bridged indolocarbazole imides (XVIa,b).

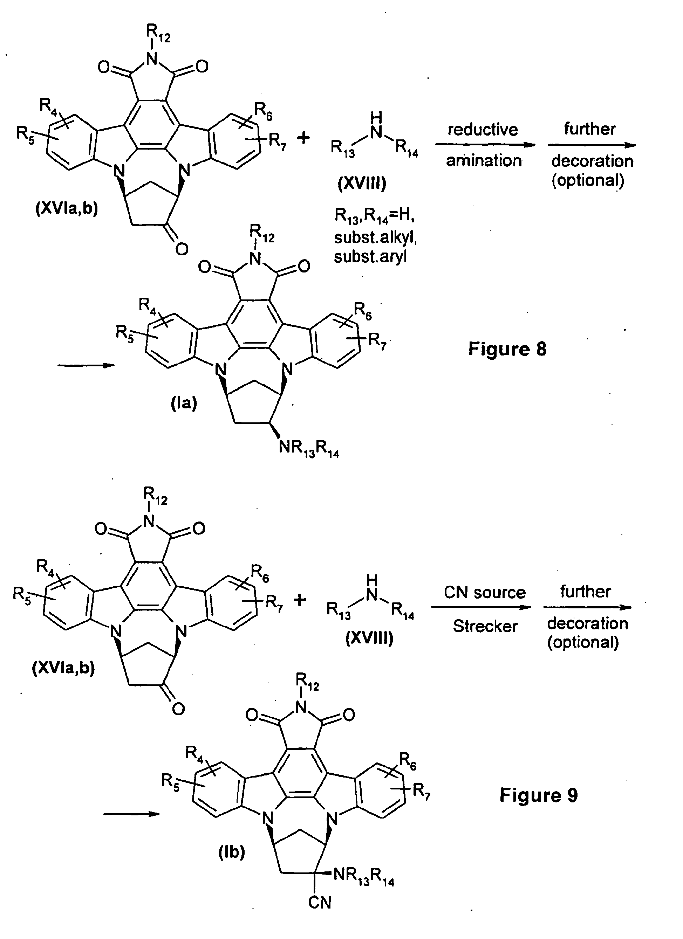

**Fig. 8** shows a detailed pathway for the synthesis of compounds (Ia) from keto bridged indolocarbazole imides (XVIa,b) as examples for the preparation of the members of the general class of compounds depicted in the general formula I.

Condensation between keto bridged indolocarbazole imides (XVIa,b) and suitable primary or secondary amines (XVIII), mostly commercially available or prepared using precursors and pathways known to those skilled in the art, was performed using reductive amination conditions including, but not limited to, sodium cyanoborohydride in tetrahydrofuran at rt for 4 hours. Further decoration of the amino function depending on the nature of R₁₃ and R₁₄ may be possible using experimental protocols well known to those skilled in the art and including, but not limited to, acylation, alkylation and a second reductive amination, affording *cis*-nitrogen substituted bridged indolocarbazole imides (Ia).

**Fig. 9** shows a detailed pathway for the synthesis of compounds (Ib) from keto bridged indolocarbazole imides (XVIa,b) as examples for the preparation of the members of the general class of compounds depicted in the general formula **I.**

Reaction of keto bridged indolocarbazole imides (XVIa,b) with suitable primary or secondary amines (XVIII), mostly commercially available or prepared using precursors and pathways known to those skilled in the art, in presence of a suitable nitrile source such as, but not limited to, trimethylsilyl cyanide and potassium cyanide, was performed using Strecker experimental protocols including, but not limited to, titanium isopropoxide in tetrahydrofuran at rt for 18 hours under nitrogen. Further decoration of the amino function depending on the nature of R₁₃ and R₁₄ may be possible using experimental protocols well known to those skilled in the art and including, but not limited to, acylation, alkylation and reductive amination, affording cis-nitrogen, *trans*-cyano substituted bridged indolocarbazole imides (Ib).

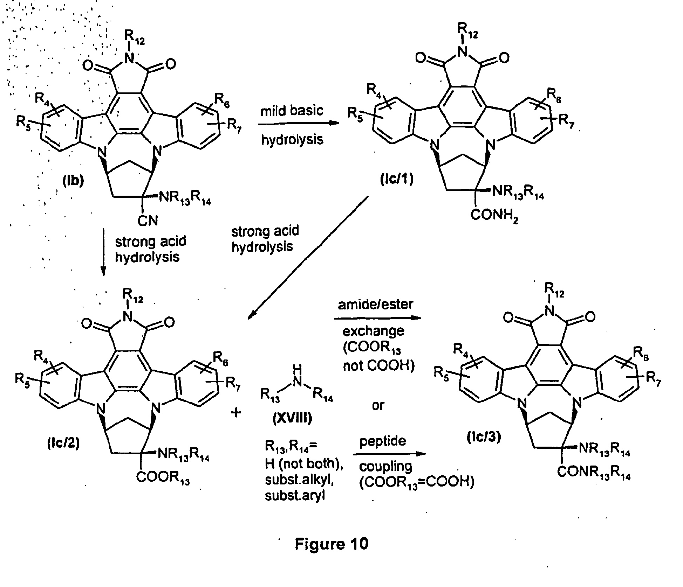

**Fig. 10** shows a detailed pathway for the synthesis of compounds (Ic/1-3) from *cis*-nitrogen, *trans*-cyano substituted bridged indolocarbazole imides (Ib) as examples for the preparation of the members of the general class of compounds depicted in the general formula I.

Treatment of *cis*-nitrogen, *trans*-cyano substituted bridged indolocarbazole imides (Ib) in mild basic hydrolytic conditions including, but not limited to, lithium hydroxide in a mixture of hydrogen peroxide and tetrahydrofuran at 5°C for 4 hours affords the α-amino carboxamide substituted bridged indolocarbazole imides (Ic/1). Treatment of *cis*-nitrogen, *trans*-cyano substituted bridged indolocarbazole imides (Ib) in strong acidic hydrolytic conditions including, but not limited to, concentrated sulfuric acid in alcohol or in water at reflux for 16 hours affords the α-amino carboxamide or α-amino ester substituted bridged indolocarbazole imides (Ic/2).

Compounds (Ic/2) may also be conveniently obtained by treatment of α-amino carboxamide substituted bridged indolocarbazole imides (Ic/1) using the same strong acidic hydrolytic conditions. Treatment of α-amino ester substituted bridged indolocarbazole imides (Ic/2, COOR₁₃ not COOH) with suitable primary or secondary amines (XVIII), mostly commercially available or prepared using precursors and pathways known to those skilled in the art, in experimental conditions including, but not limited to, tetrahydrofuran at rt for 16 hours or at reflux for 6 hours, affords the α-amino carboxamide substituted bridged indolocarbazole imides (Ic/3, either one or both R₁₃ and R₁₄ on the amide function different from H). Compounds (Ic/3, either one or both R₁₃ and R₁₄ on the amide- function different from H) may also be conveniently obtained by treatment of α-aminoacid substituted bridged indolocarbazole imides (Ic/2, COOR₁₃=COOH) using the same amines (XVIII) in typical peptide coupling conditions including, but not limited to, N-hydroxybenzotriazole/diisopropylethylamine or carbonyldiimidazole in tetrahydrofuran or dimethylformamide at rt for 16 hours. Further decoration of the amino function and of the amide function of (Ic/3) and (Ic/1) and of the ester function of (Ic/2) depending respectively on the nature of R₁₃ and R₁₄ may be possible using experimental protocols well known to those skilled in the art and including, but not limited to, acylation, alkylation and reductive amination, affording compounds (Ic/1-3) with a higher degree of functionalization.

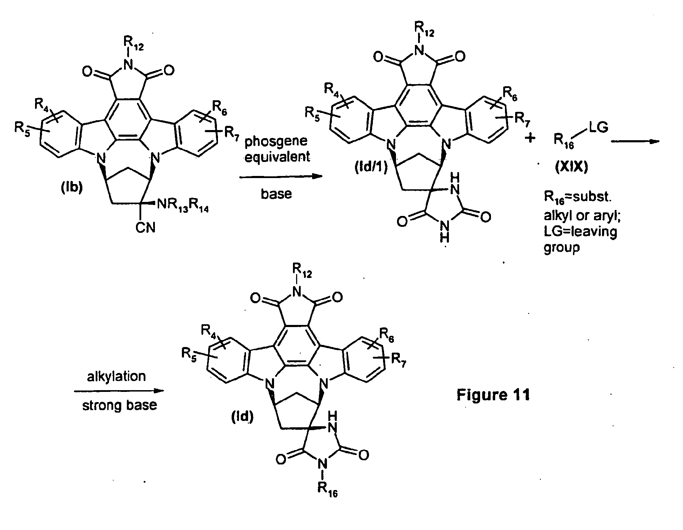

**Fig. 11** shows a detailed pathway for the synthesis of compounds (Id) and (Id/1) from cis-amino, *trans*-cyano substituted bridged indolocarbazole imides (Ib) as examples for the preparation of the members of the general class of compounds depicted in the general formula I.

Treatment of *cis*-amino, *trans*-cyano substituted bridged indolocarbazole imides (Ib) with a phosgene equivalent in presence of a base in experimental conditions and reagents including, but not limited to, triphosgene and triethylamine in tetrahydrofuran at rt for 4 hours yields spiro hydantoin substituted bridged indolocarbazole imides (Id/1). Treatment of spiro hydantoin substituted bridged indolocarbazole imides (Id/1) with alkylating agents (XIX) in strong basic conditions including, but not limited to, sodium hydride in dimethylformamide at 50°C for 4 hours affords N-monoalkylated spiro hydantoin substituted bridged indolocarbazole imides (Id, R₁₆ not H).

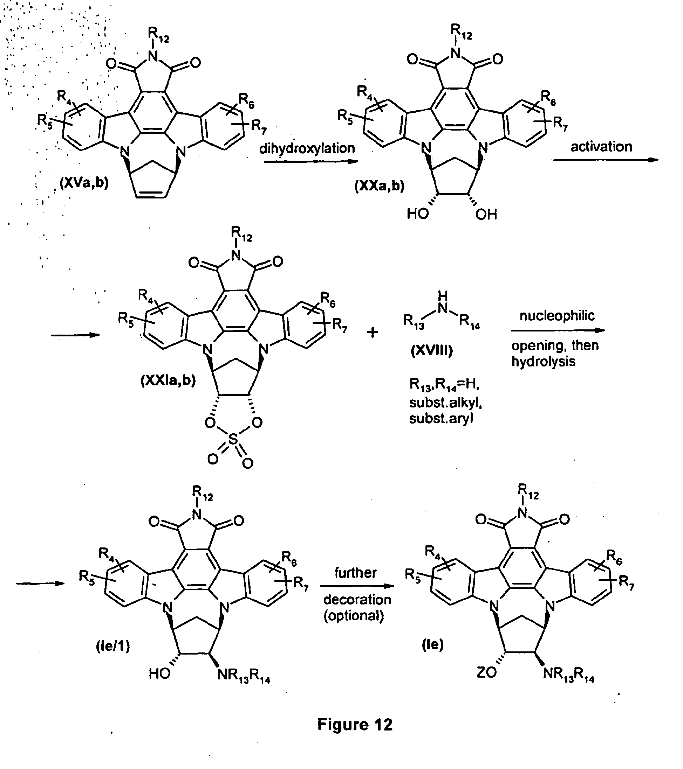

**Fig. 12** shows a detailed pathway for the synthesis of compounds (Ie) and (Ie/1) from alkene bridged indolocarbazole imides (XVa,b) as examples for the preparation of the members of the general class of compounds depicted in the general formula I.

Reaction of alkene bridged indolocarbazole imides (XVa,b) with dihydroxylating reagents such as, but not limited to, N-methylmorpholine N-oxide in presence of catalytic osmium tetroxide, in experimental conditions such as, but not limited to, a mixture of acetone and water at rt for 24 hours, leads to the cis-diol bridged indolocarbazole imides (XXa,b). Compounds (XXa,b) may be activated for nucleophilic substitutions by reacting them with bifunctional reagents such as, but not limited to, sulfonyldiimidazole in experimental conditions such as, but not limited to, diazabicycloundecane (DBU) in tetrahydrofuran at rt for 18 hours to give the cyclic sulfate-bridged indolocarbazole imides (XXIa,b). Compounds (XXIa,b) may be reacted with suitable primary or secondary amines (XVIII), mostly commercially available or prepared using precursors and pathways known to those skilled in the art, in experimental conditions including, but not limited to, tetrahydrofuran or dimethylformamide at temperatures ranging from rt to 100°C for times ranging from 4 to 24 hours, affording, after acidic sulfate hydrolysis, β *cis-*amino, *trans-*hydroxy bridged indolocarbazole imides (Ie/1). Further decoration of the hydroxy and of the amino function may be possible using experimental protocols well known to those skilled in the art and including, but not limited to, acylation, alkylation and reductive amination, affording compounds (Ie) with a higher degree of functionalization.

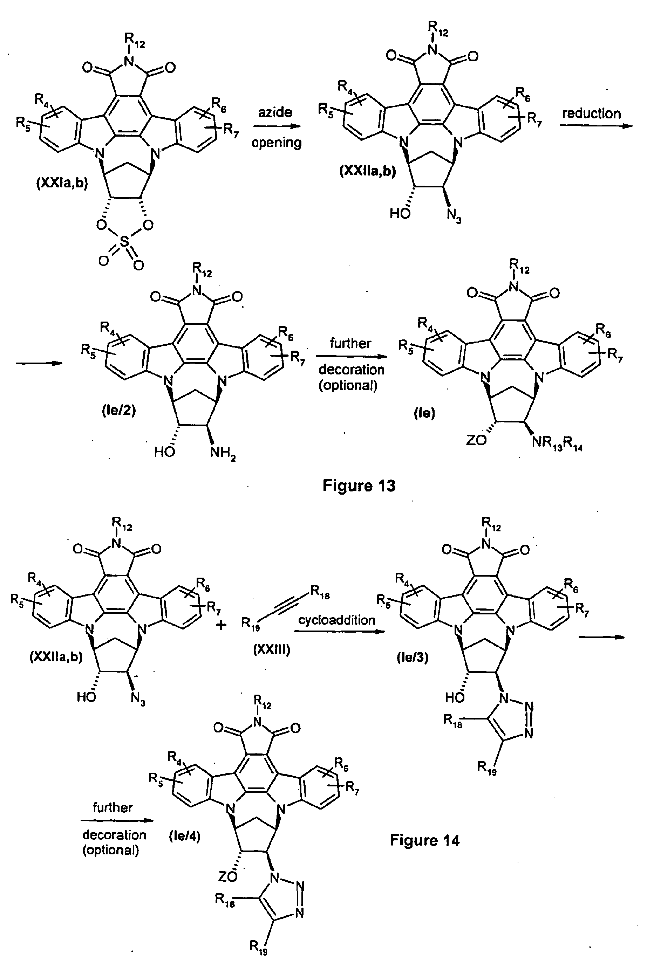

**Fig. 13** shows an alternative detailed pathway for the synthesis of compounds (Ie) and (Ie/2) from cyclic sulfate-bridged indolocarbazole imides (XXIa,b) as examples for the preparation of the members of the general class of compounds depicted in the general formula I.

Cyclic sulfate-bridged indolocarbazole imides (XXIa,b) may be treated with azidation reagents such as, but not limited to, sodium azide in various experimental conditions, such as, but not limited to, dimethylformamide at 80°C for 3 hours, yielding, after acidic sulfate hydrolysis, β *cis*-azido, *trans*-hydroxy bridged indolocarbazole imides (XXIIa,b). Compounds (XXIIa,b) may be reduced using a variety of reagents and experimental conditions such as, but not limited to, catalytic hydrogenation with Pd/C 10% at 200PSI of hydrogen in dimethylformamide at rt for 3 hours providing the unsubstituted β cis-amino, *trans*-hydroxy bridged indolocarbazole imides (Ie/2). Further decoration of the hydroxy and of the amino function may be possible using experimental protocols well known to those skilled in the art and including, but not limited to, acylation, alkylation and reductive amination, affording compounds (Ie) with a higher degree of functionalization.

**Fig. 14** shows a detailed pathway for the synthesis of compounds (Ie/3) and (Ie/4) from *cis-*azido, *trans*-hydroxy bridged indolocarbazole imides (XXIIa,b) as examples for the preparation of the members of the general class of compounds depicted in the general formula I.

*Cis*-azido, *trans*-hydroxy bridged indolocarbazole imides (XXIIa.b) may be treated with suitable alkynes (XXIII), mostly commercially available or prepared using precursors and pathways known to those skilled in the art, in a variety of experimental conditions including, but not limited to, copper (I) iodide and diisopropylethylamine in tetrahydrofuran at rt for 6 hours, affording the *cis*-triazolyl, *trans*-hydroxy bridged indolocarbazole imides (Ie/3). Further decoration of the hydroxy function, and of the heterocyclic substituents depending on the nature of R₁₈ and R₁₉, may be possible using experimental protocols well known to those skilled in the art and including, but not limited to, acylation and alkylation, affording compounds (Ie/4) with a higher degree of functionalization.

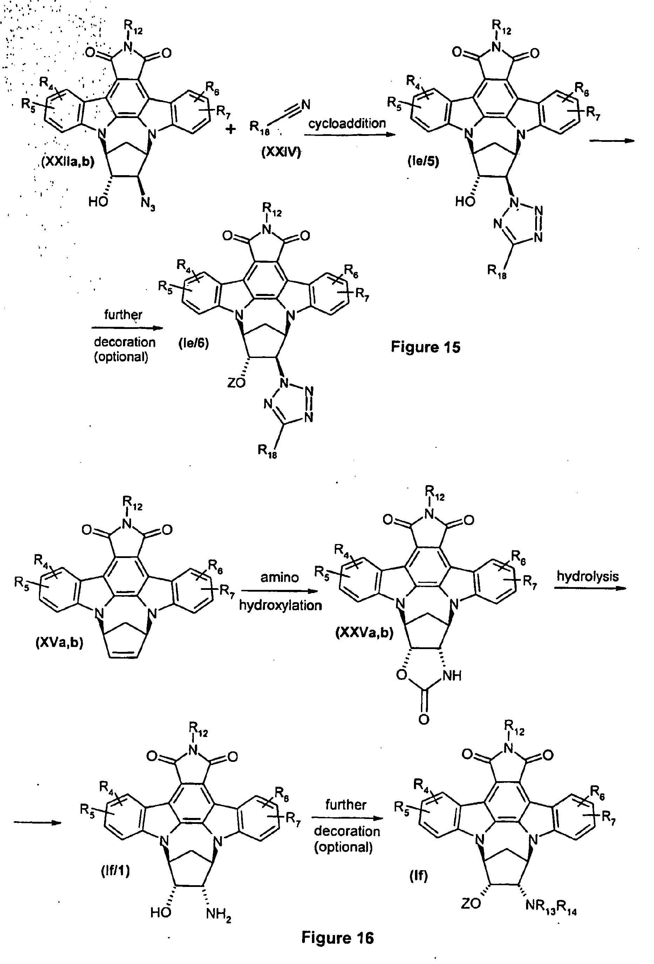

**Fig. 15** shows a detailed pathway for the synthesis of compounds (Ie/5) and (Ic/6) from *cis-*azido, *trans-hydroxy* bridged indolocarbazole imides (XXIIa,b) as examples for the preparation of the members of the general class of compounds depicted in the general formula I.

*Cis*-azido, *trans-*hydroxy bridged indolocarbazole imides (XXIIa,b) may be treated with suitable nitriles (XXIV), mostly commercially available or prepared using precursors and pathways known to those skilled in the art, in a variety of experimental conditions including, but not limited to, copper (I) iodide and diisopropylethylamine in tetrahydrofuran at rt for 6 hours, affording the *cis*-tetrazolyl, *trans*-hydroxy bridged indolocarbazole imides (Ie/5). Further decoration of the hydroxy function, and of the heterocyclic substituent depending on the nature of R₁₈, may be possible using experimental protocols well known to those skilled in the art and including, but not limited to, acylation and alkylation, affording compounds (Ie/6) with a higher degree of functionalization.

**Fig. 16** shows a detailed pathway for the synthesis of compounds (If) and (If/1) from alkene bridged indolocarbazole imides (XVa,b) as examples for the preparation of the members of the general class of compounds depicted in the general formula I.

Alkene bridged indolocarbazole imides (XVa,b) may be reacted in aminohydroxylating protocols using a variety of reagents and experimental conditions including, but not limited to, catalytic potassium osmate dihydrate, 1,3-dichloro-5,5-dimethylhydantoin, ethyl urethane and sodium hydroxide in a water-tetrahydrofuran mixture at rt for 16 hours, affording the oxazolidone bridged indolocarbazole imides (XXVa,b). Compounds (XXVa,b) may be hydrolyzed in strong basic conditions such as, but not limited to, sodium hydroxide in water at 80°C for 4 hours yielding the unsubstituted β *cis* amino, *cis* hydroxy bridged indolocarbazole imides (If/1). Further decoration of the hydroxy and of the amino function may be possible using experimental protocols well known to those skilled in the art and including, but not limited to, acylation, alkylation and reductive amination, affording compounds (If) with a higher degree of functionalization.

It will be appreciated by the person skilled in the art that the compounds (Ia-f) may contain one or more chiral centers, and may be isolated in optically active or racemic forms. Thus, all chiral, diastereomeric, racemic forms and all geometric isomeric forms of a structure may be obtained either using regio-, diastereo- and/or stereoselective reagents and experimental protocols which are well known to those skilled in the art, or by using suitable separation techniques also well known to those skilled in the art to isolate individual components from diastereomeric or racemic mixtures.

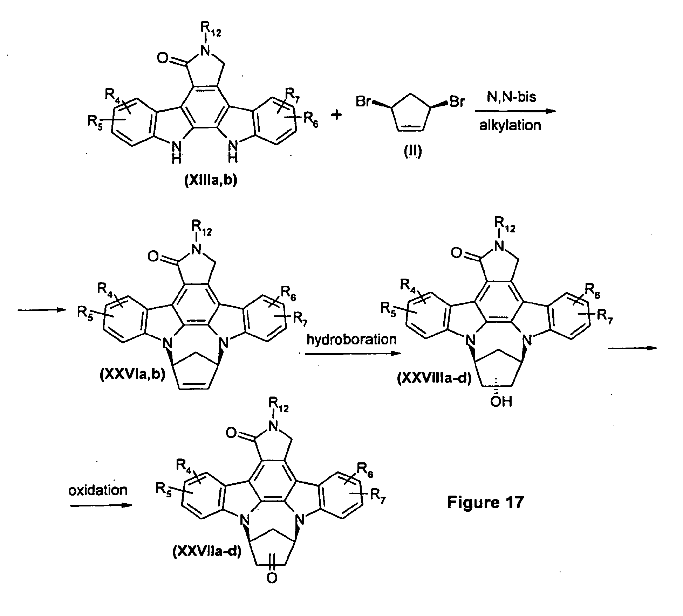

**Fig. 17** shows the synthesis of key N-lactam-protected, N,N-bridged indolocarbazole lactams (XXVIa,b) and (XXVIIa-d) which have been used for the synthesis of the compounds of the general formula I when only one among R₈,R₉ and R₁₀,R₁₁ represents a carbonyl group. N-protected indolocarbazole lactam aglycones (XIIIa,b) and cis-1,4-dibromo cyclopentene (II) are the intermediates for the specific synthetic strategy leading to compounds (XXVIa,b) and (XXVna-d).

N-protected indolocarbazole lactam aglycones (XIIIa,b) may be N,N-dialkylated with compound (II) using experimental conditions including, but not limited to, sodium hydride in THF at 0°C for 2 hours, affording the key alkene bridged indolocarbazole lactams (XXVIa,b). Compounds (XXVIa,b) may be hydroxylated using experimental conditions including, but not limited to, borane/tetrahydrofuran complex in THF at rt for 16 hours, affording *trans*-hydroxy bridged indolocarbazole lactams (XXVIIIa-d). Compounds (XXVIIIa-d) may be oxidized using experimental conditions including, but not limited to, pyridinium chlorochromate in 1,2-dichloroethane at rt for 4 hours, or oxalyl chloride and dimethyl sulfoxide in 1,2-dichloroethane at rt for 16 hours, affording the key keto bridged indolocarbazole lactams (XXVIIa-d).

It will be appreciated by the person skilled in the art that the compounds (XXVIIa-d) and (XXVHIa-d) may be obtained as a mixture of regioisomers due to the unsymmetrical nature of the lactam moiety. We define from here on the *entgegen* (E) regioisomer as the one where the carbonyl of the lactam and the cyclopentane substituent are pointing towards opposite directions and the *zusammen* (Z) couple as the one where the carbonyl of the lactam and the cyclopentane substituent are pointing towards the same direction.

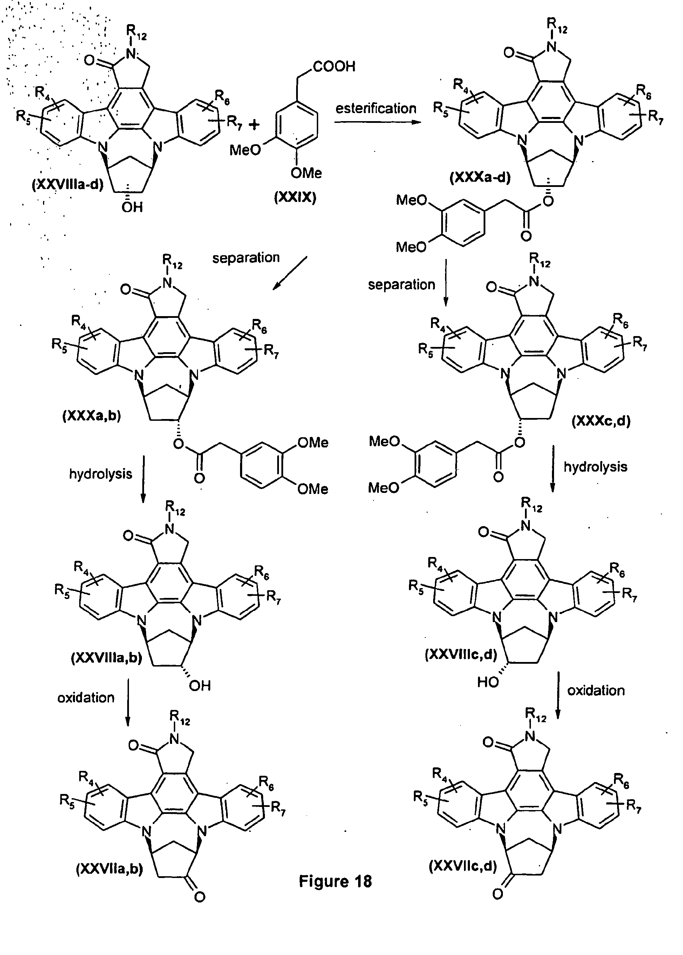

**Fig. 18** shows the synthesis of regioisomerically pure key keto bridged indolocarbazole lactam E- (XXVIIa,b) and Z-couples (XXVIIc,d) from the regioisomeric alcohol mixture (XXVIIIa-d). Trans-hydroxy bridged indolocarbazole lactams (XXVIIIa-d) may be esterified with a suitable, commercially available carboxylic acid such as, but not limited to, 3,4-dimethoxyphenylacetic acid (XXDC) in experimental conditions such as, but not limited to, water-soluble carbodiimide and dimethylaminopyridine in tetrahydrofuran at rt for 3 hours, affording the *trans* esterified bridged indolocarbazole lactams (XXXa-d). Compounds (XXXa-d) may be separated by a variety of preparative analytical methods such as, but not limited to, direct phase chromatography (silicagel) using a variety of solvents including, but not limited to, mixtures of ethyl acetate and petroleum ether, providing the two racemic mixture of diastereomerically pure E- and Z*-trans* esterified bridged indolocarbazole lactam couples (XXXa,b) and (XXXc,d). The two couples may be then hydrolyzed in mild basic conditions such as, but not limited to, sodium methoxide in a mixture of methanol and tetrahydrofuran at rt for 2 hours, affording the pure E- and Z*-trans* hydroxy bridged indolocarbazole lactam couples (XXVIIIa,b) and (XXVIIIc,d). Compounds (XXVIIIa,b) and (XXVIIIc,d) may be separately oxidized using experimental conditions including, but not limited to, pyridinium chlorochromate in 1,2-dichloroethane at rt for 4 hours, affording the key E- and Z*-trans* keto bridged indolocarbazole lactam couples (XXVIIa,b) and (XXVIIc,d).

It will be appreciated by the person skilled in the art that the two E- and Z-couples (XXVIIa,b) and (XXVIIc,d) may be functionalized introducing amino substituents on the carbacycle ring using a wide variety of reagents and experimental conditions as shown, but not limited to, the ones used for indolocarbazole imides in Figs. 8-11.

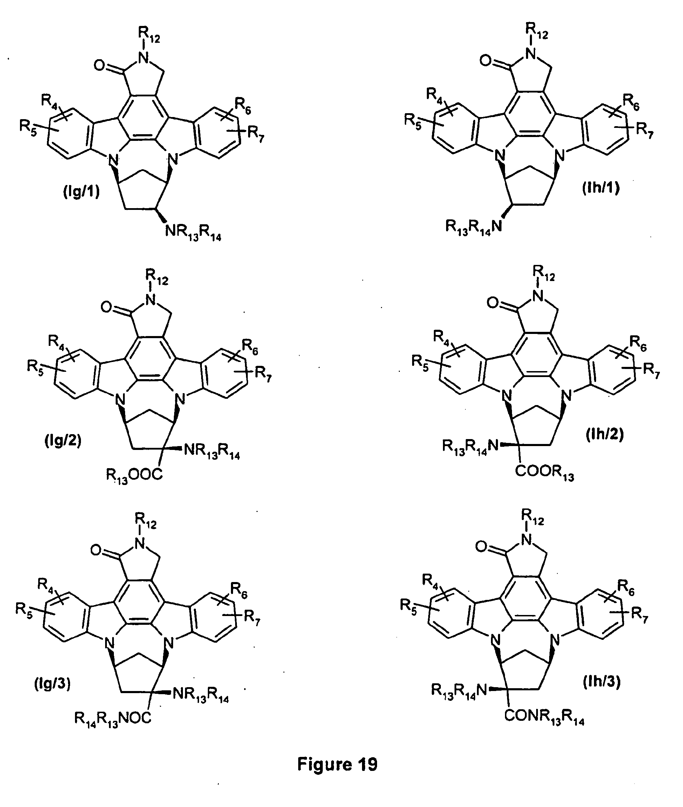

**Fig. 19** for example shows the structures of E-couples of amino substituted indolocarbazole lactams (Ig/1-3) and of Z-couples of amino substituted indolocarbazole lactams (Ih/1-3) which have been prepared with the same protocols as mentioned in Figs. 8-10.

The following non-limiting examples describe the synthesis of single diastereoisomers, so that final compounds 1-20 and all the chiral intermediates to their synthesis are isolated as racemates. The chosen representation arbitrarily depicts only one of the two enantiomers composing the racemic mixture, Compounds 21 to 48 are listed in Table 1 and have been prepared using methods described above. The isolation of a specific enantiomer could be performed by methods known in the art and is described, for example, in Examples 49 to 55.

### Examples:

### Example 1

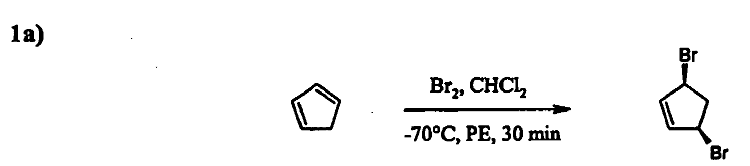

A solution of bromine (47.2 mL, 0.926 mol) in CHCl₃ (50 mL) was added to a solution of freshly distilled cyclopentadiene (64.45 g, 0.975 mol) in CHCl₃ (150 mL) at -70°C under nitrogen atmosphere. After 30 minutes, petroleum ether (2 L) was added and the solution was stirred for 1 hour at -70°C. The white precipitate was filtered under nitrogen and washed with cold petroleum ether (500 mL) to give 3,5-dibromocyclopentene (80.5 g, yield 38.1 %) which was directly used in the following step. 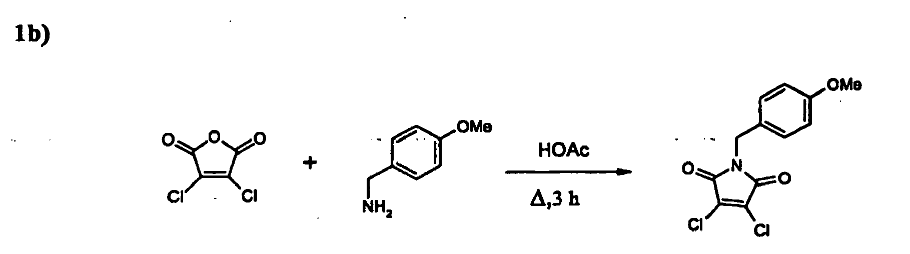 p-Methoxybenzyl amine (75.3 g, 549 mmol) was added dropwise to a stirred solution of dichloromaleic anhydride (91.6 g, 549 mmol) in AcOH (850 mL) at room temperature. The solution was refluxed for 3 hours and left overnight at room temperature. The precipitate was filtered and washed with AcOH (2x100 mL) and ice-cold EtOH (2x100 mL) to give after drying in vacuo the pure title compound (76.4 g). The filtrate was concentrated to 300 mL and cooled to -5°C for 4 hours to give a second crop of pure title compound (49.5 g, total yield 80.3 %).

¹H-NMR (300 MHz, DMSO-*d*_{*6*}):δ 3.61 (3H, s, OCH₃), 4.60 (2H, s, N-CH₂), 6.85 (2H, d,

Harom) 7.20 (2H, d, Harom). MS (ESI) m/z 286 [M+H]⁺.

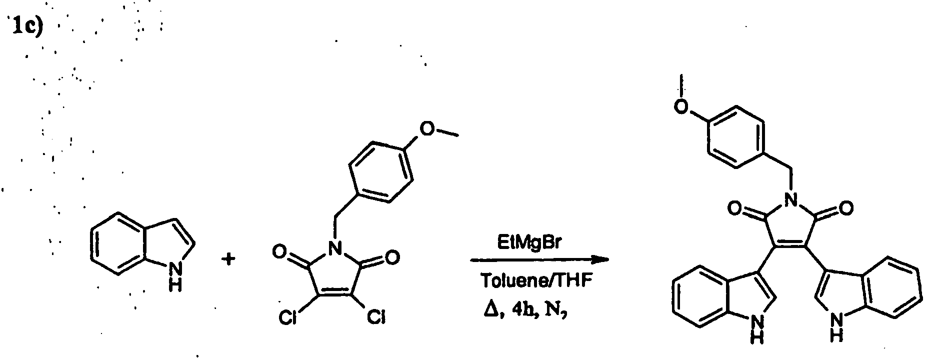

Ethylmagnesium bromide (3 M in Et₂O, 100 mL, 0.3 mol) was added dropwise under stirring to a solution of indole (35.1 g, 0.3 mol) in toluene (600 mL) and THF (60 mL) under nitrogen atmosphere. The greenish solution was stirred for 1 hour at room temperature before adding a solution of 1b (39 g, 0.136 mol) within 1 hour. The reaction mixture was then refluxed for 4 hours, cooled to room temperature and stirred overnight. The solvent was eliminated in vacuo and the residue dissolved in EtOAc (800 mL), treated with a saturated solution of NH₄Cl (150 mL), then washed with water. The organic phase was then dried over sodium sulfate, and concentrated. The crude residue was refluxed in CH₂Cl₂ (200 mL), cooled to -20°C, filtered and washed twice with cold CH₂Cl₂ to afford after drying the pure title compound (40.5 g, yield 67.0 %).

¹H-NMR (300 MHz, DMSO-d₆): δ 3.72 (3H, s, OCH₃), 4.69 (2H, s, CH₂-N), 6.62-7.00 (8H, m, Harom), 7.30 (2H, d, Harom), 7.35 (2H, d, Harom), 7.89 (2H, s, indole H-2), 11.70 (2H, bs, indole NH). MS (ESI) m/z 448 [M+H]⁺.

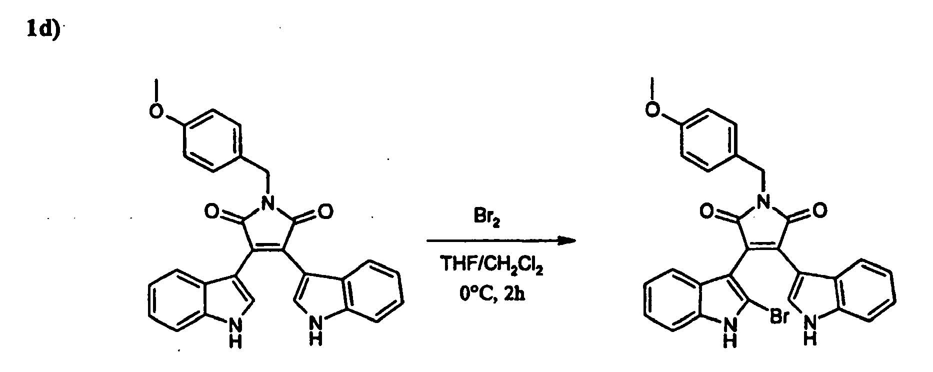

A clear red solution of 1c (35 g, 78.2 mmol) in CH₂Cl₂/THF 7/6 (1.3 L) was cooled to 0°C. Bromine (12.5 g, 78.2 mmol) in CH₂Cl₂ (25 mL) was added dropwise within 1 hour. The dark brown solution was stirred for 1 hour at 0°C, the cooling bath was removed, the solution was stirred for additional 30 minutes and was washed with a saturated solution of NaHCO₃ (200 mL), dried over sodium sulfate and concentrated in vacuo to yield a dark red foam (49.7 g). The crude product was recrystallized in acetonitrile (55 mL) to give a first crop of the expected compound (14.4 g). Mother liquors were concentrated to dryness and crystallized in CH₂Cl₂ to give a second crop of the-pure title-compound (16.7 g, total yield 75.9 %).

¹H-NMR (300 MHz, DMSO-d₆): δ 3.70 (3H, s, OCH₃), 4.65 (2H, s, CH₂-N), 6.35 (2H, d, Harom), 6.50-6.60 (2H, m, Harom), 6.90-7.20 (4H, m, Harom), 7.30-7.40 (4H, d, Harom), 8.10 (1H, s, indole H-2), 11.90 (1H, bs, indole NH) 12.45 (1H, bs, indole NH). MS (ESI) m/z 526 [M+H]⁺.

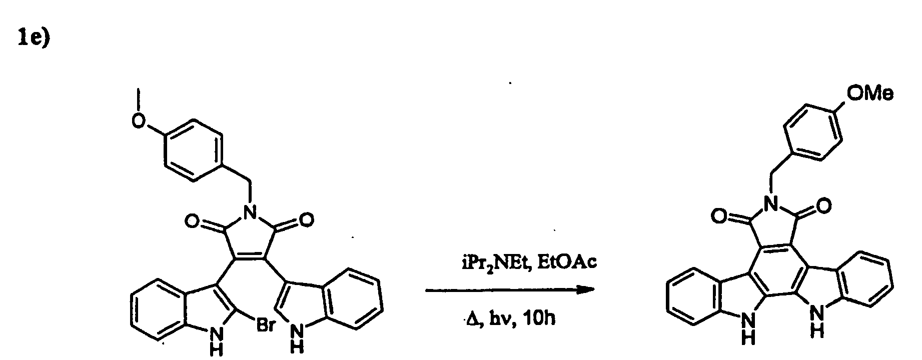

N-diisopropylethylamine (38 mL, 226 mmol) was added to a suspension of 1d (59.4 g, 113 mmol) in EtOAc (1 L) and the mixture was irradiated with a halogen lamp. After 10 h at reflux, the warm solution was washed with diluted HCl, water and brine and dried over sodium sulfate. The solvent was concentrated until appearance of a precipitate. The flask was cooled with an ice bath and the solid was filtered, washed with cold EtOAc and dried in vacuo at 40°C to give the title compound as a yellow powder (50 g, yield 99.2 %).

¹H-NMR (300 MHz, DMSO-d₆): δ 3.70 (3H, s, OCH₃), 4.80 (2H, s, CH₂-N), 6.90 (2H, d, Harom), 7.37 (4H, m, Harom), 7.55 (2H, m, Harom), 7.80 (2H, d, Harom), 8.98 (2H, s, Harom), 11.71 (2H, bs, indole NH). MS (ESI) m/z 446 [M+H]⁺.

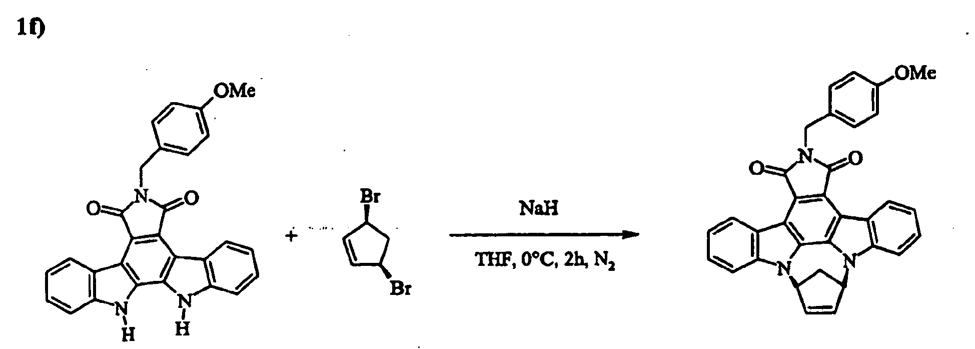

Sodium hydride (60% suspension in oil, 6.6 g, 165 mmol) was added to a solution of le (33.6 g, 75 mmol) in THF (800 mL) under nitrogen atmosphere at 0°C. The mixture was stirred for 45 min at this temperature, then a solution of 1a (20.3 g, 90 mmol) in THF (50 mL) was added dropwise within 20 min. After 30 min at 0°C (70% conversion by TLC monitoring) additional NaH (3 g, 75 mmol) was added and the reaction mixture was stirred for 1 h at room temperature. The reaction mixture was filtered through a short pad of celite, the solvent evaporated and the dark brown residue was triturated with EtOH (300 mL). The precipitate was filtered, washed-with-EtOH (50 mL) and dried in vacuo at 40°C to give the pure title compound (34.5 g, yield 90.4 %).

¹H-NMR (300 MHz, DMSO-*d*_{*6*}): δ 2.72 (1H, bs, 2-CH₂), 3.10 (1H, m, 2-CH₂), 3.35 (3H, s, OCH₃), 4.75 (2H, s, CH₂-PMB), 6.21 (2H, d, CH-N), 6.40 (2H, s, CH=CH), 6.85 (2H, d, Harom), 7.37 (4H, m, Harom), 7.60 (2H, bt, Harom), 7.98 (2H, d, Harom), 9.01 (2H, s, Harom). MS (ESI) m/z 510 [M+H]⁺.

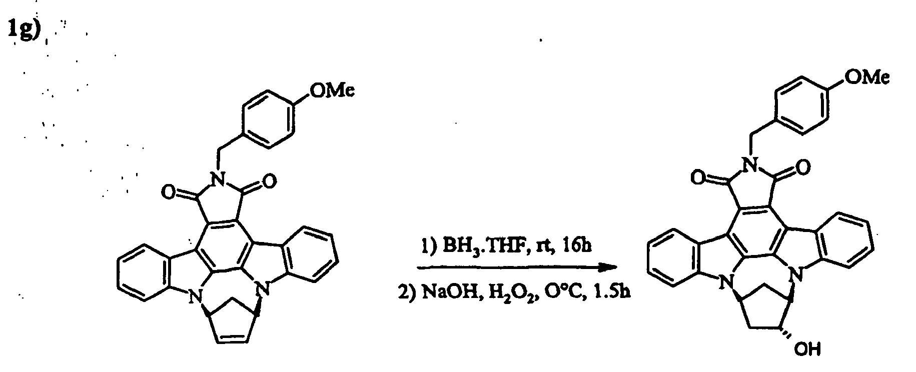

Borane (1M in THF, 285 mL, 285 mmol) was added at room temperature to a solution of 1f (29.0 g, 57 mmol) in THF (600 mL). The mixture was stirred overnight, then cooled to 0°C. 30% NaOH (30 mL) was added carefully, then 30% H₂O₂ (90 mL) was added dropwise within 90 min. The insoluble salts were filtered off and the solvent was removed in vacuo. The residue was dissolved in EtOAc (300 mL), washed with water (100 mL), brine (100 mL) and dried over sodium sulfate. The solvent was removed in vacuo and the residue was triturated in EtOH (150 mL). The orange solid was filtered, washed with cold EtOH (100 mL) and dried in vacuo at 50 °C to give the pure title compound (26 g, yield 86.4 %).

¹H-NMR (300 MHz, DMSO-d₆): δ 1.92 (1H, m, 5-CH₂), 2.42 (1H, m, 5-CH₂), 2.68 (1H, bs, 2-CH₂), 3.15 (1H, m, 2-CH₂), 3.35 (3H, s, OCH₃), 4.20 (1H, m, 4-CH-OH), 4.82 (2H, s, CH₂₋PMB), 5.36 (1H, d, 3-CH-N), 5,65 (1H, d, OH), 5.91 (1H, m, 1-CH-N), 6.91 (2H, m, Harom), 7.37 (4H, m, Harom), 7.62 (2H, bt, Harom), 7.96 (2H, d, Harom), 9.01 (2H, s, Harom). MS (ESI) m/z 528 [M+H]⁺.

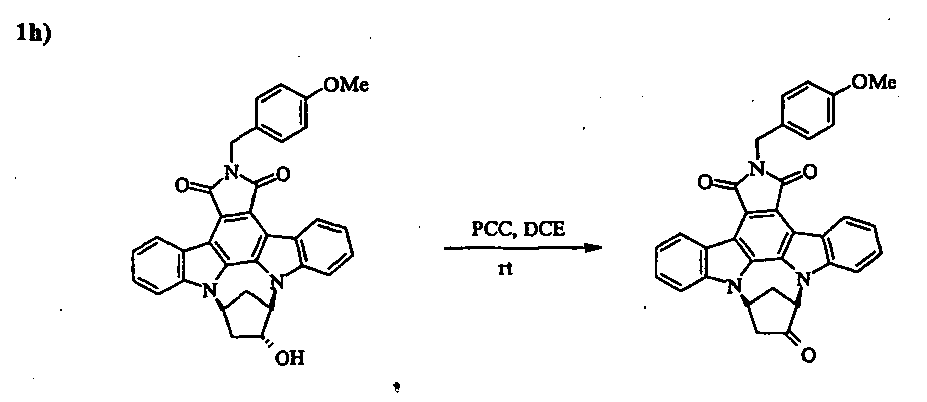

Pyridinium chlorochromate (16.2 g, 75 mmol) was added portionwise to a suspension of **1g** (26.5 g, 50 mmol) in 1,2-dichloroethane (1-L). The reaction mixture was stirred at room temperature for 4 hours and the solvent was removed in vacuo. Purification by flash chromatography (silica gel, CH₂Cl₂ as eluant mixture) gave the pure target compound as an orange powder (10.1 g, yield 39.6 %). ¹H-NMR (300 MHz, DMSO-d₆): δ 2.45 (1H, dd, 2-CH₂), 3.07 (1H, dd, 2-CH₂), 3.25 (1H, dd, 5-CH₂), 3.48 (1H, m, 5-CH₂), 3.68 (3H, s, OCH₃), 4.73 (2H, s, CH₂-PMB), 5.59 (1H, d, 3-CH-N), 6.13 (1H, bt, 1-CH-N), 6.85 (2H, d, Harom), 7.37 (4H, m, Harom) , 7.65 (2H, bt, Harom), 7.98 (2H, dd, Harom), 9.01 (2H, s, Harom). MS (ESI) m/z 526 [M+H]⁺.

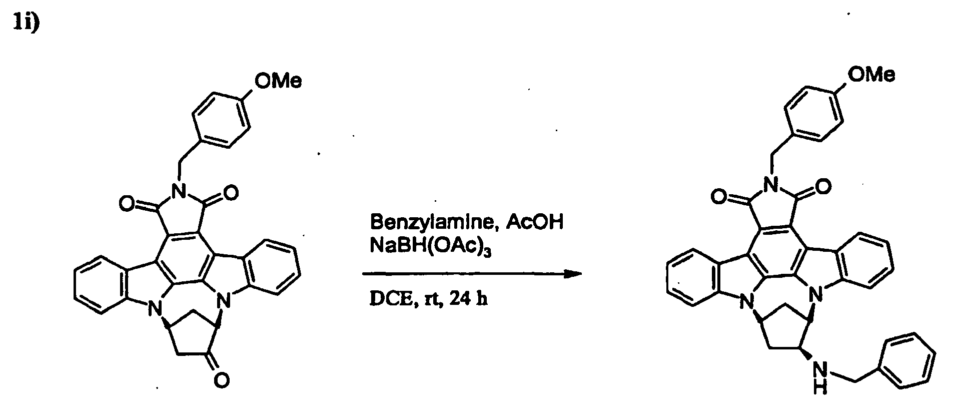

A solution of 1h (530 mg, 1 mmol) in 1,2-dichloroethane (15 mL) was treated dropwise with benzylamine (110 µl, 1 mmol) and AcOH (60 µl, 1 mmol). The solution was stirred for one hour at room temperature and sodium triacetoxyborohydride (320 mg, 1.5 mmol) was added portionwise. The reaction mixture was stirred for additional 24 hours and was poured onto ice cold NaOH (2M, 40 mL), extracted with EtOAc (2x30 mL), dried over magnesium sulfate, filtered and concentrated in vacuo. The crude was purified by flash chromatography (silica gel, CH₂Cl₂/EtOAc 9/1 as eluant mixture) to give the pure target compound as a yellow powder (460 mg, yield 74.7 %).

¹H-NMR (300 MHz, DMSO-d₆): δ 1.17 (1H, m, NH), 1.55 (1H, m, 5-CH₂. 2.65 (1H, bd, 2-CH₂), 2.92 (2H, m, 5-CH₂+ 2-CH₂), 3.54 (2H, d, CH₂-NH), 3.62 (1H, m, 4-CH-NH), 3.69 (3H, s, OCH₃), 4.80 (2H, s, CH₂-NCO), 5.65 (2H, m, 1-CH-N + 3-CH-N), 6.90-6.95 (4H, d, Harom), 7.01-7.11 (3H, m, Harom), 7.32-7.41 (4H, bt, Harom), 7.55-7.65 (2H, m, Harom), 7.85-7.90 (1H,d, Harom), 800-8.05 (1H, d, Harom), 9.00 (1H, d, indole H-4), 9.10 (1H, d, indole H-4). MS (ESI) m/z 617 [M+H]⁺.

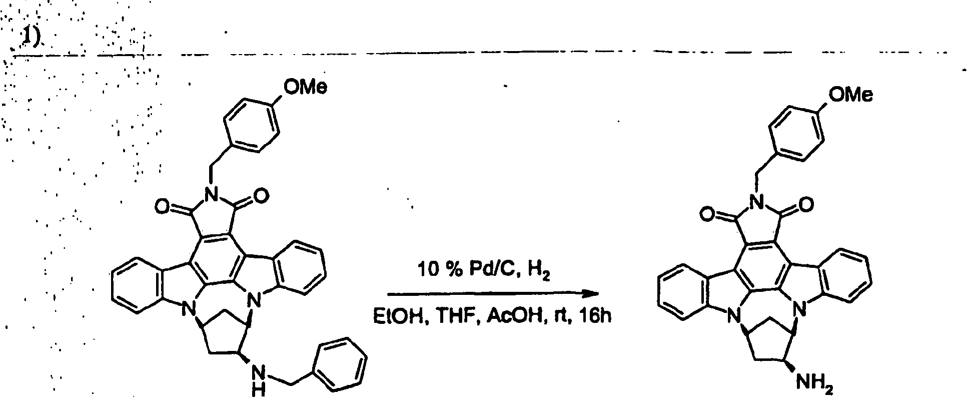

AcOH (0.5 mL) and Pd/C 10 % (50 mg) were added to a suspension of li (380 mg, 0.61 mmol) in EtOH (20 mL) and THF (5 mL). The mixture was hydrogenated at atmospheric pressure and rt overnight. The catalyst was filtered off and the solution was poured onto 1M NaOH (50 mL), extracted with EtOAc (2x50 mL), dried over sodium sulfate, filtered and concentrated in vacuo. The crude was purified by two successive preparative TLCs (CH₂Cl₂/methanol 95/5 as eluant mixture) to yield the pure target compound (40 mg, yield 12.5 %).

¹H-NMR (300 MHz, DMSO-d₆): δ 1.15 (1H, m, 5-CH₂, 1.26 (2H, bs, NH₂), 2.56 (1H, bd, 2-CH₂), 2.92 (2H, m, 5-CH₂+ 2-CH₂), 3.70 (3H, s, OCH₃), 3.83 (1H, m, 4-CH-NH₂), 4.84 (2H, s, CH₂-PMB), 5.36 (1H, bt, 3-CH-N), 5.67 (1H, bt, 1-CH-N), 6.90 (2H, d, Harom), 7.38 (4H, m, Harom), 7.60 (2H, bt, Harom), 7.87 (1H, d, Harom), 7.98 (1H,d, Harom), 9.02 (1H, d, indole H-4), 9.09 (1H, d, indole H-4). MS (ESI) m/z 527 [M+H]⁺.

### Example 2

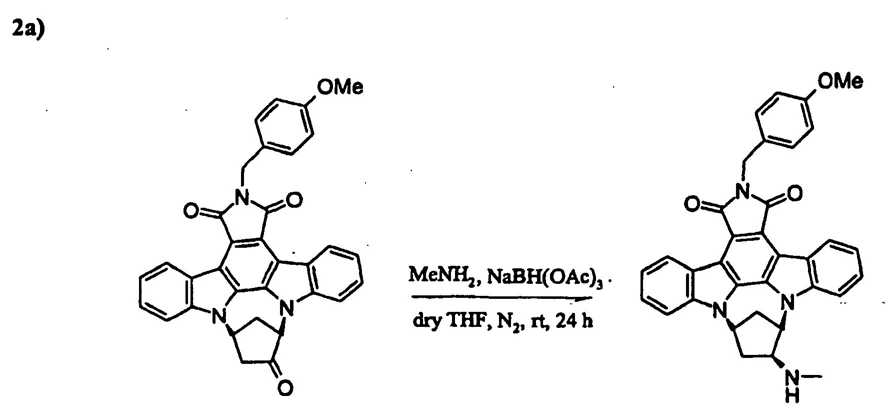

A solution of **1h** (530 mg, 1 mmol) in dry THF (10 mL) was treated dropwise with a solution of methylamine in THF (2M, 0.6 mL, 1.2 mmol). The solution was stirred for 2 hours at room temperature and sodium triacetoxyborohydride (320 mg, 1.5 mmol) was added. The reaction mixture was stirred for additional 24 hours and was poured onto ice cold NaOH (2M, 40 mL), extracted with EtOAc (2x30 mL), dried over magnesium sulfate, filtered and concentrated in vacuo. The crude was purified by flash chromatography (silica gel, CH₂Cl₂/EtOAc 7/3 then CH₂Cl₂/methanol 95/5 as eluant mixtures) to give the pure target compound (210 mg, yield 38.9 %).

¹H-NMR (300 MHz, DMSO-*d*_{*6*}): δ 1.17 (2H, m, NH + 5-CH₂), 2.07 (3H, s, N-CH₃), 2.62 (1H, bs, 2-CH₂), 2.90-2.95 (2H, m, 2-CH₂ + 5-CH₂), 3.55 (1H, m, 4-CH-NH), 3.69 (3H, s, OCH₃), 4.80 (2H, s, CH₂-PMB), 5.55-5.70 (2H, dt, 1-CH-N + 3-CH-N). 6.90-6.95 (2H, d, Harom), 7.30-7.40 (4H, m, Harom), 7.55-7.65 (2H, bt, Harom), 7.85-7.95 (2H, m, Harom), 9.00 (1H, d, indole H-4), 9.10 (1H, d, indole H-4). MS (ESI) m/z 541 [M+H]⁺.

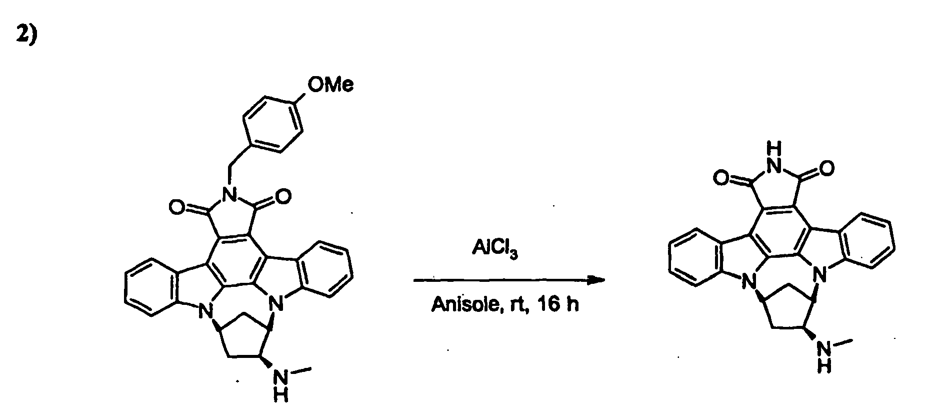

Aluminium chloride (200 mg, 1.5 mmol) was added at room temperature to a solution of 2a (82 mg, 0.15 mmol) in anisole (5 mL). The suspension was stirred overnight and was quenched with 1/1 ice/water (20 mL) and saturated NaHCO₃ (15 mL). The mixture was extracted with EtOAc (2x20 mL), washed with brine (15 mL), dried over sodium sulfate, filtered and concentrated in vacuo. MeOH (10 mL) was added to the residue and the precipitate was filtered to give the pure target compound (44 mg, yield 70.0 %).

¹H-NMR (300 MHz, DMSO-*d*_{*6*}): δ 1.13-1.20 (2H, m, NH + 5-CH₂), 2.09 (3H, s, N-CH₃), 2.62 (1H, bd, 2-CH₂), 2.89-2.98 (2H, m, 5-CH₂+ 2-CH₂), 3.53 (1H, m, 4-CH-NH), 5.59 (1H, bt, 3-CH-N), 5.70 (1H, bt, 1-CH-N), 7.37 (2H, t, Harom), 7.60 (2H, bt, Harom), 7.92 (2H, m, Harom), 9.00.(1H, d, indole H-4), 9.10 (1H, d, indole H-4), 11.05 (1H, s, imide NH). MS (ESI) m/z 421 [M+H]⁺

### Example 3

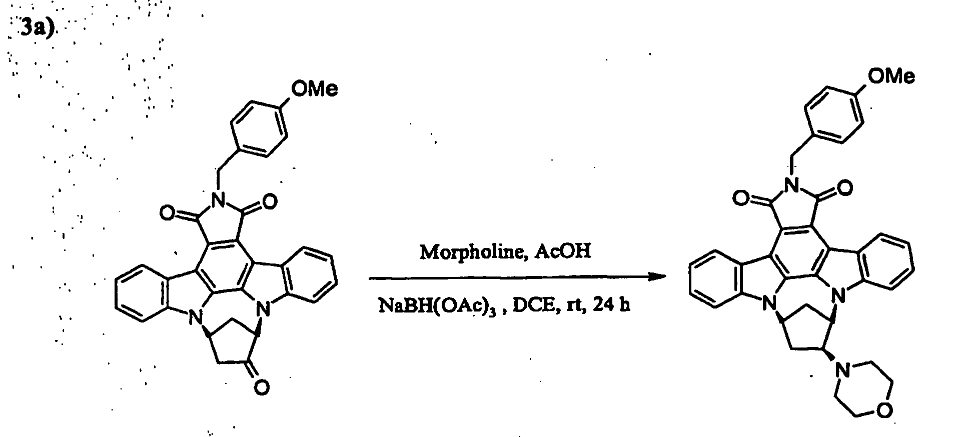

A solution of compound **1h** (200 mg, 0.38 mmol) in 1,2-dichloroethane (10 mL) was treated dropwise with morpholine (35 µL, 0.38 mmol) and acetic acid (25 µL, 0.38 mmol). The solution was stirred for one hour at room temperature and sodium triacetoxyborohydride (121 mg, 0.57 mmol) was added. The mixture was stirred for additional 24 hours and was poured onto ice-cold NaOH (1N, 40 mL), extracted with EtOAc (2x30 mL), dried over magnesium sulfate, filtered and concentrated in vacuo. The crude was purified by flash chromatography (silica gel, CH₂Cl₂/EtOAc 8/2 as eluant mixture) to give the pure target compound as a yellow powder (140 mg, yield 61.9 %).

¹H-NMR (300 MHz, DMSO-d_{*6*}): δ 1.44 (1H, m, 5-CH₂), 1.86 (2H, m, N-CH₂morpholine), 2.45 (2H, m, N-CH₂ morpholine), 2.53 (1H, bd, 2-CH₂), 2.71(1H, m, 5-CH₂), 2.90 (1H, m, 2-CH₂), 2.97 (4H, m, O-CH₂ morpholine), 3.36 (1H, m, 4-CH-N), 3.70 (3H,s , OCH₃), 4.83 (2H, s, CH₂₋PMB), 5.66 (1H, bt, 3-CH-N), 5.75 (1H, bt, 1-CH-N), 6.92 (2H, d, Harom), 7.38 (4H, m, Harom), 7.62 (2H, bt, Harem), 7.90 (1H, d, Harom), 7.97 (1H,d, Harom), 9.03 (1H, d, indole H-4), 9.09 (1H, d, indole H-4). MS (ESI) m/z 597 [M+H]⁺.

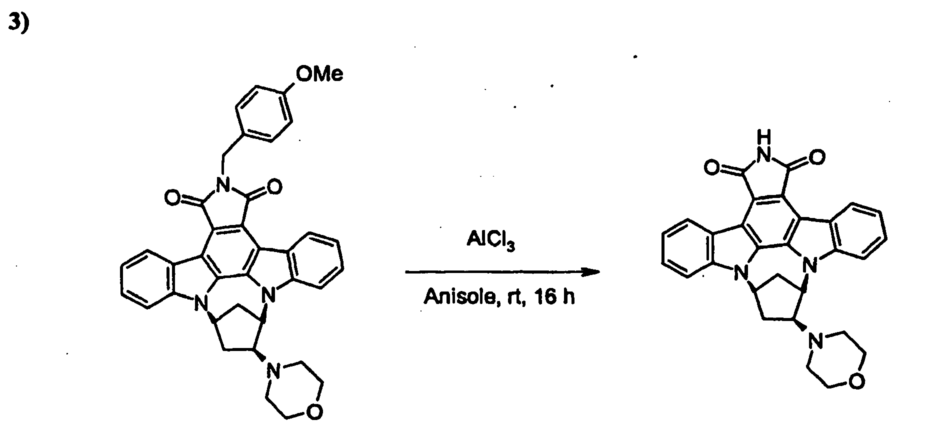

Aluminium chloride (200 mg, 1.5 mmol) was added at room temperature to a solution of 3a (90 mg, 0.15 mmol) in anisole (5 mL). The suspension was stirred overnight and was quenched with 1/1 ice/water (20 mL) and saturated NaHCO₃ (15 mL). The mixture was extracted with EtOAc (2x20 mL), washed with brine (15 mL), dried over sodium sulfate, filtered and concentrated in vacuo. MeOH (10 mL) was added to the residue and the precipitate was filtered to give the pure target compound (50 mg, yield 70.0 %).

¹H-NMR (300 MHz, DMSO-*d*_{*6*}): δ 1.46 (1H, m, 5-CH₂), 1.88 (2H, m, CH₂ morpholine), 2.47 (2H, m, CH₂ morpholine), 2.58 (1H, bd, 2-CH₂), 2.68 (1H, m, 5-CH₂), 2.91 (1H, m, 2-CH₂), 2.98 (4H, m, CH₂ morpholine), 3.33 (1H, m, 4-CH-N), 5.66 (1H, bt, 3-CH-N), 5.76 (1H, bt, 1-CH-N), 7.37 (2H, m, Harom), 7.60 (2H, bt, Harom), 7.93 (2H, m, Harom), 9.07 (1H. d. indole H-4), 9.13 (1H, d, indole H-4), 11.08 (1H, bs, imide NH). MS (ESI) m/z 477 [M+H]⁺.

### Example 4

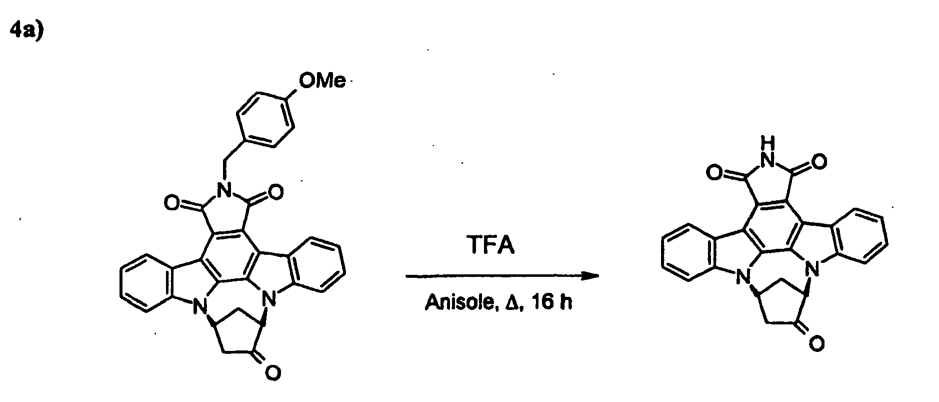

A solution of 1h (17.3 g, 32.9 mmol) in anisole (150 mL) and trifluoroacetic acid (200 mL) was refluxed overnight. The solvent mixture was concentrated, the residue was taken up with EtOH (200 mL) and triturated to give, after filtration, washings (EtOH and petroleum ether) and drying the pure title compound (11.73 g, yield 88.3 %).

¹H-NMR (300 MHz, DMSO-d₆): δ 2.95-3.05 (1H, dd, 2-CH₂), 3.20-3.25 (1H, dd, 2-CH₂), 3.40-3.55 (2H, m, 5-CH₂), 5.60 (1H, bd, 3-CH-N), 6.16 (1H, bt, 1-CH-N), 7.43 (2H, m, Harom), 7.64 (2H, bt, Harom), 7.95 (2H, m, Harom), 9.02-9.08 (2H, d, indole H-4), 11.14 (1H, bs, imide NH). MS (ESI) m/z 406 [M+H]⁺.

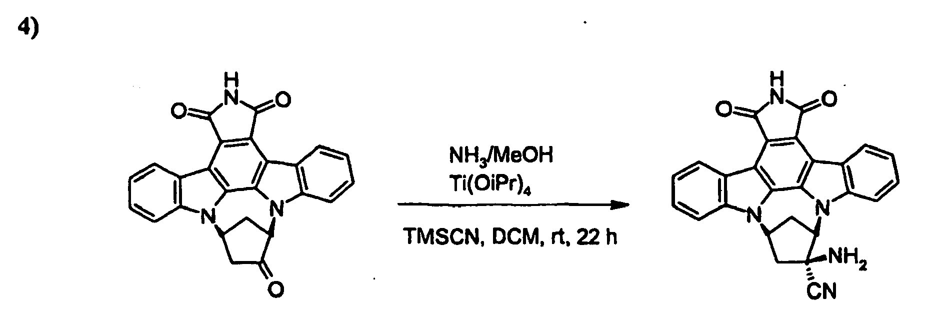

Methanolic ammonia (7N, 2 mL, 14 mmol) and titanium (IV) isopropoxide (0.5 mL, 1.70 mmol) were added at room temperature to a suspension of 4a (570 mg, 1.4 mmol) in 1,2-dichloroethane (10 mL). The solution was stirred for 3 hours then trimethylsilyl cyanide (0.18 mL, 1.40 mmol) was added and the reaction mixture was allowed to stir for 18 hours. After addition of 2. drops of water, the solid was removed by filtration over a celite layer and the solvent concentrated in vacuo. Purification by flash chromatography (silica gel, CH₂Cl₂/EtOAc 95/5 as eluant mixture) gave the pure target compound (310 mg, yield 51.1 %).

¹H-NMR (300 MHz, DMSO-d₆): δ 1.60 (1H, bd, 5-CH₂), 2.35 (2H, bs, NH₂), 2.75 (1H, m, 2-CH₂), 3.37 (2H, m, 2-CH₂ + 5-CH₂), 5.77 (1H, d, 3-CH-N), 5.92 (1H, bt, 1-CH-N), 7.50 (2H, m, Harom), 7.67 (2H,-bt, Harom), 7.89 (1H, d, Harom-, 8.03-8.07 (1H, m, Harom), 9.00 (1H, d, indole H-4), 9.08 (1H, d, indole H-4), 11.05 (1H, s, NH imide). MS (ESI) m/z 432 [M+H]⁺.

### Example 5

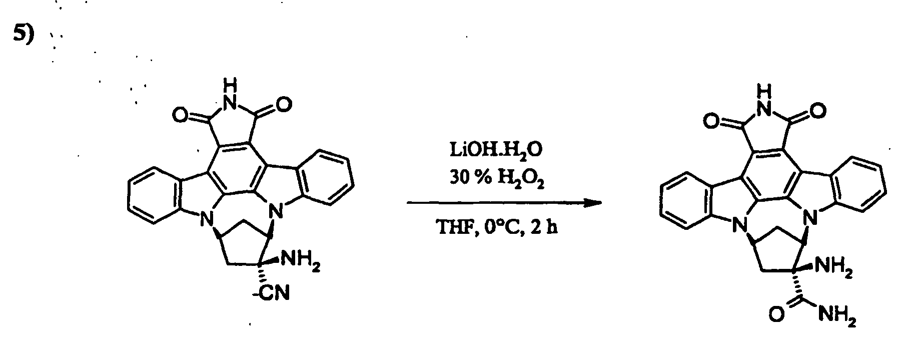

Lithium hydroxide monohydrate (30 mg, 0.69 mmol) and hydrogen peroxide (30%, 0.1 mL) were added at 0°C to a solution of 4 (200 mg, 0.46 mmol) in THF (10 mL). The solution was stirred at 0°C for 1 hour and additional lithium hydroxide monohydrate (30 mg, 0.69 mmol) and water (3 mL) were added. After 1 hour stirring, the media was concentrated to a third and poured into 1/1 CH₂Cl₂/water (50 mL). The reaction mixture was filtered, the orange precipitate was washed with CH₂Cl₂ and dried in vacuo to yield the pure target compound (120 mg, yield 58.3 %).

¹H-NMR (300 MHz, DMSO-*d*_{*6*}): δ 1.54 (1H, bd, 5-CH₂), 2.65 (1H, bd, 2-CH₂), 3.15 (2H, m, 2-CH₂+ 5-CH₂), 5.49 (1H, d, 3-CH-N), 5.80 (1H, bt, 1-CH-N), 7.15 (1H, bs, CONH₂), 7.50 (2H, m, Harom), 7,60 (1H, bs, CONH₂), 7.68 (2H, bt, Harom) 7.85 (1H. d, Harom), 7.93 (1H, m, Harom), 9.05 (2H, bt, indole H-4), 10.8 (1H, bs, NH imide). MS (ESI) m/z 451 [M+H]⁺.

### Example 6

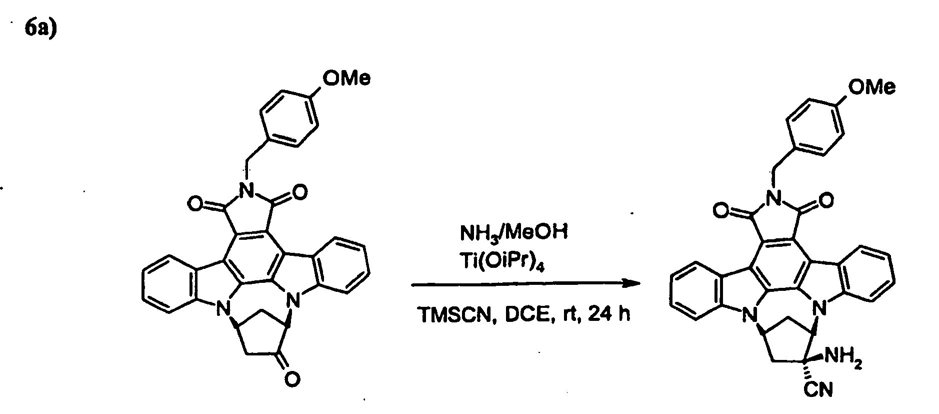

Methanolic ammonia (7M, 0.7 mL, 5 mmol) and titanium (IV) isopropoxide (180 µL, 0.6 mmol) were added to a stirred solution of compound 1h (260 mg, 0.5 mmol) in 1,2-dichloroethane (10 mL) at room temperature under nitrogen. After 4 hours trimethylsilyl cyanide (63 µL, 0.5 mmol) was added and the solution was stirred overnight. The mixture was then quenched with water (10 mL) and extracted with EtOAc (2x20 mL). The combined organic phases were washed with saturated ammonium chloride (15 mL), dried over sodium sulfate, filtered and concentrated in vacuo. The crude was further purified by flash chromatography (silica gel, petroleum ether/EtOAc 4/2 as eluant mixture) to give the pure target compound as a yellow powder (160 mg, yield 58.1 %)

¹H-NMR (300 MHz, DMSO-*d*_{*6*}): δ 1.63 (1H, bd, 5-CH₂), 2.40 (2H, s, NH₂), 2.87 (1H, bd, 2-CH₂), 3.25 (2H, m, 2-CH₂+ 5-CH₂), 3.69 (3H, s, OCH₃), 4.80 (2H, s, CH₂-PMB), 5.78 (1H, bd, 3-CH-N), 5.87 (1H, bt, 1-CH-N), 6.89 (2H, d, Harom), 7.50 (4H, m, Harom), 7.68 (2H, bt, Harom), 7.89 (1H, d, Harom), 8.05 (1H, m, Harom), 9.00 (1H, d, indole H-4), 9.08 (1H, d, indole H-4). MS (ESI) m/z 552 [M+H]⁺.

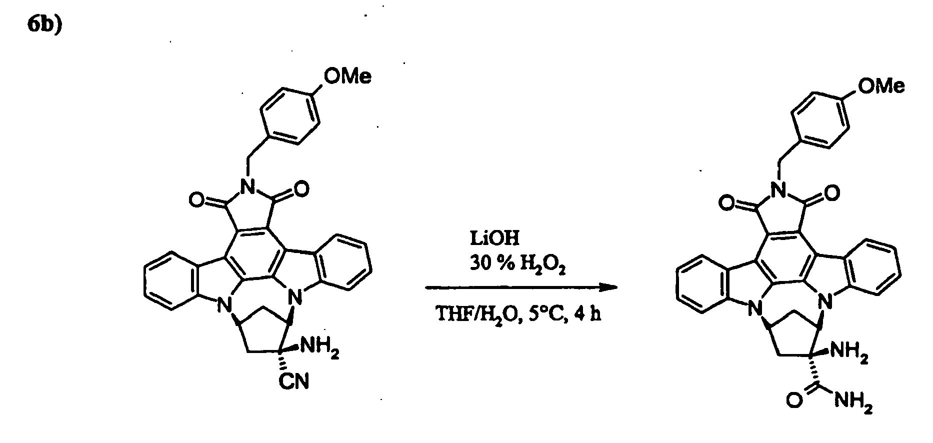

Lithium hydroxide monohydrate (0.6 g, 15 mmol), hydrogen peroxide (30%, 5 mL) and water (20 mL) were added at 5°C to a solution of 6a (1.6 g, 2.90 mmol) in THF (100 mL). The solution was stirred at 5°C for 4 hours and water (25 mL) was added. The reaction mixture was filtered, the orange precipitate was washed, dissolved in CH₂Cl₂ (50 mL) and washed with water (25 mL). The organic layer was dried over sodium sulfate, filtered and-concentrated in vacuo to yield the pure target compound as a yellow powder (1.35 g, yield 81.8 %).

¹H-NMR (300 MHz, DMSO-d_{*6*}): δ 1.40 (2H, bs, NH₂), 1.45 (1H, d, 2-CH₂), 2.65 (1H, bd, 2-CH₂),3.09-3.25 (2H, m, 5-CH₂), 3.70 (3H, s, OCH₃), 4.81 (2H, s, CH₂-PMB), 5.48 (1H, bd, 3-CH-N), 5.78 (1H, bt, 1-CH-N), 6.88 (2H, m, Harom), 7.30-7.42 (5H, m, CONH₂ + Harom), 7.58-7.65 (3H, m, CONH₂+Harom), 7.76 (1H, d, Harom), 7.92 (1H, d, Harom), 9.05 (2H, dd, Harom). MS (ESI) m/z 570 [M+H]⁺.

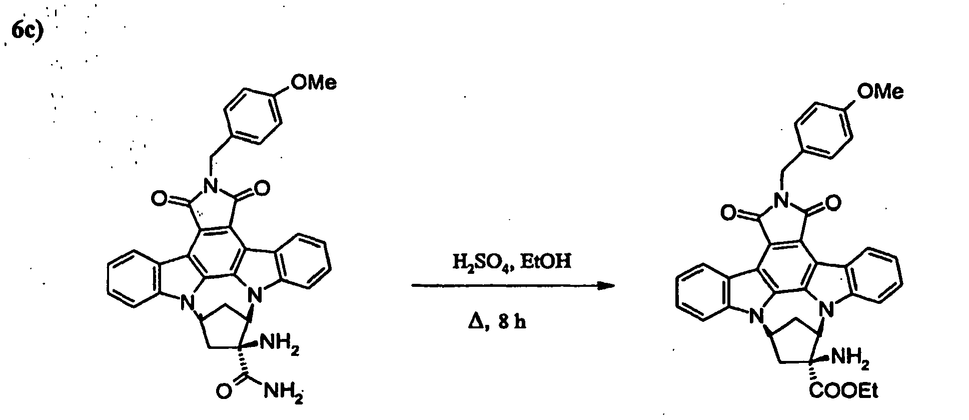

Concentrated sulfuric acid (2 mL) was added to a solution of 6b (300 mg, 0.52 mmol) in EtOH (10 mL) and the mixture was refluxed for 8 hours. After cooling, the reaction mixture was poured into water (50 mL) and extracted with EtOAc (2x70 mL). The combined organic layers were dried over sodium sulfate, filtered and concentrated in vacuo. Purification by flash chromatography (silica gel, CH₂Cl₂ /EtOAc 95/5 as eluant mixture) gave the pure target compound as a yellow powder (170 mg, yield 54.7 %).

¹H-NMR (300 MHz, DMSO-*d*_{*6*}): δ 1.35 (3H, t, COOCH₂-CH₃), 1.48 (2H, bs, NH₂), 1.55 (1H, d, 2-CH₂), 2.75 (1H, bd, 2-CH₂), 3.02 (1H, m, 5-CH₂), 3.20 (1H, m, 5-CH₂), 3.70 (3H, s, OCH₃), 4.30 (2H, q, COOCH₂-CH₃), 4.85 (2H, s, CH₂-PMB), 5.65 (1H, bd, 3-CH-N), 5.75-5.80 (1H, bt, 1-CH-N), 6.85-6.90 (2H, m, Harom), 7.35-7.45 (4H, m, Harom), 7.60-7.70 (2H, bt, Harom) 7.80-7.85 (1H, d, Harom), 7.90-7.95 (1H, m, Harom), 9.05 (2H, dd, indole H-4). MS (ESI) m/z 599 [M+H]⁺.

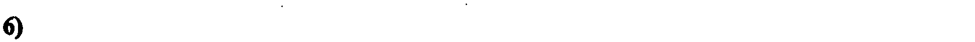

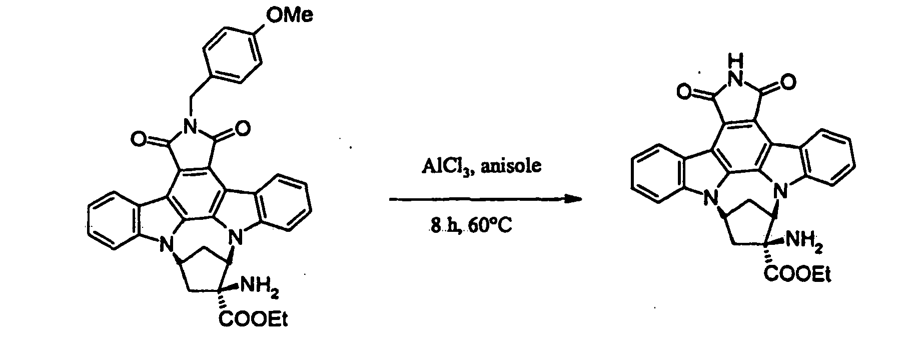

Aluminium chloride (100 mg, 2.3 mmol) was added at room temperature to a solution of 6c (140 mg, 0.23 mmol) in anisole (10 mL). The suspension was stirred for 8 hours at 60°C. The reaction mixture was cooled and poured onto water (30 mL), extracted with EtOAc (2x30 mL), dried over sodium sulfate, filtered and concentrated in vacuo. Purification by preparative TLC (silica gel, CH₂Cl₂ /EtOAc 95/5 as eluant mixture) gave the pure target compound as a yellow powder (42 mg, yield 38.2 %).

¹H-NMR (300 MHz, DMSO-*d*_{*6*}): δ 1.36 (3H, t, COOCH₂-CH₃), 1.48 (2H, bs, NH₂), 1.60 (1H, bd, 5-CH₂), 2.72 (1H, bd, 2-CH₂), 3.05 (1H, m, 5-CH₂), 3.25 (1H, m, 2-CH₂), 4.25 (2H, q, COOCH₂-CH₃), 5.65 (1H, bd, 3-CH-N), 5.85 (1H, bt, 1-CH-N), 7.40 (2H, m, Harom), 7.65 (2H, q, Harom) 7.77 (1H, d, Harom), 7.92 (1H, m, Harom), 9.05 (2H, bt, indole H-4), 11.06 (1H, s, NH imide). MS (ESI) m/z 479 [M+H]⁺.

### Example 7

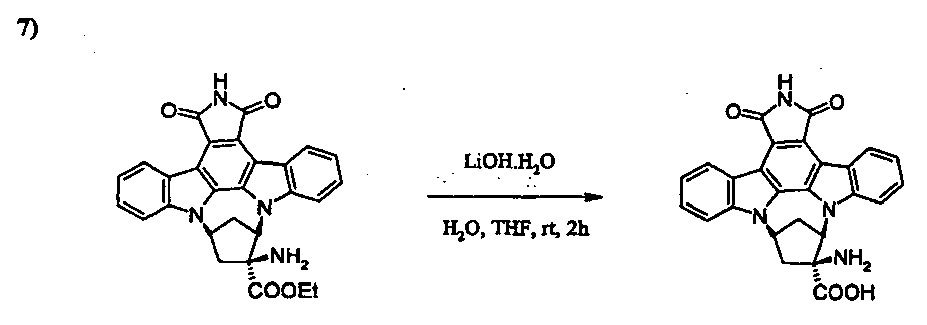

A solution of lithium hydroxide monohydrate (35 mg, 0.84 mmol) dissolved in water (1 mL) was added dropwise to a stirred solution of 6 (200 mg, 0.42 mmol) in THF (10 mL). The solution was stirred for 2 hours at room temperature, then THF was removed in vacuo. The aqueous solution was then neutralised with HCl and the precipitate was filtered and dried in vacuo at 60°C to give the pure target compound as an orange powder (140 mg, yield 73.7 %).

¹H-NMR (300 MHz, CD₃OH): δ 1.45-1.50 (1H, bd, 5-CH₂). 2.58 (1H, bd, 2-CH₂)' 3.05 (2H, m, 2-CH₂ + 5-CH₂), 5.50 (1H, bd, 3-CH-N), 5.80 (1H, bt, 1-CH-N), 7.40 (2H, m, Harom) , 7.65 (2H, q, Harom) , 7.75-7.80 (1H, d, Harom) , 7.90-7.95 (1H, m, Harom ), 9.05 (2H, bt, indole H-4), 11.06 (1H, s, NH imide). MS (ESI) m/z 451 [M+H]⁺.

### Example 8

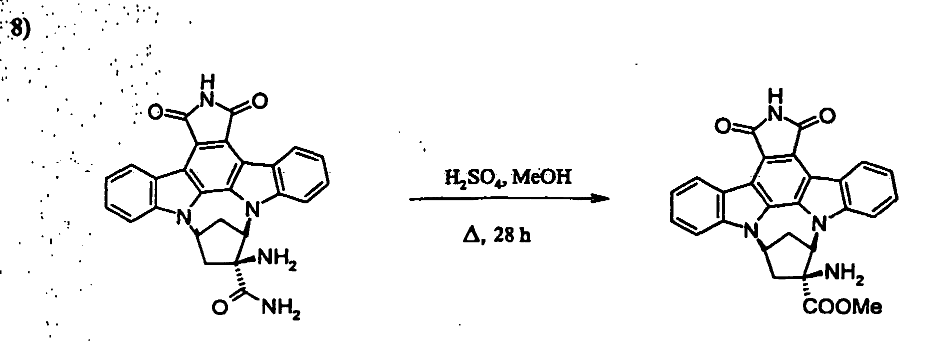

Concentrated sulfuric acid (10 mL) was added to a solution of 5 (2.25g, 5 mmol) in MeOH (100 mL) and the mixture was refluxed for 28 hours. The reaction mixture was cooled to 0°C and the precipitate was filtered, dissolved in EtOAc (100 mL) and washed with saturated NaHCO₃ (50 mL). The organic layer was dried over sodium sulfate, filtered and concentrated in vacuo. Purification by flash chromatography (silica gel, CH₂Cl₂/MeOH 98/2 as eluant mixture) gave the pure target compound as an orange powder (1.20 g, yield 52.2 %).

¹H-NMR (300 MHz, CD₃OH): δ 1.50 (2H, bs, NH₂), 1.59 (1H, bd, 5-CH₂), 2.76 (1H, bd, 2-CH₂), 3.03 (1H, m, 5-CH₂), 3.24 (1H, m, 2-CH₂), 3.86 (3H, s, COOMe), 5.67 (1H, bd, 3-CH-N), 5.82 (1H, bt, 1-CH-N), 7.40 (2H, m, Harom), 7.65 (2H, q, Harom) 7.80 (1H, d, Harom), 7.92 (1H, m, Harom), 9.05 (2H, bt, indole H-4), 11.00 (1H, s, NH imide). MS (ESI) m/z 465 [M+H]⁺.

### Example 9

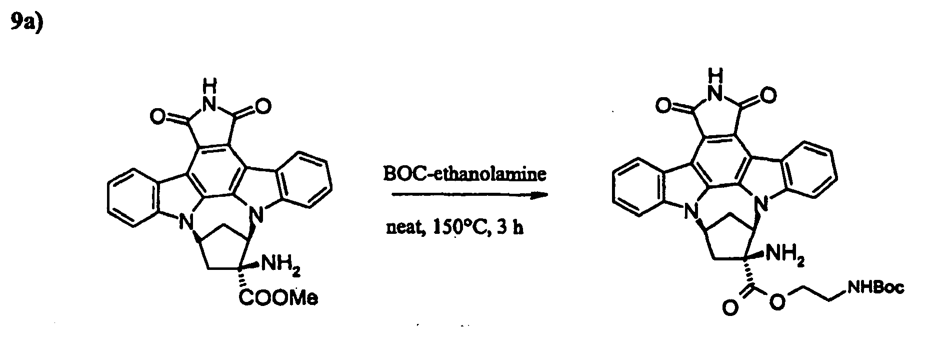

N-Boc-ethanolamine (1 mL, large excess) was added to 8 (150 mg, 0.32 mmol). The reaction mixture was heated at 150°C for 3 hours. After cooling, the crude was purified by flash chromatography (silica gel, CH₂Cl₂/acetone 9/1 as eluant mixture) to give the pure target compound (60 mg, yield 32.0 %).

¹H-NMR (300 MHz, DMSO-*d*_{*6*}): δ 1.24 (1H, bd, 5-CH₂), 1.38 (9H, s, C(CH₃)), 1.55 (2H, bs, NH₂), 2.74 (1H, bd, 2-CH₂) , 3.10 (1H, m, 2-CH₂) , 3.25 (1H, m, 5-CH₂). 3.40 (2H, CH₂₋NHBoc), 4.25 (2H, t, CH₂-OCO), 5.68 (1H, bd, 3-CH-N), 5.80 (1H, bt, 1-CH-N), 7.21 (1H, bt, Boc-NH), 7.40 (2H, m, Harom), 7.65 (2H, m, Harom), 7.85 (2H, dd, Harom), 9.05 (2H, bt, indole H-4), 11.06 (1H,s, NH imide). MS (APCI) m/z 594 [M+H]⁺.

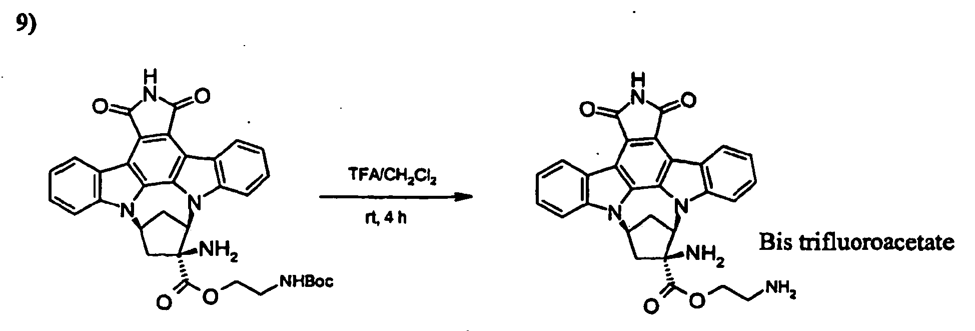

Trifluoroacetic acid (1 mL) was added to a stirred solution of 9a (60 mg, 0.1 mmol) in CH₂Cl₂ (10 mL). The reaction mixture was stirred at rt for 4 hours and the solvent was concentrated in vacuo. The residue was taken up in Et₂O (5 mL) and the yellow precipitate was filtered, dried in vacuo to yield the pure target compound (56 mg, yield 78.0 %).

¹H-NMR (300 MHz, DMSO-*d*_{*6*}): δ 1.85 (1H, bd, 5-CH₂. 2.82 (1H, bd, 2-CH₂), 3.20 (1H, m, 5-CH₂), 3.45 (2H, m, CH₂-NHBoc), 3.60 (1H, m, 2-CH₂), 4.55 (2H, t, CH₂-OCO), 5.90 (1H, bd, 3-CH-N), 6.10 (1H, bt, 1-CH-N), 7.40 (2H, m, Harom), 7.65 (2H, m, Harom), 7.85 (2H, dd, Harom), 7.8-8.6 (6H, m, NH₃⁺), 9.15 (2H, dd, indole H-4),11.06 (1H,s, NH imide). MS (APCI) m/z 494 [M+H]⁺.

### Example 10

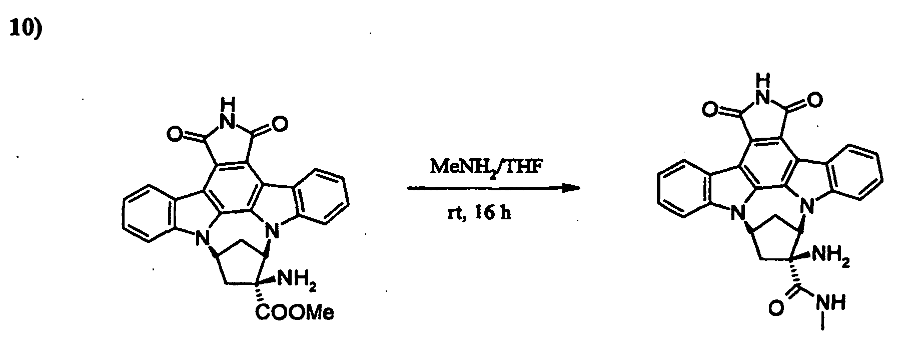

Methylamine (2M in THF, 10 mL, 20 mmol) was added to a stirred solution of 8 (205 mg, 0.54 mmol) in THF (6 mL). The sealed flask was stirred overnight at room temperature, then the solvent was eliminated in vacuo. Purification by flash chromatography (silica gel, CH₂Cl₂/MeOH/NH₄OH 95/4.5/0.5 as eluant mixture) gave the pure target compound (55 mg, yield 21.9%).

¹H-NMR (300 MHz, DMSO-d₆): δ 1.37 (2H, bs, NH₂), 1.55 (1H, bd, 5-CH₂), 2.70 (1H, bd, 2-CH₂), 2.75 (3H, d, N-CH₃), 3.10 (1H,m, 5-CH₂), 3.27 (1H, m, 2-CH₂) 5.45 (1H, bd, 3-CH-N), 5.80 (1H, bt, 1-CH-N), 7.40 (2H, m, Harom), 7.65 (2H, q, Harom) 7.77 (1H, d, Harom), 7.92 (1H, m, Harom), 8.12 (1H, m, NH amide), 9.05 (2H, bt, indole H-4), 11.06 (1H, s, NH imide). MS (ESI) m/z 464 [M+H]⁺.

### Example 11

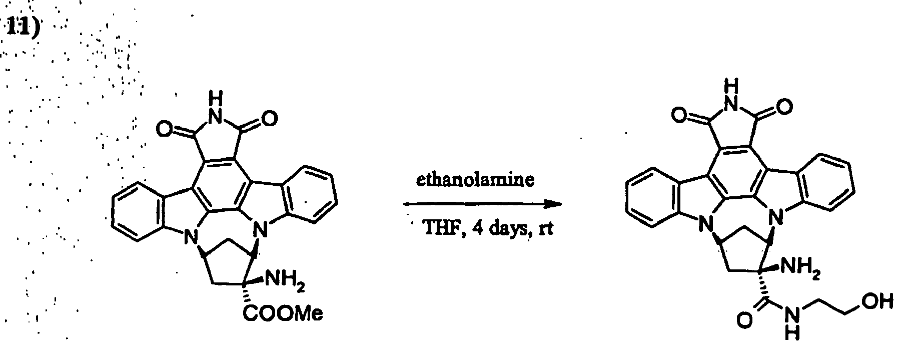

Ethanolamine (20 µL, 0.51 mmol) was added to a solution of 8 (240 mg, 0.51 mmol) in THF (10 mL). The reaction mixture was stirred for 4 days at room temperature. The solvent was concentrated in vacuo then purification by flash chromatography (silica gel, CH₂Cl₂/MeOH/NH₄OH 95/4.5/0.5 as eluant mixture) gave the pure target compound (75 mg, yield 30.0 %).

¹H-NMR (300 MHz, DMSO-*d*_{*6*}): δ 1.40 (2H, bs, NH₂), 1.55 (1H, bd, 5-CH₂), 2.68 (1H, bd, 2-CH₂), 3.10 (1H, m, 5-CH₂), 3.25 (3H, m, 2-CH₂ + N-CH₂-CH₂-OH), 3.55 (2H, m, N-CH₂-CH₂₋OH), 4.75 (1H, t, OH), 5.45 (1H, bd, 3-CH-N), 5.80 (1H, bt, 1-CH-N), 7.40 (2H, m, Harom) , 7.70 (2H, q, Harom) , 7.77 (1H, d, Harom) , 7.92 (1H, m, Harom), 8.10 (1H, bt, NH amide), 9.05 (2H, bt, indole H-4). 11.07 (1H, s, NH imide). MS (ESI) m/z 494 [M+H]⁺.

### Example 12

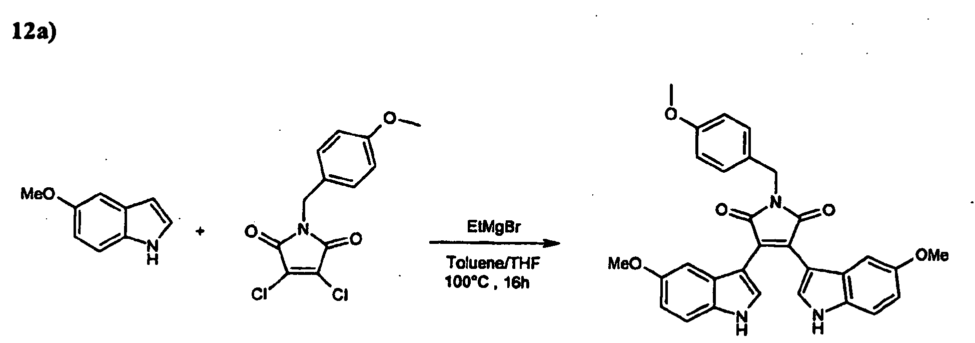

Ethylmagnesium bromide (3 M in Et₂O, 60 mL, 176 mmol) was added dropwise under stirring to a solution of 5-methoxyindole (25.9 g, 176 mmol) in toluene (600 mL) and THF (60 mL) under nitrogen atmosphere. The solution was stirred for 1 hour at room temperature before adding a solution of compound 1b (22.9 g, 80 mmol) in toluene/THF (10/1, 220 mL) within 1 hour. The reaction mixture was then refluxed overnight, cooled to room temperature and a saturated solution of NH₄Cl (500 mL) was added. The mixture was extracted with EtOAc (500 mL) and the organic layer was dried over sodium sulfate, filtered and concentrated in vacuo. Purification by flash chromatography (silica gel, EtOAc/petroleum ether 1/5 to 1/1) afforded the expected compound (29.15 g, yield 72.0 %)

¹H-NMR (300 MHz, DMSO-*d*_{*6*}): δ 3.06 (6H, s, indole OCH₃), 3.72 (3H, s, PMB OCH₃), 4.69 (2H, s, CH₂-PMB), 6.19 (2H, s, Harom ), 6.56 (2H, dd, Harom), 6.92 (2H, d, Harom), 7.31 (4H, m, Harom), 7.89 (2H, s, indole H-2), 11.60 (2H, bs, indole NH). MS (APCI) m/z 508 [M+H]⁺.

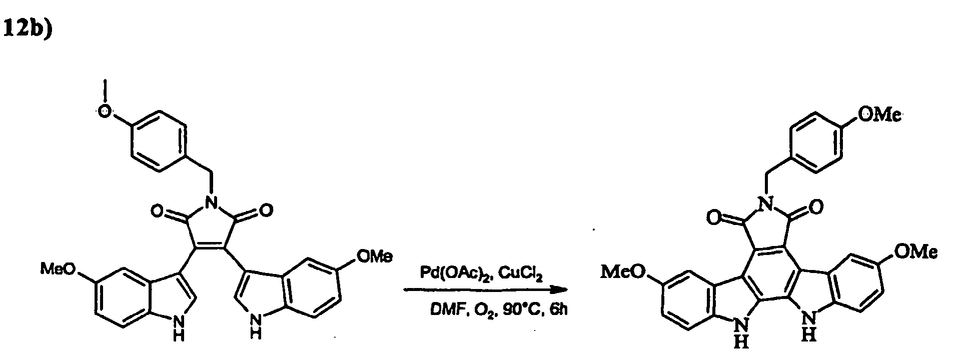

Palladium diacetate (0.55 g, catalytic) was added to a solution of 12a (25.37 g, 50 mmol) in DMF (200 mL). The reaction mixture was stirred for 10 minutes at rt and CuCl₂ (6.60 g, 50 mmol) was added. The reaction mixture was then heated at 90°C under oxygen atmosphere for 6 hours and overnight at rt. The solvent was concentrated in vacuo, the residue was taken in EtOAc (200 mL) and washed with diluted NH₄OH (100 mL). The organic layer was dried over sodium sulfate, filtered and concentrated in vacuo. The residue was purified by flash chromatography,(silica gel, EtOAc/petroleum ether 1/2) and gave the pure expected compound (3.5 g, yield 14.1 %).

¹H-NMR (300 MHz, DMSO-d_{*6*}):δ 3.65 (3H, s, indole OCH₃), 3.85 (6H, s, PMB OCH₃), 4.85 (2H, s, CH₂-PMB), 6.85 (2H, d, Harom), 7.20 (2H, d, Harom), 7.35 (2H, d, Harom), 7.70 (2H, dd, Harem), 8.98 (2H, s, Harom), 11.71 (2H, bs, indole NH). MS (APCI) m/z 506 [M+H]⁺.

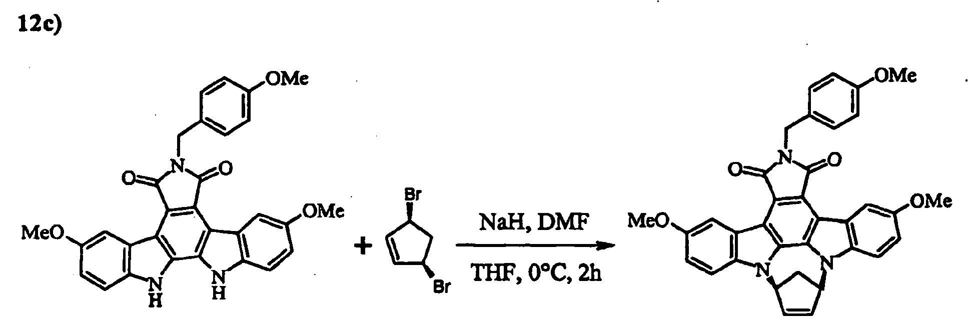

Sodium hydride (60% in mineral oil, 0.26 g, 6.43 mmol) was added to a solution of 12b (1.30 g, 2.57 mmol) in THF (50 mL) at 0°C. The reaction mixture was stirred for 2 hours at 0°C and a solution of 1a (0.70 g, 3.08 mmol) in THF (10 mL) was added dropwise within 30 min. After 1 hour sodium hydride (60% in mineral oil, 0.2 g, 5 mmol) was added and the reaction mixture was stirred at 0°C for additional 30 minutes. The solvent was removed in vacuo and the residue was dissolved in EtOAc (100 mL) and washed with water (2x50 mL). The organic layer was dried over sodium sulfate, filtered and concentrated in vacuo. The crude target compound was used directly in the next step without further characterization (1.35 g, theoretical yield 92 %).

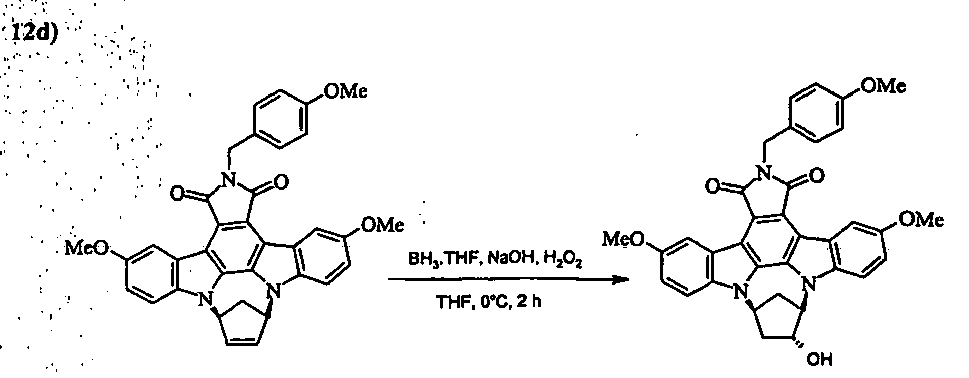

Borane (1M in THF, 11.4 mL, 11.4 mmol) was added at 0°C to a solution of **12c** (1.30 g, 2.28 mmol) in THF (50 mL). The mixture was stirred for 1 hour at 0°C and NaOH (32%, 1.2 mL) was added carefully, then H₂O₂ (30%, 4 mL) was added dropwise within 30 min. The mixture was stirred for 2 hours at 0°C, then the insoluble salts were filtered off and the solvent was removed in vacuo. The residue was dissolved in EtOAc (60 mL), washed with water (50 mL) and brine (50 mL) and dried over sodium sulfate. The solvent was removed in vacuo and the residue purified by flash chromatography (silicagel, EtOAc/petroleum ether 7/3 as eluant mixture) to give the pure target compound (330 mg, yield 24.5 %).

¹H-NMR (300 MHz, DMSO-d₆): δ 1.95 (1H, m, 5-CH₂), 2.32 (1H, m, 5-CH₂), 2.65 (1H, bd, 2-CH₂), 3.15 (1H, m, 2-CH₂), 3.35 (3H, s, PMB OCH₃), 3.90 (6H, s, indole OCH₃), 4.16 (1H, m, 4-CH-OH), 4.82 (2H, s, CH₂ -PMB), 5.28 (1H, d, 3-CH-N), 5,62 (1H, d, OH), 5.85 (1H, m, 1-CH-N), 6.91 (2H, d, Harom), 7.30 (4H, m, Harom), 7.82 (2H, m, Harom), 8.55 (1H, d, Harom), 8.60 (1H, d, Harom). MS (APCI) m/z 588 [M+H]⁺.

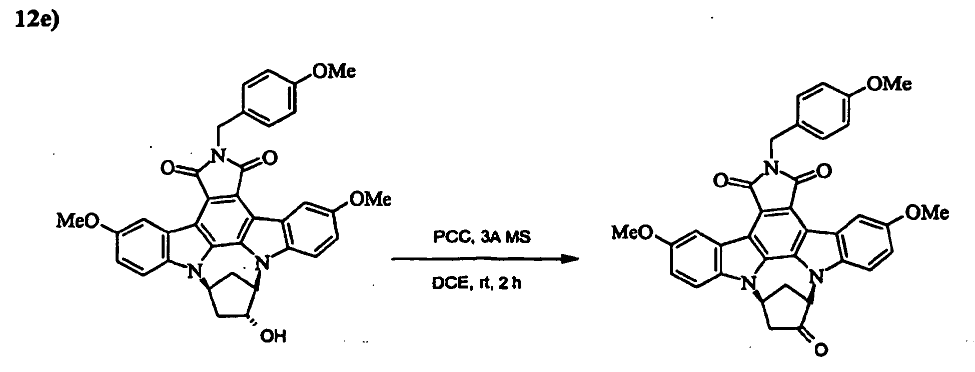

Pyridinium chlorochromate (0.70 g, 3.30 mmol) was added portionwise to a suspension of **12d** (26.5 g, 50 mmol) and 3Å molecular sieves (1g) in 1,2-dichloroethane (1 L). The reaction mixture was stirred at room temperature for 2 hours and the solvent was removed in vacuo. Purification by flash chromatography (silica gel, EtOAc/CH₂Cl₂ 1/9 as eluant mixture) gave the pure target compound as an orange powder (400 mg, yield 31.0 %).

¹H-NMR (300 MHz, DMSO-*d*_{*6*}): δ3.03 (1H, d, 2-CH₂), 3.25 (3H, m, 5-CH₂ + 2-CH₂), 3.70 (3H, s, PMB OCH₃), 3.91 (6H, s, indole OCH₃), 4.84 (2H, s, CH₂-PMB), 5.51 (1H, d, 3-CH-N), 6.08 (1H, bt, 1-CH-N), 6.90 (2H, d, Harom), 7.35 (4H, m, Harom), 7.90 (2H, dd, Harom), 8.50 (2H, s, Harom). MS (APCI) m/z 586 [M+H]⁺.

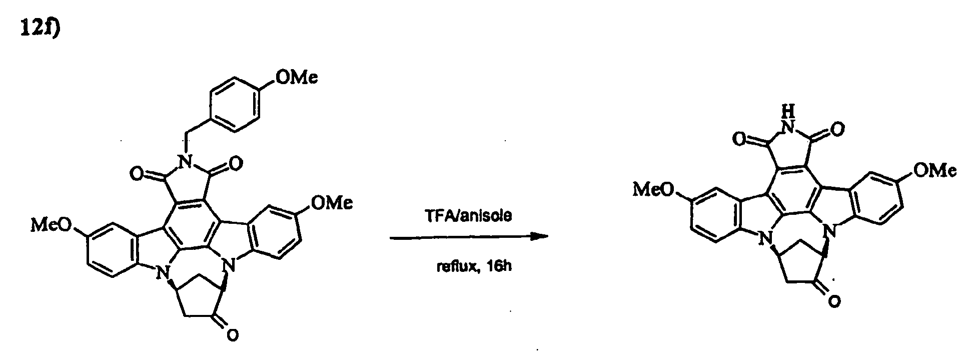

Trifluoroacetic acid (20 mL) was added to a suspension of **12e** (360 mg, 0.61 mmol) in anisole (10 mL). The reaction mixture was stirred at reflux overnight, then the solvent was removed in vacuo. EtOH (10 mL) was added, the precipitate was filtered off, washed with cold EtOH (2 mL) and petroleum ether (2 mL) and dried in vacuo to yield the target compound (280 mg, theoretical yield 100 %) which was used without further purification in the next step.

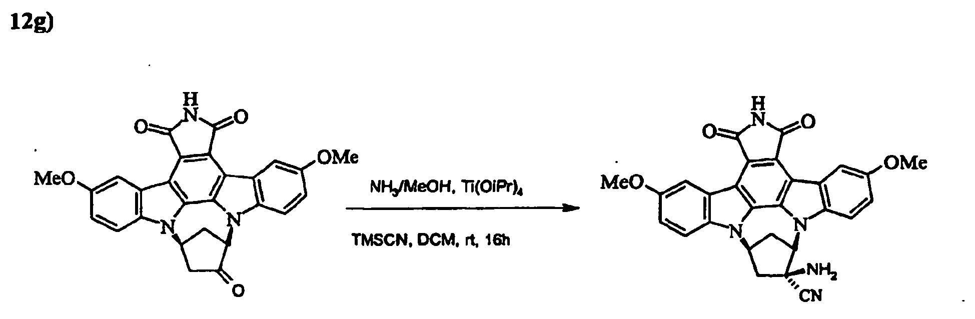

Methanolic ammonia (7M, 1 mL, 7 mmol) and titanium (IV) isopropoxide (200 µL, 0.72 mmol) were added to a stirred solution of compound 12f (280 mg, 0.6 mmol) in CH₂Cl₂ (10 mL) at room temperature under nitrogen atmosphere. After 2 hours trimethylsilyl cyanide (70 µL, 0.6 mmol) was added and the solution was stirred overnight. The reaction mixture was then quenched with water (10 mL) and extracted with EtOAc (2x20 mL). The combined organic phases were washed with saturated ammonium chloride (15 mL), dried over sodium sulfate, filtered and concentrated in vacuo. The crude residue (240 mg, theoretical yield 81.3 %) was used in the next step without any purification. '

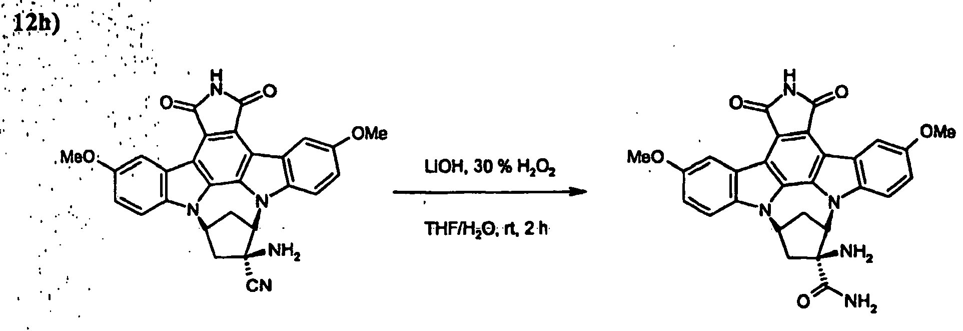

Lithium hydroxide monohydrate (250 mg, 4.9 mmol), hydrogen peroxide (30%, 2 mL) and water (10 mL) were added at rt to a solution of **12g** (240 mg, 0.49 mmol) in THF (20 mL). The solution was stirred at rt for 2 hours and water (25 mL) was added. The reaction mixture was filtered, the' orange precipitate was washed with water (2 mL) and dried in vacuo at 50°C to yield the pure target compound as a yellow powder (130 mg, yield 52.0 %).

¹H-NMR (300 MHz, DMSO-*d*_{*6*}): δ1.40-1.45 (3H, m, NH₂+ 2-CH₂), 2.65 (1H, bd, 2-CH₂), 3.10 (1H, m, 5-CH₂), 3.40-3.45 (1H, m, 5-CH₂), 3.90 (6H, s, OCH₃), 5.43 (1H, bd, 3-CH-N), 5.82 (1H, bt, 1-CH-N), 7.27 (2H, m, Harom) , 7.66 (2H, m, Harom), 7.82 (2H, m, Harom) 9.05 (2H, dd, Harom), 11.01 (1H, s, imide NH). MS (ESI) m/z 510 [M+H]⁺.

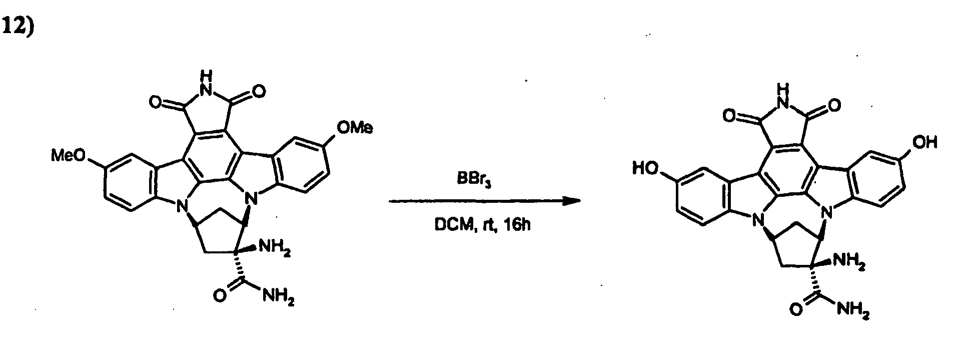

Boron tribromide (1M in CH₂Cl₂, 2.3 mL, 2.3 mmol) was added to a suspension of 12h (120 mg, 0.23 mmol) in CH₂Cl₂ (3 mL) and the mixture was stirred at rt overnight. The reaction mmixture was poured onto ice (10 mL) and extracted with EtOAc (2x20 mL). the aqueous layer was basified wih sat. NaHCO₃ (20 mL) and extracted with EtOAc (2x20 mL). The organic layer was dried over sodium sulfate, filtered and concentrated in vacuo. Purification by flash chromatography (silica gel, CH₂Cl₂/acetone 1/1 as eluant mixture) gave the pure expected compound (45 mg, yield 41.0 %).

¹H-NMR (300 MHz, DMSO-d₆): δ 1.40-1.45 (4H, m, NH₂+ 2-CH₂), 3.15 (2H, m, 5-CH₂), 5.43 (1H, bd, 3-CH-N), 5.80 (1H, bt, 1-CH-N), 7.27 (1H, m, Harom), 7.45 (2H, bs, CONH₂), 7.66 (1H, m, Harom), 8.86 (2H, d, Harom) 9.35 (2H, dd, Harom), 10.98 (1H, s, imide NH). MS (APCI) m/z 482 [M+H]⁺.

### Example 13

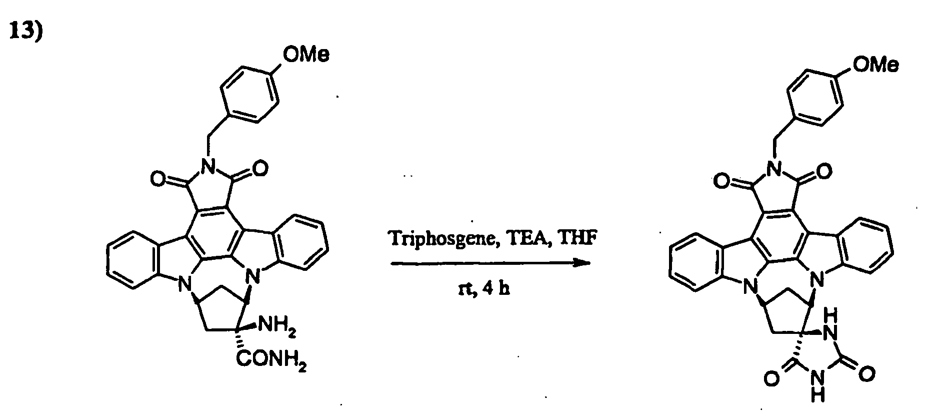

Triethylamine (0.28 mL, 2.1 mmol) and triphosgene (200 mg, 0.7 mmol) were added to a solution of **6b** (400 mg, 0.70 mmol) in THF (50 mL) under nitrogen atmosphere. The solution was stirred for 4 hours and the solvent was removed in vacuo. EtOAc (50 mL) was added and the solution was washed with cold 1N HCl (30 mL), water (30 mL) and brine (20 mL). The organic layer was dried over sodium sulfate, filtered and concentrated in vacuo. The residue was triturated in 1/1 EtOAc/EtOH (10 mL), filtered and dried in vacuo to give the expected target compound as a yellow powder (200 mg, yield 47.8 %).

¹H-NMR (300 MHz, DMSO-d₆): δ 1.63 (1H, bd, 5-CH₂), 2.75 (1H, bd, 2-CH₂), 3.08 (1H, m, 5-CH₂) 3.21 (1H, m, 2-CH₂), 3.70 (3H, s, OCH₃), 4.85 (2H, s, CH₂-PMB), 5.71 (1H, bd, 3-CH-N), 5.95 (1H, bt, 1-CH-N), 6.91 (2H, m, Harom), 7.35-7.45 (4H, m, Harom), 7.50 (1H, s, CONH), 7.55-7.65 (2H, m, Harom), 7.85-7.95 (2H, dd, Harom), 9.05-9.10 (2H, dd, indole H-4), 10.92 (1H, s, CONHCO). MS (ESI) m/z 596 [M+H]⁺.

### Example 14

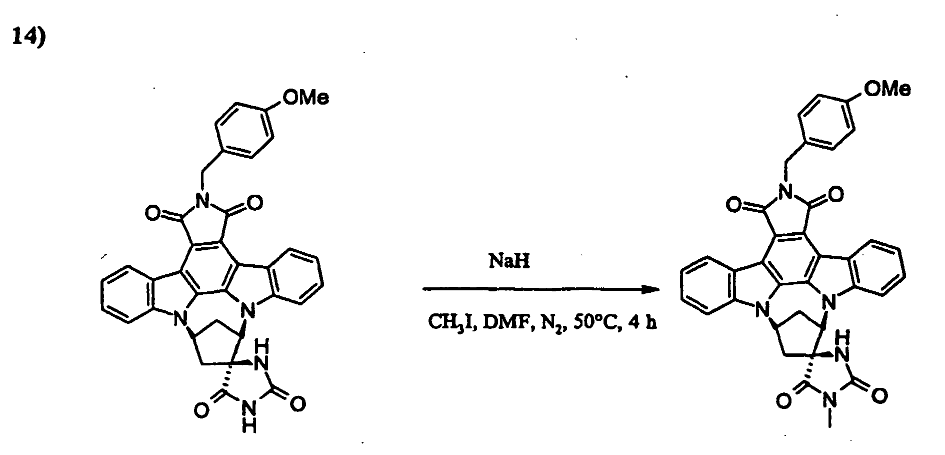

Sodium hydride (25 mg, 0.8 mmol) and methyl iodide (44 µL, 0.71 mmol) were added to a solution of **13** (170 mg, 0.29 mmol) in DMF (20 mL) at room temperature under nitrogen atmosphere. The solution was stirred for 4 hours at 50°C. The solvent was concentrated in vacuo and the residue was dissolved in AcOEt (20 mL), washed with water (10 mL) and brine (10 mL). Purification by flash chromatography (silica gel, CH₂Cl₂/EtOAc 95/5 as eluant mixture) gave the pure target compound as an orange powder (38 mg, yield 21.5 %).

¹H-NMR (300 MHz, DMSO-*d*_{*6*}): δ 1.63 (1H, bd, 5-CH₂), 2.75 (1H, bd, 2-CH₂), 2.92 (3H, s, N-CH₃), 3.08 (1H, m, 5-CH₂), 3.25 (1H, m, 2-CH₂), 3.70 (3H, s, OCH₃), 4.85 (2H, s, CH₂- PMB), 5.68 (1H, bd, 3-CH-N), 6.00 (1H, bt, 1-CH-N), 6.90 (2H, m, Harom), 7.40 (4H, m, Harom), 7.60 (2H, m, Harom), 7.80 (1H, s, NH), 7.95 (2H, m, Harom), 9.05 (2H, dd, indole H-4). MS (ESI) m/z 610[M+H]⁺.

### Example 15

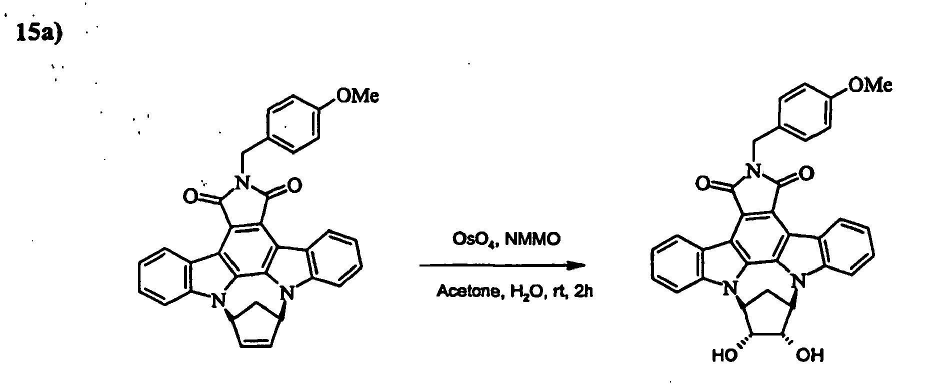

N-methylinorpholine N-oxide (120 mg, 1 mmol), osmium tetroxide (2.5 % in tBuOH, 0.5 mL, catalytic) and a few drops of water were added at rt to a solution of 1f (510 mg, 1 mmol) in acetone (150 mL). The reaction mixture was stirred for 2 hours and the precipitate was filtered off, washed with acetone (20 mL) and petroleum ether (20 mL) to yield the pure target compound (370 mg, yield 69.0 %).

¹H-NMR (300 MHz, DMSO-d₆): δ 2.42 (1H, d, 2-CH₂). 3.15 (1H, m, 2-CH₂). 3.60 (3H, s, OCH₃), 4.08 (2H, m, CH-OH), 4.77 (2H, s, CH₂-PMB), 5.45 (2H, m, CH-N), 6.91 (2H, d, Harom), 7.35 (4H, m, Harom), 7.65 (2H, bt, Harom), 7.85 (2H, d, Harom), 9.02 (2H, d, Harom). MS (ESI) m/z 544 [M+H]⁺.

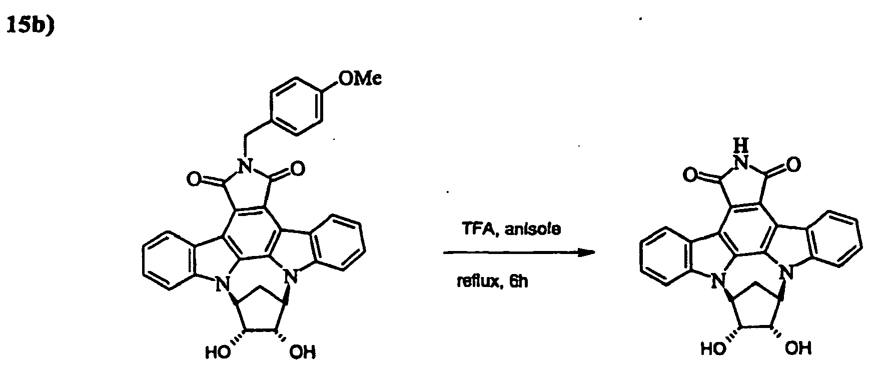

Trifluoroacetic acid (20 mL, large excess) was added to a solution of 15a (300 mg, 0.55 mmol) in anisole (10 mL). The reaction mixture was stirred at reflux for 6 hours and the solvents were eliminated in vacuo. MeOH (20 mL) and NaOH (1N, 20 mL) were added. The reaction mixture was stirred for 2 hours at rt. The reaction mixture was extracted with THF (2x50 mL). The organic layer was dried over sodium sulfate, filterede and concentrated in vacuo. Purification by flash chromatography (silica gel, CH₂Cl₂/EtOAc 7/3 as eluent mixture ) gave the pure target compound (75 mg, yield 32.1 %).

¹H-NMR (300 MHz, DMSO-d₆): δ 2.51 (1H, d, 2-CH₂, 3.25 (1H, m, 2-CH₂). 4.08 (2H, m, CH-OH), 5.47 (2H, m, CH-N), 7.40 (2H, m, Harom), 7.65 (2H, bt, Harom), 7.85 (2H, d, Harom), 9.05 (2H,d, Harom), 11.08 (1H, bs, NH). MS (ESI) m/z 424 [M+H]⁺.

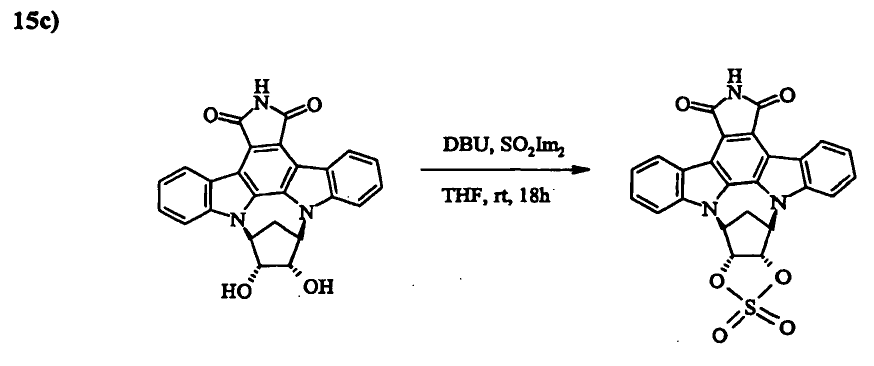

Sulfonyldiimidazole (3.7 g, 18.9 mmol) was added to a solution of 15b (2 g, 4.7 mmol) in THF (200 mL), followed by DBU (2.8 mL, 18.9 mmol) at rt. The reaction mixture was stirred for 18 hours then the solvent was concentrated in vacuo and Et₂O (100 mL) was added. The precipitate was filtered, washed with EtOAc (15 mL) and Et₂O (50 mL), and dried in vacuo to yield the pure target compound as a green powder (2.3 g, yield 100 %).

¹H-NMR (300 MHz, DMSO-*d*_{*6*}): δ 3.00 (1H, d, 2-CH₂), 3.19 (1H, m, 2-CH₂), 3.62 (2H, m, CHO), 5.70 (2H, bs, CH-N), 7.43 (2H, t, Harom) , 7.65 (2H, t, Harom) , 8.01 (2H, d, Harom), 9.15 (2H, d, Harom) , 11.03 (1H, s, imide NH). MS (APCI) m/z 486 [M+H]⁺.

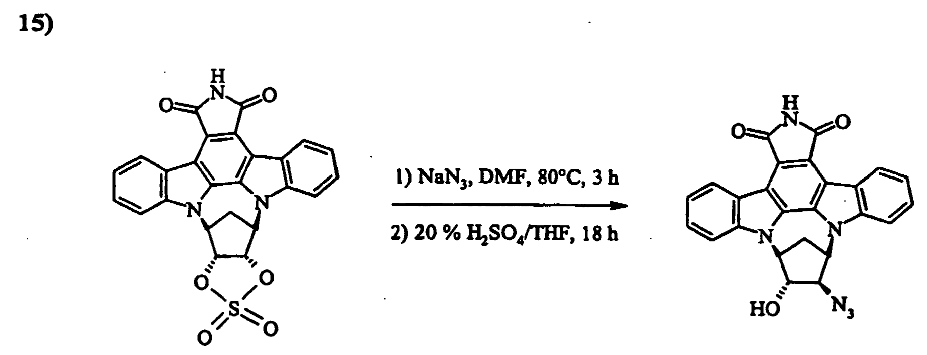

Sodium azide (2.68 g, 41.2 mmol) was added to a solution of 15c (2 g, 4.1 mmol) in DMF (100 mL) at rt. The reaction mixture was heated at 80°C for 3 hours. The solvent was eliminated in vacuo, then THF (40 mL) and 20 % aq. H₂SO₄ (80 mL) were added. The reaction mixture was stirred overnight at rt and was then diluted with THF (100 mL) and washed with saturated KCl (50 mL). The aqueous layer was extracted with THF (2x100 mL). The combined organic layers were washed with sat. NaHCO₃ (100 mL), dried over sodium sulfate, filtered and concentrated in vacuo to yield the pure target compound (493 mg, yield 24.6 %).

¹H-NMR (300 MHz, DMSO-*d*_{*6*}): δ 3.25 (2H, m, 2-CH₂), 3.70 (1H, bt, CH-OH), 4.37 (1H, s, CH-N₃), 5.31 (1H, d, 3-CH-N), 5.86 (1H, bt, 1-CH-N), 6.52 (1H, d, OH), 7.37 (2H, t, Harom), 7.60 (2H, t, Harom), 7.85 (2H, d, Harom), 9.02 (2H, t, Harom), 11.03 (1H, s, imide NH). MS (APCI) m/z 449 [M+H]⁺.

### Example 16

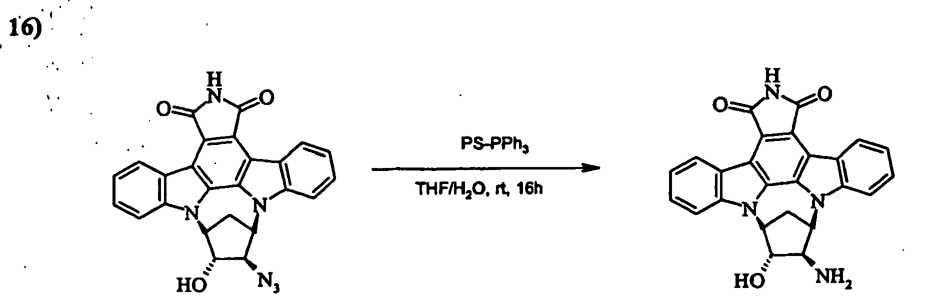

A solution of **15** (100 mg, 0.22 mmol) and water (0.5 mL) in THF (5 mL) was stirred overnight at rt in presence of polymer-supported triphenylphosphine (450 mg, 2.2 mmol). The suspension was filtered off and the resulting solution concentrated in vacuo to yield a solid residue. Purification by flash chromatography (silica gel, CH₂Cl₂/acetone 1/1 as eluant mixture) afforded the pure target compound (25 mg, yield 27.2 %).

¹H-NMR (300 MHz, DMSO-d_{*6*}): δ 2.90 (1H, d, 2-CH₂), 3.35 (1H, m, 2-CH₂), 3.90 (1H, bt, CH-OH), 4.25 (1H, m, CH-NH₂), 5.30 (1H, d, 3-CH-N), 5.70 (2H, m, 1-CH-N + OH), 7.30 (2H, dt, Harom). 7.55 (3H, m, Harom). 7.95 (1H, d, Harom) , 9.05 (2H, dd, Harom), 11.05 (1H, s, imide NH). MS (APCI) m/z 423 [M+H]⁺.

### Example 17

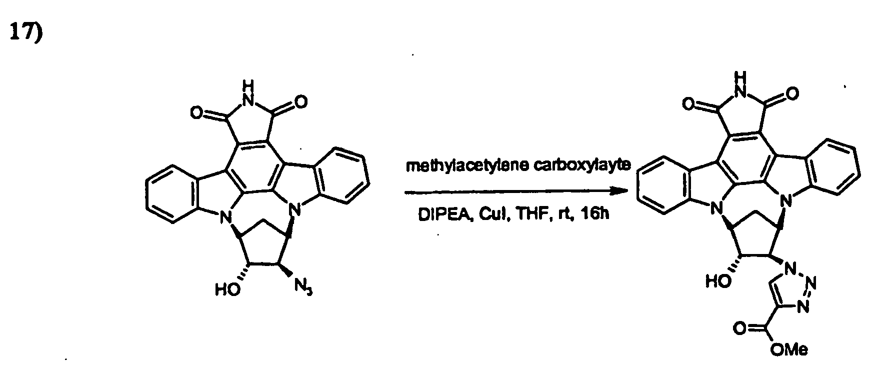

A solution of 15 (0.3 g, 0.67 mmol) in THF (10 mL) was added to a mixture of methylacetylene carboxylate (0.17 mL, 2 mmol), copper (I) iodide (380 mg, 2 mmol) and N-diisopropylethylamine (5.6 mL, 33 mmol) in THF (10 mL). The reaction mixture was stirred for 4 hours at rt and 1N HCl (20 mL) was added. The aqueous phase was extracted with THF.

The combined organic layers were dried over sodium sulfate, filtered and concentrated in vacuo. The residue-was purified by flash chromatography (silica gel, CH₂Cl₂/acetone 9/1 to 8/2 as eluent mixture) to give the pure target compound (262 mg, yield 75.3 %).

¹H-NMR (300 MHz, DMSO-*d*_{*6*}): δ 2.80 (1H, d, 2-CH₂), 3.40 (1H, m, 2-CH₂), 3.70 (3H, s, OCH₃), 4.48 (1H, bt, CH-OH), 5.55 (2H, m, 3-CH-N + 4-CH-N-N), 5.97 (1H, bt, 1-CH-N), 6.45 (1H,d,OH), 7.18 (2H,m,Harom), 7.30 (1H,t,Harom), 7.40 (1H,bt,Harom), 7.65(1H, bt, Harom), 7.95 (1H, d, Harom), 8.50 (1H, s, triazole H), 8.90 (1H, d, Harom), 9.15 (1H, d, Harom), 11.10 (1H, s, imide NH). MS (APCI) m/z 555 [M+Na]⁺.

### Example 18

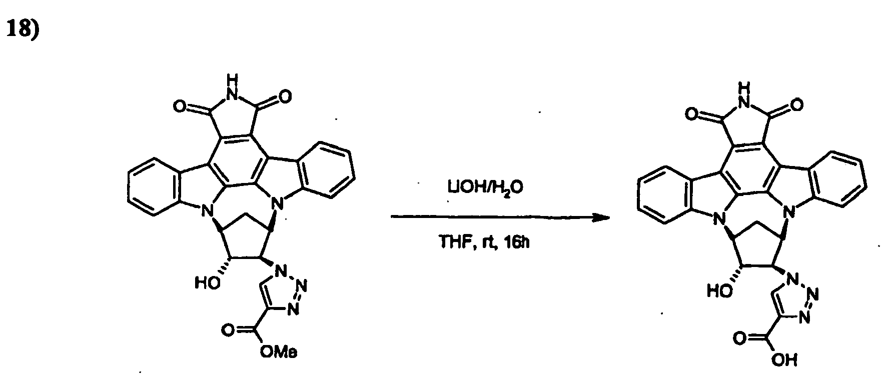

Lithium hydroxide monohydrate (77 mg, 1.83 mmol) and water (0.5 mL) were added to a solution of 17 (0.195 mg, 0.37 mmol) in THF. The reaction mixture was stirred overnight at rt and sat. NH₄OAc (2 mL) was added. The reaction mixture was heated at 120°C for 3 hours, cooled and extracted with EtOAc (15 mL) and washed with 1N HCl (2x10 mL). The organic layer was dried over sodium sulfate, filtered and concentrated in vacuo. EtOAc (10 mL) was added and the precipitate was filtered and dried in vacuo to yield the pure target compound (150 mg, yield 78.9 %).

1H-NMR (300 MHz, DMSO-d6): 2.80 (1H, d, 2-CH₂), 3.30 (1H, d, 2-CH₂). 3.97 (1H, bd, 5-CH-OH), 4.75 (1H, d, OH), 5.55 (2H, m, 3-CH-N + 4-CH-N-N), 6.00 (1H, bt, 1-CH-N), 7.18 (2H, m, Harom), 7.35 (1H, bt, Harom), 7.40 (1H, t, Harom), 7.65 (1H, bt, Harom), 7.97 (1H, d, Harom), 8.35 (1H, s, triazole H), 8.90 (1H, d, Harom), 9.10 (1H, d, Harom), 11.10 (1H, s, imide NH), 12.6 0(1H, m, COOH). MS (APCI) m/z 1035 [2M-H]⁺.

### Example 19

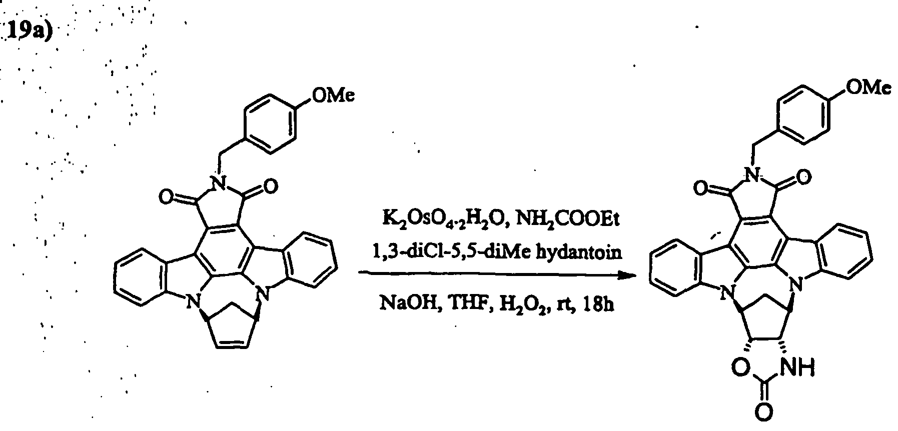

A solution of 1f (4 g, 7.85 mmol) in THF (15 mL) was added at rt to a solution of urethane (7.0 g, 78.5 mmol), 1,3-dichloro-5,5-dimethylhydantoin (7.7 g, 39.25 mmol) and NaOH (3.1 g, 78.5 mmol) in water (30 mL) and THF (15 mL). Potassium osmate dihydrate (30 mg, catalytic) was then added and stirring at rt was continued for 18 hours. The reaction was quenched by addition of EtOAc (100 mL) and solid sodium bisulfite (2g). The organic layer was washed with brine and dried to give a residue (3.16g) which was repeatedly chromatographed (silica gel, EtOAc/petroleum ether 7/3 and CH₂Cl₂/EtOAc 9/1 as eluant mixtures). A fraction containing the title compound (740 mg) was triturated with EtOAc and THF to give the pure title compound (270 mg, yield 6.04 %).

¹H-NMR (300 MHz, DMSO-*d*_{*6*}): δ 2.81 (1H, m, 2-CH₂, 3.23 (1H, m, 2-CH₂), 3.73 (3H, s, OCH₃), 4.18 (1H, m, 1-CH-N), 4.83 (2H, s, CH₂-PMB), 4.98 (1H, d, 3-CH-N), 5,65 (1H, d, OH), 5.57 (1H, dd, 4-CH-N), 5.91 (1H, m, 5-CH-O), 6.89 (2H, d, Harom), 7.35 (2H, m, Harom), 7.40 (2H, t, Harom), 7.74 (2H, m, Harom), 7.91 (2H, d, Harom), 9.04 (2H, dd, Harom). MS (ESI) m/z 569 [M+H]⁺.

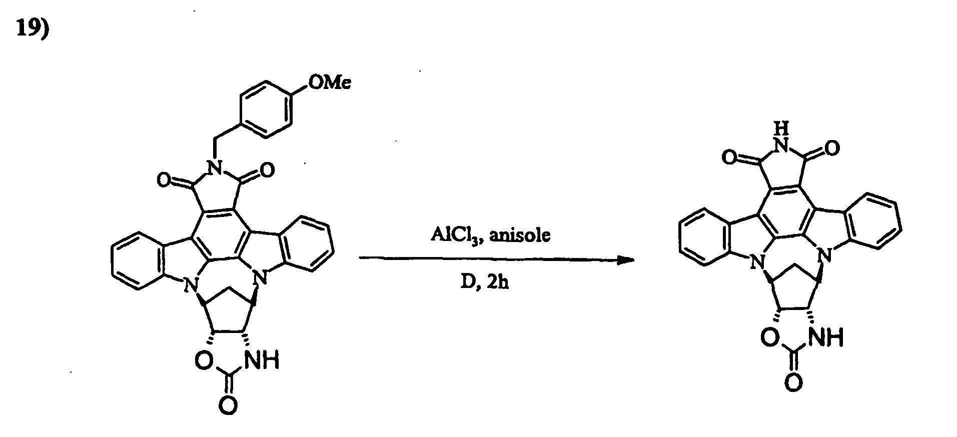

Aluminium chloride (210 mg, 1.58 mmol) was added at room temperature to a solution of **19a** (90 mg, 0.158 mmol) in anisole (10 mL). The suspension was stirred at reflux for 2 hours. The reaction mixture was cooled and poured onto water (30 mL), extracted with EtOAc/tetrahydrofuran (2x30 mL), dried over sodium sulfate, filtered and concentrated in vacuo. Purification by preparative TLC (silica gel, CH₂Cl₂/EtO Ac 9/1 as eluant mixture) gave the pure target compound (50 mg, yield 67.4 %).

¹H-NMR (300 MHz, DMSO-*d*_{*6*}): δ 2.78 (1H, bd, 2-CH₂), 3.45 (1H, m, 2-CH₂), 4.17 (1H, d, 1-CH-N), 5.01 (1H, dd, 3-CH-N), 5.60 (1H, dd, 4-CH-N), 5.89 (1H, m, 5-CH-O), 6.87 (2H, d, Harom), 7.32 (2H, m, Harom), 7.44 (2H, t, Harom), 7.78 (2H, m, Harom). 7.95 (2H, d, Harom), 9.08 (2H, dd, Harom). MS (ESI) m/z 449 [M+H]⁺.

### Example 20

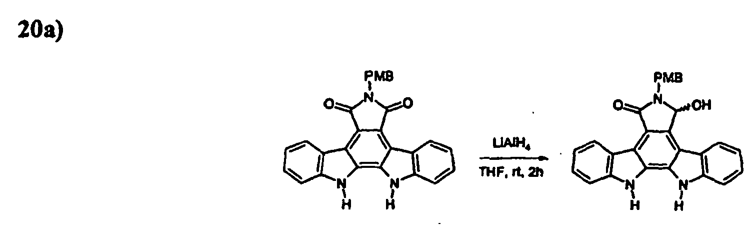

Lithium aluminium hydride (15.0 g, 400 mmol) was added portionwise to a stirred solution of 1e (35.4 g, 800 mmol) in THF (1L) without exceeding a gentle reflux. The reaction mixture was stirred for an additional 1h at rt before being quenched with wet sodium sulfate. The solid salts were filtered off and the filtrate was concentrated in vacuo. The residue was taken up in EtOAc (1 L), washed with water and brine. The organic layer was dried over sodium sulfate and concentrated in vacuo to yield the pure target compound (33.7 g, yield 95.2 %).

¹H-NMR (300 MHz, DMSO-*d*_{*6*}): δ 3.72 (3H, s, OCH₃), 4.44 (1H, d, CH₂-PMB), 5.06 (1H, d, CH₂PMB), 6.14 (1H, d, CH-OH), 6.81 (1H, d, OH) , 6.92 (2H, d, Harom) , 7.26 (2H, Harom), 7.36 (2H, d, Harom) , 7.4-6 (2H, m, Harom), 7.75 (2H, m, Harom), 8.32 (1H, d, Harom), 9.19 (1H, d, Harom), 11.39 (1H, s, NH), 11.51 (1H, s, NH). MS (APCI) m/z 448 [M+H]⁺.

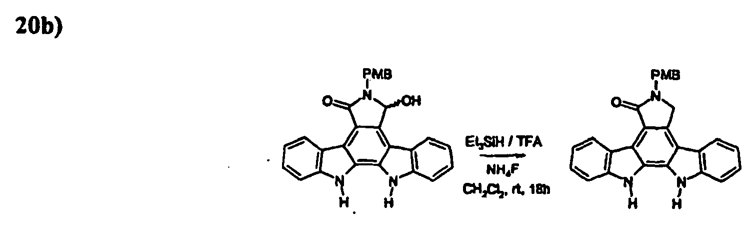

Trifluoroacetic acid (30 mL, 370 mmol) was added to a stirred mixture of 20a (33.7g, 75.0 mmol), ammonium fluoride (3.6 g,100 mmol) and triethylsilane (15.6 mL, 100 mmol) in CH₂Cl₂ (200 mL) cooled in an ice-bath. The reaction mixture was then allowed to stir at rt for 18 hours to be then poured onto ice-water. The precipitate was filtered, washed repeatedly with water and dried to give a first batch of pure target compound (18.6 g). The organic layer was washed by sat. aqueous NaHCO₃ and brine, dried over sodium sulfate and concentrated to give (30.9 g, yield 94.9 %).

¹H-NMR (300 MHz, DMSO-d₆): δ 3.72 (3H, s, OCH₃), 4.84 (2H, s, lactam-CH₂), 4.94 (2H, s, CH₂-PMB), 6.93 (2H, d, Harom), 7.26 (2H, Harom), 7.36 (2H, d, Harom), 7.48 (2H, m, Harom), 7.74 (1H, d, Harom), 7.80 (1H, d, Harom), 9.28 (1H, d, Harom), 11.46 (1H, s, NH), 11.62 (1H, s, NH). MS (APCI) m/z 432 [M+H]⁺.

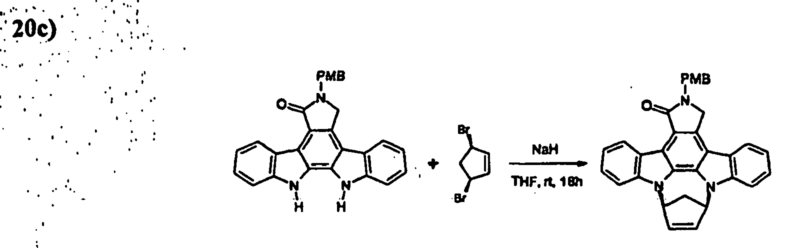

Sodium hydride (60% suspension in oil, 5.7 g, 214.8 mmol) was added portionwise to a solution of 20b (30.9 g, 71.6 mmol) in THF (1 L) cooled in an ice-bath. After 1 hour 1a (32.1 g, 143.2 mmol) in THF (80 mL) was added, the reaction mixture was allowed to stir at rt for 1 hour and additional sodium hydride (1.9 g, 71.6 mmol) was added. The reaction mixture was stirred at rt for additional 16 hours, then water (1 L) was added and the reaction mixture was extracted with EtOAc (2x500 mL). The combined organic layers were washed with water and brine, dried over sodium sulfate and concentrated. Precipitation of the residue (petroleum ether) yielded the pure target compound (41.3 g, quantitative yield) in sufficient purity for the next step.

¹H-NMR (300 MHz, DMSO-d₆): δ 2.64 (1H, d, 2-CH₂), 3.13 (1H, m, 2-CH₂), 3.71 (3H, s, OCH₃), 4.83 (2H, s, lactam-CH₂), 4.94 (2H, s, CH₂ -PMB), 6.20 (2H, m, CH-N), 6.37 (2H, m, CH=CH , 6.93 (2H, d, Harom), 7.33 (4H, m, Harom), 7.52 (2H, m, Harom), 7.96 (3H, m, Harom), 9.34 (1H, d, Harom). MS (APCI) m/z 496 [M+H]⁺.

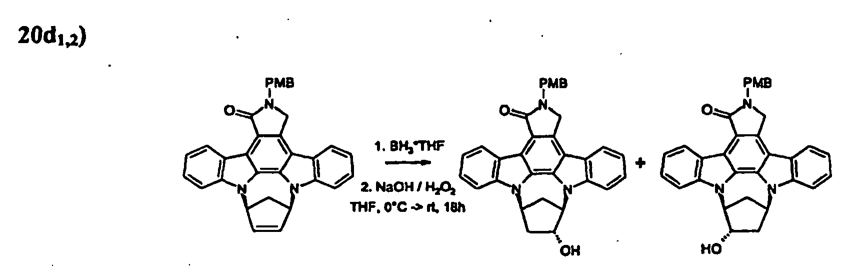

Borane (1M in THF, 416 mL, 416 mmol) was added to a stirred solution of 20c (41.3 g, 83.3 mmoL) in THF (1 L) cooled in an ice-bath. The reaction mixture was stirred for 90 min at 0°C, then sodium hydroxide (32%, 52 mL, 416 mmol) was carefully added followed by hydrogen peroxide (30%, 140 mL, 1.25 mol). The reaction mixture was allowed to stir at rt for 18h. The organic layer was decanted, water was added (500 mL) and the resulting mixture was extracted by ethyl acetate (3x500 mL). The combined organic layers were washed by brine, dried over sodium sulfate and concentrated. Purification by flash chromatography (silica gel, CH₂Cl₂/AcOEt 95/5 to 80/20 as eluant mixture) afforded the pure regioisomeric mixture 20d_{1,2} (14.6 g, yield 34.4 %).

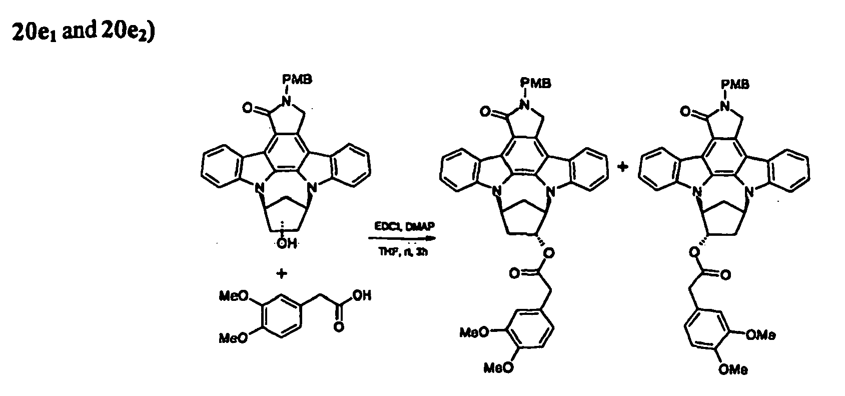

A mixture of 20d_{1,2} (14.6 g, 28.4 mmol), N'-(3-dimethylaminopmpyl)-N-ethylcarbodiinude hydrochloride (10.9 g, 56.8 mmol), N-dimethylaminopyridine (347 mg, 2.84 mmole) and 3,4-dimethoxyphenylacetic acid (11.14 g, 56.8 mmol) in THF (500 mL) was stirred at rt for 3 hours. The reaction mixture was diluted by EtOAc, washed (water, 1N HCl, water, 1N NaOH, water and brine), dried over sodium sulfate and concentrated. Purification by flash chromatography (silica gel,. CH₂Cl₂/AcOEt 99/1 to 97.5/2.5 as eluant mixture) gave both pure target compounds as separate fractionsesters (E-lactam 20e₁, 7.2 g, yield 37.0 %, Z-lactam 20e₂, 6.9 g, yield 34.8 %).

**20e**_{**1**}**:** ¹H-NMR (300 MHz, DMSO-*d*_{*6*}): δ 2.10 (1H, dd, 5-CH₂), 2.58 (1H, dd, 5-CH₂), 2.66 (2H, d, 2-CH₂), 3.02 (1H, m, 2-CH₂), 3.71 (3H, s, OCH₃), 3.75 (5H, s, OCH₃, CH₂-COO), 3.78 (3H, s, OCH₃), 4.77 (1H, d, lactam-CH₂), 4.83 (1H, d, lactam-CH₂), 4.92 (2H, s, CH₂-PMB), 5.09 (1H, m, 5-CH-O), 5.56 (1H, d, 3-CH-N), 5.96 (1H, t, 1-CH-N), 6.92 (5H, m, Harom), 7.30 (4H, m, Harom), 7.51 (2H, m, Harom), 7.81 (1H, d, Harom), 7.83 (1H, d, Harom), 8.00 (1H, d, Harom), 9.29 (1H, m, Harom). MS (APCI) m/z 692 [M+H]+.

**20e**_{**2**}**:** ¹H-NMR (300 MHz, DMSO-*d*_{*6*}): δ 2.14 (1H, dd, 5-CH₂), 2.36 (1H, m, 5-CH₂), 2.66 (2H, m, 2-. CH₂), 3.05 (1H, m, 2-CH₂), 3.72 (5H, s, OCH₃, CH₂-COO), 3.75 (3H, s, OCH₃), 3.78 (3H, s, OCH₃), 4.79 (1H, d, lactam-CH₂), 4.85 (1H, d, lactam-CH₂), 4.94 (2H, s, CH₂-PMB), 5.11 (1H, m, 5-CH-O), 5.58 (1H, d, 3-CH-N), 5.95 (1H, t, 1-CH-N), 6.94 (5H, m, Harom), 7.32 (4H, m, Harom), 7.52 (2H, m, Harom), 7.79 (1H, d, Harom), 7.89 (1H, d, Harom), 8.00 (1H, d, Harom), 9.33 (1H, m, Harom). MS (APCI) m/z 692 [M+H]⁺.

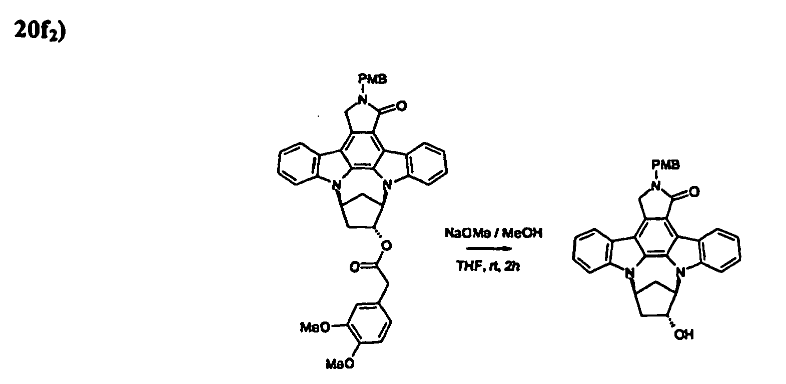

Sodium methoxide (10 mg, catalytic) was added to a solution of **20e**_{**2**} (6.6 g, 9.5 mmol) in THF (100 mL) and MeOH (100 mL) and stirred for 2 hours at rt. The reaction mixture was concentrated and purified by flash chromatography (silica gel, CH₂Cl₂/EtOAc 9/1 to pure EtOAc as eluant mixture) to yield the pure target compound (4.40 g, yield 90.1 %).

¹H-NMR (300 MHz, DMSO-*d*_{*6*}): δ 1.91 (1H, dd, 5-CH₂), 2.36 (1H, dd, 5-CH₂), 2.57 (1H, d, 2-CH₂), 3.16 (1H, m, 2-CH₂), 3.72 (3H, s, OCH₃), 4.16 (1H, m, CH-OH), 4.79 (1H, d, lactam-CH₂), 4.85 (1H, d, lactam-CH₂), 4.94 (2H, s, CH₂-PMB), 5.33 (1H, d, 3-CH-N), 5.60 (1H, d, OH) 5.87 (1H, t, 1-CH-N), 6.92 (2H, d, Harom), 7.34 (4H, m, Harom), 7.54 (2H, m, Harom), 7.86 (1H, d, Harom), 7.89 (1H, d, Harom), 7.99 (1H, d, Harom), 9.34 (1H, d, Harom). MS (APCI) m/z 514 [M+H]⁺.

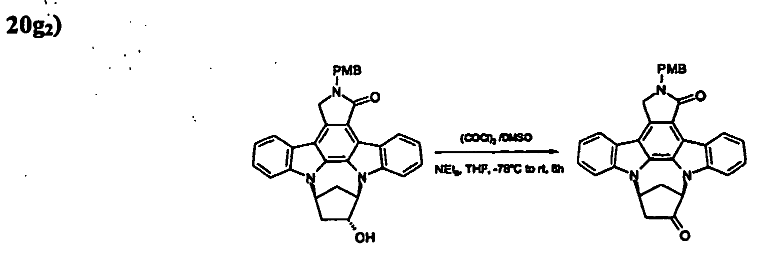

Dimethylsulfoxide (2.9 mL, 40.8 mmol) in THF (20 mL) was added dropwise to a stirred solution of oxalyl chloride (1.8 mL, 20.4 mmol) in THF (20 mL) at -78°C under nitrogen maintaining the internal temperature below -60°C. After 10 min a solution of **20f**_{**2**} (4.2 g, 8.2 mmol) in THF (200 mL) was added dropwise (T < -60°C). After further 10 min triethylamine (11.8 mL, 84.8 mmol) was added and the reaction mixture was allowed to warm to rt. The reaction mixture was quenched and extracted (EtOAc, 2x50 mL). The combined organic layers were washed (water, brine), dried over sodium sulfate and concentrated. Purification by flash chromatography (silica gel, pure CH₂Cl₂ to CH₂Cl₂/AcOEt 99/1 as eluant mixture) gave the pure title compound (3.1 g, yield 74.3 %).

¹H-NMR (300 MHz, DMSO-d₆): δ 2.36 (1H, dd, 5-CH₂), 2.98 (1H, dd, 5-CH₂), 3.20 (1H, dd, 2-CH₂), 3.50 (1H, m, 2-CH₂), 3.72 (3H, s, OCH₃), 4.83 (2H, s, lactam-CH₂), 4.95 (2H, s, CH₂₋PMB), 5.55 (1H, d, 3-CH-N), 6.12 (1H, t, 1-CH-N), 6.93 (2H, d, Harom), 7.33 (4H, m, Harom), 7.55 (2H, m, Harom ), 7.88 (1H, m, Harom), 7.90 (1H, d, Harom), 8.02 (1H, d, Harom), 9.32 (1H, d, Harom). MS (APCI) m/z 512 [M+H]⁺.

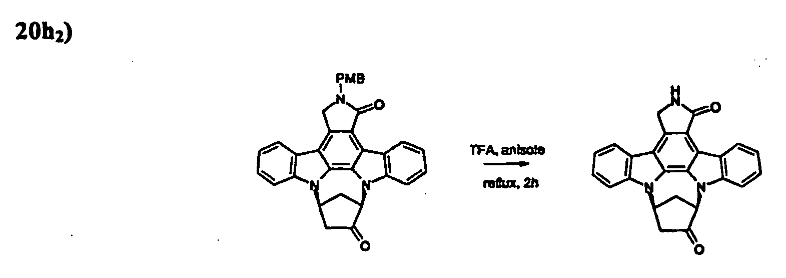

A suspension of **20g**_{**2**} (2.5 g, 4.9 mmol) in anisole (20 mL) was treated with trifluoroacetic acid (100 mL) and heated at reflux for 2 hours. After concentration the residue was diluted with EtOAc (200 mL) and THF (50 mL), and washed (sat. NaHCO₃-solution, water and brine). The organic layer was concentrated, the residue was treated with ethanol (200 mL) and the resulting precipitate was filtered. Washings (diethyl ether) and drying produced the pure title compound (1.7 g, yield 91.2%).

¹H-NMR (300 MHz, DMSO-d₆): δ 2.33 (1H, dd, 5-CH₂), 3.00 (1H, dd, 5-CH₂), 3.21 (1H, dd, 2-CH₂), 3.50 (1H, m, 2-CH₂), 4.97 (2H, s, lactam-CH₂), 5.55 (1H, d, 3-CH-N), 6.13 (1H, t, 1-CH-N), 7.30 (1H, t, Harom), 7.37 (1H, t, Harom), 7.53 (1H, m, Harom), 7.57 (1H, m, Harom), 7.87 (1H, d, Harom), 7.90 (1H, d, Harom), 8.07 (1H, d, Harom) 8.58 (1H, s, NH), 9.26 (1H, d, Harom). MS (APCI) m/z 392 [M+H]⁺.

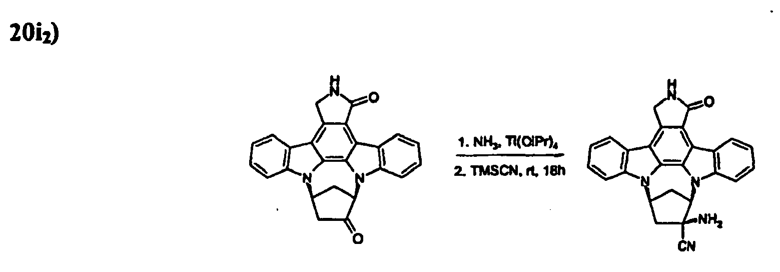

Titanium (IV) isopropoxide (1.2 mL, 4.2 mmol) and 7M methanolic ammonia (5.4 mL, 38 mmol) were sequentially added to a stirred mixture of 20h₂ (1.5 g, 3.8 mmol) in 1,2-dichloroethane (100 mL) at rt. Trimethylsilyl cyanide (0.6 mL, 5.2 mmol) was added after 2 hours, and the reaction mixture was stirred at rt for 18 hours. Water (50 mL) was then added, and the mixture was extracted (CH₂Cl₂, 3x50 mL). The combined organic layers were dried over sodium sulfate and concentrated in vacuo. After purification by chromatography (silicagel, AcOEt/CH₂Cl₂ 3/7 as eluant mixture) the pure title compound was obtained (1.28 g, yield 80.6%).

¹H-NMR (300 MHz, *DMSO-d*_{*6*}*):* δ 1.61 (1H, m, 5-CH₂), 2.32 (2H, s, NH₂), 2.74 (1H, m, 5-CH₂), 3.25 (2H, m, 2-CH₂), 4.98 (2H, s, lactam-CH₂), 5.72 (1H, d, 3-CH-N), 5.93 (1H, dd, 1-CH-N), 7.32 (2H, t, Harom), 7.53 (2H, m, Harom), 7.84 (1H, d, Harom), 7.96 (1H, d, Harom), 8.07 (1H, d, Harom), 8.56 (1H, s, NH), 9.31 (1H, d, Harom). MS (ESI) m/z 418 [M+H]⁺.

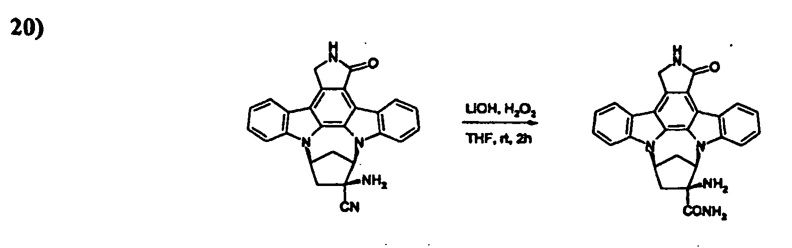

Lithium hydroxide monohydrate (84 mg, 2.0 mmol) and hydrogen peroxide (30%, 2 mL, large excess) were added at rt to a solution of **20i**_{**2**} (86 mg, 0.2 mmol) in THF (10 mL). The reaction mixture was stirred for 2 hours at rt, then concentrated in vacuo. The residue was diluted with water (50 mL), the precipitate was filtered, washed with water and dried in vacuo at 50°C to yield the pure title compound (50 mg, yield 57.1 %).

¹H-NMR (300 MHz, DMSO-*d*_{*6*}): δ 1.32 (2H, s, NH₂), 1.48 (1H, d, 5-CH₂), 2.61 (1H, d, 5-CH₂), 3.15 (1H, dd, 2-CH₂), 3.23 (1H, dd, 2-CH₂), 4.98 (2H, s, lactam-CH₂), 5.44 (1H, d, 3-CH-N), 5.77 (1H, m, 1-CH-N), 7.28 (1H, t, Harom), 7.34 (1H, t, Harom), 7.50 (2H, m, Harom), 7.61 (2H, bs, CONH₂), 7.68 (1H, d, Harom), 7.89 (1H, d, Harom), 8.07 (1H, d, Harom), 8:56 (1H, s, NH), 9.31 (1H, d, Harom). MS (APCI) m/z 436 [M+H]⁺.

### Examples 21-48

Compounds 21 to 48 were prepared by methods known to the skilled person and/or described herein.

### Example 49

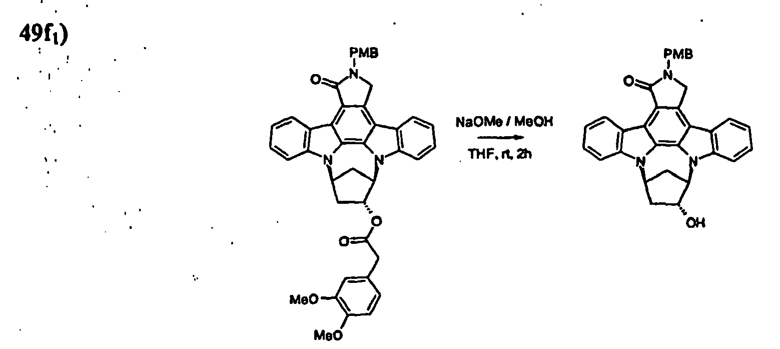

The previously prepared 20e₁ (7.2 g, 10.4 mmol) in THF (150 mL) and methanol (150 mL) was stirred for 2h at rt in the presence of a catalytic amount of sodium methoxide. The reaction mixture was concentrated in vacuo and purified by flash chromatography (silica gel, gradient system: CH₂Cl₂ 100% - 10% AcOEt -100 % AcOEt) to afford the pure alcohol (5.22 g, 98%).

¹H-NMR (300 MHz, DMSO-d₆): δ 1.88 (1H, dd, 5-CH₂), 2.36 (1H, dd, 5-CH₂), 2.57 (1H, d, 2-CH₂), 3.16 (1H, m, 2-CH₂), 3.71 (3H, OCH₃), 4.15 (1H, m, 4-CH-OH), 4.79 (1H, d, lactam-CH₂), 4.85 (1H, d, lactam-CH₂), 4.94 (2H, s, CH₂-PMB), 5.31 (1H, d, 3-CHN), 5.58 (1H, d, OH) 5.87 (1H, t, 1-CHN), 6.91 (2H, d, Harom), 7.26 (1H, m, Harom), 7.33 (3H, m, Harom), 7.47 (1H, td, Harom), 7.56 (1H, td, Harom), 7.85 (1H, d, Harom) , 7.90 (1H, d, Harom), 8.01 (1H, d, Harom) , 9.27 (1H, d, Harom). MS (APCI) m/z 514 [M+H]⁺.

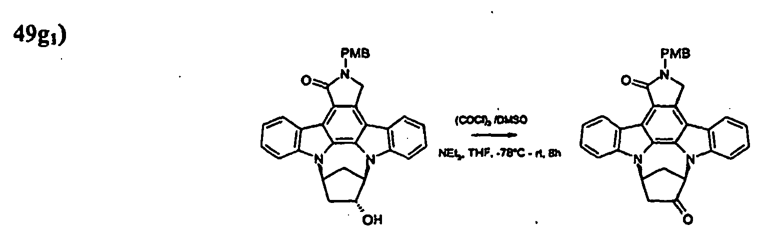

To a stirred solution of oxalylchloride (1.98 mL, 22.8 mmol) in THF (20 mL) at -78°C under nitrogen was added a solution of DMSO (3.2 mL, 45.5 mmol) in THF (20 mL) in a way that the internal temperature remained < -60°C. After 10 min, a solution of **49f**_{**1**} (4.7 g, 9.1 mmol) in THF (200 mL) was added (T < -60°C) dropwise. After further 10 min, triethylamine (13.2 mL, 94.6 mmol) was added and the reaction mixture was allowed to warm up to rt. The reaction mixture was hydrolysed and extracted by ethyl acetate (2x). The combined organic layers were washed with water followed by brine, dried over sodium sulfate and concentrated in vacuo. Purification by flash chromatography (silica gel, CH₂Cl₂ then 1 % AcOEt) gave the pure compound (2.9 g, 62.3 %).

¹H-NMR. (300 MHz, DMSO-*d*_{*6*}): δ 2.45 (1H, dd, 5-CH₂), 2.98 (1H, dd, 5-CH₂), 3.20 (1H, dd, 2-CH₂), 3.49 (1H, m, 2-CH₂), 3.71 (3H, s, OCH3), 4.78 (1H, d, lactam-CH₂), 4.84 (1H, d, lactam-CH₂, 4.93 (2H, s, CH₂-PMB), 5.54 (1H, d, 3-CHN), 6.11 (1H, t, 1-CHN), 6.91n (2H, d, Harom), 7.32 (4H, m, Harom), 7.54 (2H, m, Harom), 7.82 (1H, d, Harom), 7.92 (1H, d, Harom), 8.00 (1H, d, Harom), 9.35 (1H, d, Harom). MS (APCI) m/z 512 [M+H]⁺.

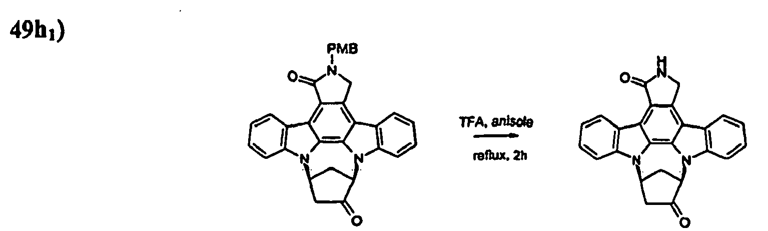

A mixture of 49g₁ (2.4 g, 4.7 mmol) in anisole (20 mL) was treated with trifluoroacetic acid (100 mL) and heated under reflux for 2h. The reaction mixture was concentrated in vacuo. The residue was diluted with ethylacetate and THF and washed with aq. sat. NaHCO₃-solution, water and brine. The organic emulsion layer was concentrated in vacuo. The residue was diluted with ethanol, and the resulting precipitate was filtered off, washed by diethyl ether and dried in vacuo to obtain the compound (1.5 g, 81%).

¹H-NMR (300 MHz, DMSO-*d*_{*6*}): δ 2.33 (1H, dd, 5-CH₂), 2.98 (1H, dd, 5-CH₂), 3.21 (1H, dd, 2-CH₂), 3.50 (1H, m, 2-CH₂), 4.96 (2H, s, lactam-CH₂), 5.55 (1H, d, 3-CHN), 6.12 (1H, t, 1-CHN), 7.29 (1H, t, Harom), 7.37 (1H, t, Harom), 7.52 (1H, m, Harom), 7.58 (1H, m, Harom), 7.81 (1H, d, Harom), 7.94 (1H, d, Harom), 8.08 (1H, d, Harom), 8.60 (1H, s, NH), 9.29 (1H, d, Harom). MS (APCI) m/z 392 [M+H]⁺.

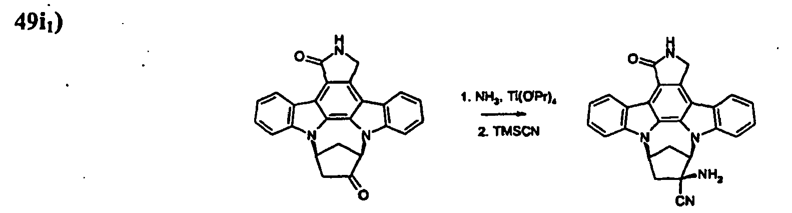

To a stirred mixture of 49g₁ (1.7 g, 4.3 mmol) in dichloroethane (100 mL) at rt was added titanium isopropylate (1.4 mL, 4.8 mmol) followed by a 7 N solution of ammonia in methanol (6.1 mL, 43 mmol). After 2h, trimethylsilyl cyanide (0.7 mL, 5.2 mmol) were added. The reaction mixture was stirred at rt for 18h. Water (50 mL) were added, and the mixture was extracted 3x by dichloromethane. The combined organic layers were dried over sodium sulfate and concentrated in vacuo. After purification by chromatography on silica (eluent: AcOEt /CH₂Cl₂ = 3/7), 1.12g of the aminonitrile has been obtained (62.4%).

¹H-NMR (300 MHz, DMSO-d₆): δ 1.58 (1H, m, 5-CH₂), 2.38 (2H, s, NH₂), 2.75 (1H, m, 5-CH₂), 3.25 (2H, m, 2-CH₂) , 4.98 (2H, s, lactam-CH₂), 5.73 (1H, d, 3-CHN), 5.94 (1H, dd, 1-CHN), 7.26 (1H, t, Harom) , 7.37 (1H, t, Harom), 7.53 (2H, m, Harom), 7.80 (1H, d, Harom), 8.05 (2H, m, Harom), 8.58 (1H, s, NH), 9.26 (1H, d, Harom), MS (ESI) m/z 418 [M+H]⁺.

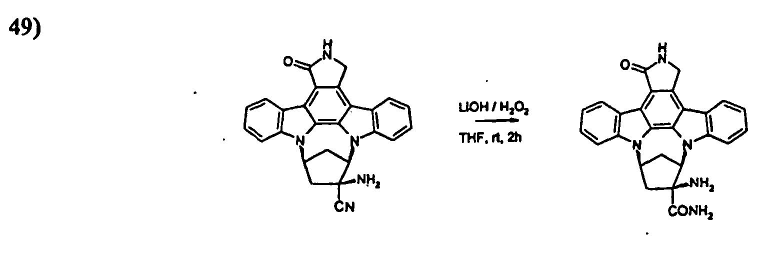

To a solution of the previously prepared amino nitrile (1.48 g, 3.54 mmol) in THF (100 mL) at rt was added lithium hydroxide hydrate (1.48 g, 35.4 mmol) followed by 12 ml of a 30% hydrogen peroxide solution. The reaction mixture was stirred for 2h at rt, then concentrated in vacuo. The residue was diluted by water, the formed precipitate was filtered off, washed with water and dried in vacuo at 50°C to yield 1.25 g (81 %) of the expected compound.

¹H-NMR (300 MHz, DMSO-*d*_{*6*}): δ 1.33 (2H, s, NH₂), 1.45 (1H, d, 5-CH₂), 2.60 (1H, d, 5-CH₂), 3.13 (1H, dd, 2-CH₂), 3.23 (1H, dd, 2-CH₂), 5.00 (2H, s, lactam-CH₂) 5.45 (1H, d, 3-CHN), 5.76 (1H, m, 1-CHN), 7.26 (1H, t, Harom), 7.36 (1H, t, Harom), 7.51 (2H, m, Harom), 7.62 (2H, broad, CONH₂), 7.75 (1H, d, Harom), 7.80 (1H, d, Harom), 8.09 (1H, d, Harom), 8.56 (1H, s, NH), 9.27 (1H, d, Harom). MS (APCI) m/z 436 [M+H]⁺.

### Example 50

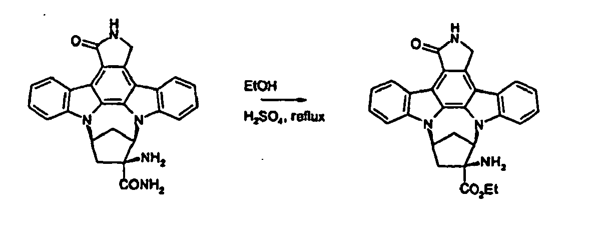

To a solution of 49 (1.24 g, 2.85 mmol) in ethanol (150 mL) at rt was added dropwise concentrated sulphuric acid (15 mL) (attention: very exothermic). The reaction mixture was stirred under reflux for 24h then poured onto ice-water (500 mL) and neutralized by sodium carbonate. The aqueous layer was extracted 3x by ethyl acetate. The combined organic layers were washed with brine, dried over sodium sulfate and concentrated in vacuo to obtain 1.2g of the crude compound (90%). Purification by flash chromatography on silica gel (eluent: gradient dichloromethane / ethyl acetate 7/3 to 1 / 1) gave 0.38g (29%) of the desired compound.

¹H-NMR (300 MHz, DMSO-*d*_{*6*}): δ 1.36 (3H, t, OCH₂CH₃), 1.48 (1H, d, 5-CH₂), 2.61 (1H, d, 5-CH₂), 3.15 (1H, dd, 2-CH₂), 3.23 (1H, dd, 2-CH₂), 4.31 (2H, m, OCH₂CH₃) 4.98 (2H, s, lactam-CH₂), 5.62 (1H, d, 3-CHN), 5.79 (1H, m, 1-CHN), 7.25 (1H, t, Harom), 7.35 (1H, t, Harom), 7.48 (1H, m, Harom), 7.55 (1H, m, Harom), 7.75 (1H, d, Harom), 7.81 (1H, d, Harom), 8.08 (1H, d, Harom), 8.56 (1H, s, NH), 9.26 (1H, d, Harom). MS (APCI) m/z 465 [M+H]⁺.

### Example 51

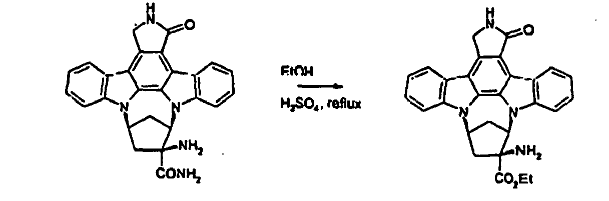

To a solution of 20 (1.66 g, 3.81 mmol) in ethanol (200 mL) at rt was added dropwise concentrated sulfuric acid (20 mL). The reaction mixture was stirred under reflux for 24h then poured onto ice-water (500 mL) and neutralized by sodium carbonate. The aqueous layer was extracted 3x by ethyl acetate. The combined organic layer was washed with brine, dried over sodium sulfate and concentrated in vacuo to obtain 1.6g of the crude compound (90%). Part was purified by flash chromatography on silica gel (eluent: gradient dichloromethane / ethyl acetate 7 / 3 to 1 /1) gave 0.64g (37%) of the desired compound.

¹H-NMR (300 MHz, DMSO-d₆): δ 1.36 (3H, t, OCH₂CH₃), 1.52 (1H, d, 5-CH₂), 2.66 (1H, d, 5-CH₂). 3.05 (1H, dd, 2-CH₂), 3.23 (1H, dd, 2-CH₂), 4.31 (2H, m, OCH₂CH₃) 4.98 (2H, s, lactam-CH₂), 5.63 (1H, d, 3-CHN), 5.81 (1H, m, 1-CHN), 7.28 (1H, t, Harom), 7.35 (1H, t, Harom), 7.48 (1H, m, Harom), 7.53 (1H, m, Harom), 7.70 (1H, d, Harom), 7.89 (1H, d, Harom), 8.08 (1H, d, Harom), 8.55 (1H, s, NH), 9.30 (1H, d, Harom). MS (APCI) m/z 465 [M+H]⁺.

### Example 52

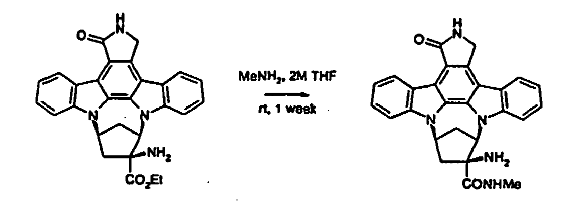

To 50 (0.33 g, 0.71 mmol) was added a solution of methylamine in THF (2M; 100 mL). The reaction mixture was stirred at rt for 1 week (controlled by HPLC) then concentrated in vacuo. The crude was purified by chromatography on silica gel (eluent: dichloromethane / ethyl acetate 7/3 then 1/1 followed by methanol / ethyl acetate) to give 0.25 g of the desired compound (77% yield).

To a solution of the base (0.3g, 0.66 mmol) in acetone was added a solution of HCl in ethanol. The obtained mixture was concentrated in vacuo and the salt was titurated in ethyl acetate, filtered off, washed with ethyl acetate and dried in vacuo at 40°C to give 0.262 g (81 %) of the desired compound.

¹H-NMR (300 MHz, DMSO-*d*_{*6*}): δ 1.28 (2H, s, NH₂), 1.41 (1H, d, 5-CH₂), 2.61 (1H, d, 5-CH₂), 2.73 (3H, d, NH-CH₃), 3.11 (1H, dd, 2-CH₂), 3.23 (1H, dd, 2-CH₂), 4.99 (2H, s, lactam-CH₂), 5.41 (1H, d, 3-CHN), 5.76 (1H, m, 1-CHN), 7.25 (1H, t, Harom), 7.35 (1H, t, Harom), 7.48 (1H, m, Harom), 7.54 (1H, m, Harom), 7.76 (1H, d, Harom), 7.81 (1H, d, Harom), 8.10 (2H, m, Harom, CONHMe), 8.55 (1H, s, NH), 9.26 (1H, d, Harom). MS (APCI) m/z 450 [M+H]⁺.

### Example 53

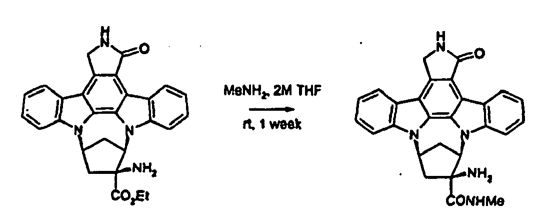

To 51 (0.62 g, 1.33 mmol) was added a solution of methylamine in THF (2M; 100 mL). The reaction mixture was stirred at rt for 1 week (controlled by HPLC) then concentrated in vacuo. The crude was purified by chromatography on silica gel (eluent: dichloromethane / ethyl acetate 7/3 then 1/1 followed by methanol / ethyl acetate) to give 0.36 g of the desired compound (60% yield).

To a solution of the base (0.185 g, 0.41 mmol) in acetone was added a solution of HCl in ethanol. The obtained mixture was concentrated in vacuo and the salt was titurated in ethyl acetate, filtered off, washed with ethyl acetate and dried in vacuo at 40°C to give 0.188 g (94%) of the desired compound.

¹H-NMR (300 MHz, DMSO-*d*_{*6*}): δ 1.28 (2H, s, NH₂), 1.49 (1H, d, 5-CH₂), 2.61 (1H, d, 5-CH₂), 2.73 (3H, d, NH-CH₃), 3.11 (1H, dd, 2-CH₂), 3.23 (1H, dd, 2-CH₂), 4.97 (2H, s, lactam-CH₂), 5.41 (1H, d, 3-CHN), 5.78 (1H, m, 1-CHN), 7.28 (1H, t, Harom), 7.34 (1H, t, Harom), 7.47 (1H, m, Harom), 7.53 (1H, m, Harom), 7.67 (1H, d, Harom), 7.88 (1H, d, Harom), 8.07 (1H, m, Harom) 8.12, (1H, broad, CONHMe), 8.55 (1H, s, NH), 9.31 (1H, d, Harom). MS (APCI) m/z 450 [M+H]⁺.

### Example 54

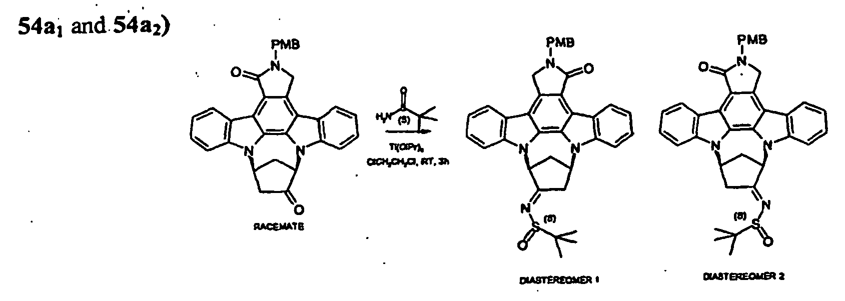

To a stirred mixture of the PMB-protected ketone (1.6 g, 3.1 mmol) in dichloroethane (100 mL) at rt was added titanium isopropylate (11.1 mL, 37.5 mmol) followed by (S)-t-butylsulfinamide (2.3g, 18.7 mmol). The reaction mixture was heated under reflux for 3h. After cooling down to rt, water was added, the formed precipitate was filtered off and thoroughly washed with dichloromethane and THF. The filtrate was washed with brine followed by water. The organic layer was dried over sodium sulfate and concentrated in vacuo. After purification by chromatography on silica (eluent: AcOEt / CH₂Cl₂ = 95/5), 0.52g of diastereomer 1, **54a**_{**1**}**,** (26 %) and 0.32g of of diastereomer 2, **54a**_{**2**}**,** (15.6 %) have been obtained (elution order).

**54a**_{**1**}**:**
¹H-NMR (300 MHz, DMSO-*d*_{*6*}): δ 0.38 (9H, s, C(CH₃)₃), 2.82 (1H, dd, 5-CH₂), 3.22 (2H, m, 2-CH₂, 5-CH₂), 3.49 (1H, m, 2-CH₂), 3.73 (3H, s, OCH3), 4.83 (2H, s, lactam-CH₂), 4.93 (2H, s, CH₂-PMB), 6.05 (2H, m, 3-CHN, 1-CHN), 6.93 (2H, d, Harom), 7.32 (4H, m, Harom), 7.54 (2H, m, Harom), 7.85 (1H, d, Harom), 7.96 (1H, d, Harom), 8.02 (1H, d, Harom), 9.34 (1H, d, Harom). MS (APCI) m/z 615 [M+H]⁺.

**54a**_{**2**}**:**
¹H-NMR (300 MHz, DMSO-*d*_{*6*}): δ 0.85 (9H, s, C(CH₃)₃), 2.85 (1H, dd, 5-CH₂), 3.22 (2H, m, 2-CH₂, 5-CH₂), 3.58 (1H, m, 2-CH₂), 3.72 (3H, s, OCH3), 4.80 (1H, d, lactam-CH₂), 4.86 (1H, d, lactam-CH₂), 4.96 (2H, s, CH₂-PMB), 6.06 (2H, m, 3-CHN, 1-CHN), 6.93 (2H, d, Harom), 7.31 (4H, m, Harom), 7.54 (2H, m, Harom), 7.83 (1H, d, Harom), 7.99 (2H, m, Harom), 9.33 (1H, d, Harom). MS (APCI) m/z 615 [M+H]⁺.

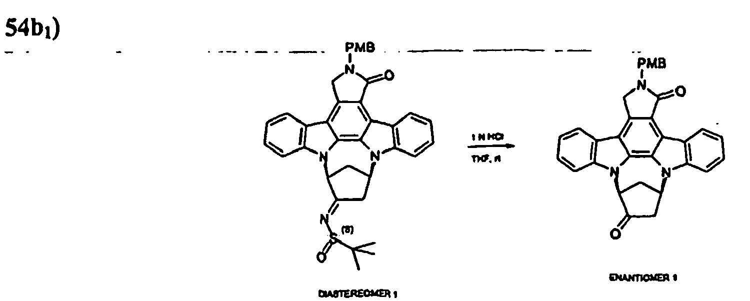

A solution of **54a**_{**1**} (0.52 g, 0.85 mmol) in THF (20 mL) was treated with 1 N HCl solution (20 mL) at rt for 3 days. After complete reaction (TLC monitoring), the reaction mixture was concentrated in vacuo. The concentrate was dissolved in ethyl acetate, washed with water and brine. The organic layer was dried over sodium sulfate and concentrated in vacuo. 0.45 g of pure chiral 54b₁ were obtained (quant. yield).

### Chiral HPLC:

Column: Daicel Chiralpak AD 10 µm, 250 x 4.6 mm

Solvent: isocratic 90 acetonitrile /10 ethanol / 0.1 diethylamine

Flow Rate: 0.5 mL /min

UV-detection: 290 nm

Retention Time: 8.99 min

¹H-NMR (300 MHz, DMSO-*d*_{*6*}): δ 2.45 (1H, dd, 5-CH₂), 2.98 (1H, dd, 5-CH₂), 3.20 (1H, dd, 2-CH₂), 3.49 (1H, m, 2-CH₂), 3.71 (3H, s, OCH3), 4.78 (1H, d, lactam-CH₂), 4.84 (1H, d, lactam-CH₂), 4.93 (2H, s, CH₂-PMB), 5.54 (1H, d, 3-CHN), 6.11 (1H, t, 1-CHN), 6.91n (2H, d, Harom), 7.32 (4H, m, Harom), 7.54 (2H, m, Harom), 7.82 (1H, d, Harom), 7.92 (1H, d, Harom), 8.00 (1H, d, Harom), 9.35 (1H, d, Harom). MS (APCI) m/z 512 [M+H]⁺.

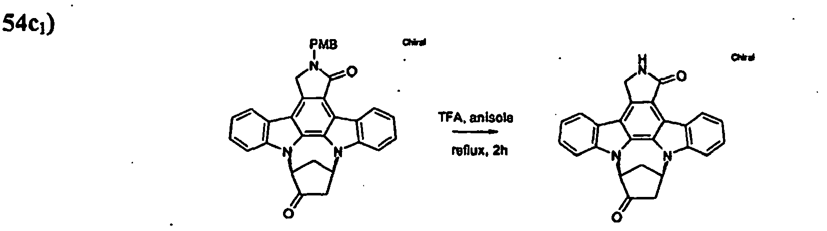

A mixture of **54b**_{**1**} (0.45 g, 0.88 mmol) in anisole (4 mL) was treated with trifluoroacetic acid (40 mL) and heated under reflux for 2h. The reaction mixture was concentrated in vacuo. The residue was diluted with ethylacetate and THF and washed with aq. sat. NaHCO₃-solution, water and brine. The organic emulsion layer was concentrated in vacuo. The residue was diluted with ethanol, and the resulting precipitate was filtered off, washed with diethyl ether and dried in vacuo to obtain compound 54c₁ (0.33 g, 96% yield).

¹H-NMR (300 MHz, DMSO-*d*_{*6*}): δ 2.33 (1H, dd, 5-CH₂), 2.98 (1H, dd, 5-CH₂), 3.21 (1H, dd, 2-CH₂), 3.50 (1H, m, 2-CH₂), 4.96 (2H, s, lactam-CH₂), 5.55 (1H, d, 3-CHN), 6.12 (1H, t, 1-CHN), 7.29 (1H, t, Harom), 7.37 (1H, t, Harom), 7.52 (1H, m, Harom) 7.58 (1H, m, Harom), 7.81 (1H, d, Harom), 7.94 (1H, d, Harom), 8.08 (1H, d, Harom), 8.60 (1H, s, NH), 9.29 (1H, d, Harom) . MS (APCI) m/z 392 [M+H]⁺.

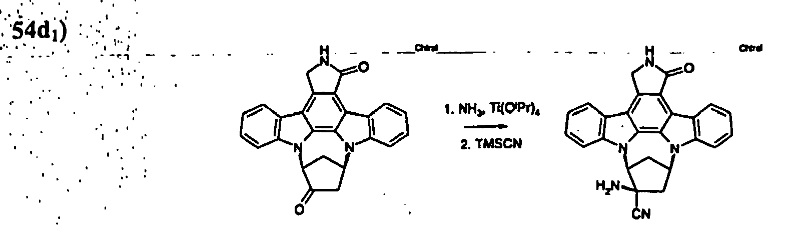

To a stirred mixture of 54c₁ (0.33 g, 0.84 mmol) in dichloroethane (20 mL) at rt was added titanium isopropylate (0.3 mL, 0.93 mmol) followed by a 7 N solution of ammonia in methanol (1.2 mL, 8.4 mmol). After 2h, trimethylsilyl cyanide (0.14 mL, 1.0 mmol) was added. The reaction mixture was stirred at rt for 18h. Water (10 mL) was added, and the mixture was extracted 3x by dichloromethane. The combined organic layers were dried over sodium sulfate and concentrated in vacuo. 0.36g of aminonitrile 54d₁ has been obtained (quant. yield).

¹H-NMR (300 MHz, DMSO-d₆): δ 1.58 (1H, m, 5-CH₂), 2.38 (2H, s, NH₂), 2.75 (1H, m, 5-CH₂), 3.25 (2H, m, 2-CH₂), 4.98 (2H, s, lactam-CH₂) , 5.73 (1H, d, 3-CHN), 5.94 (1H, dd, 1-CHN), 7.26 (1H, t, Harom), 7.37 (1H, t, Harom), 7.53 (2H, m, Harom), 7.80 (1H, d, Harom), 8.05 (2H, m, Harom), 8.58 (1H, s, NH), 9.26 (1H, d, Harom). MS (ESI) m/z 418 [M+H]⁺.

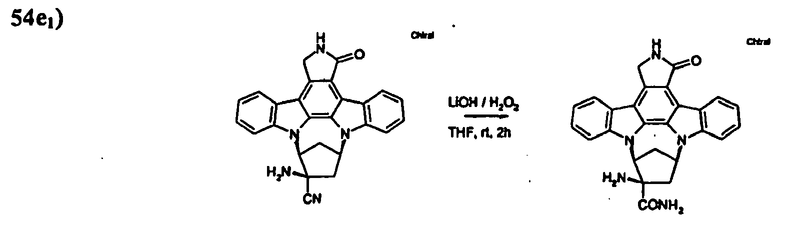

To a solution of 54d₁ (0.36 g, 0.84 mmol) in THF (50 mL) at rt was added lithium hydroxide hydrate (0.35 g, 8.4 mmol) followed by 5 ml of a 30% hydrogen peroxide solution. The reaction mixture was stirred for 2h at rt, then concentrated in vacuo. The residue was diluted with water, the formed precipitate was filtered off, washed with water and dried in vacuo at 50°C to yield 0.3 g (82 %) of the expected compound 54e₁.

¹H-NMR (300 MHz, DMSO-*d*_{*6*}): δ 1.33 (2H, s, NH₂), 1.45 (1H, d, 5-CH₂), 2.60 (1H, d, 5-CH₂), 3.13 (1H, dd, 2-CH₂), 3.23 (1H, dd, 2-CH₂), 5.00 (2H, s, lactam-CH₂), 5.45 (1H, d, 3-CHN), 5.76 (1H, m, 1-CHN), 7.26 (1H, t, Harom), 7.36 (1H, t, Harom), 7.51 (2H, m, Harom), 7.62 (2H, broad, CONH₂), 7.75 (1H, d, Harom), 7.80 (1H, d, Harom), 8.09 (1H, d, Harom), 8.56 (1H, s, NH), 9.27 (1H, d, Harom). MS (APCI) m/z 436 [M+H]⁺.

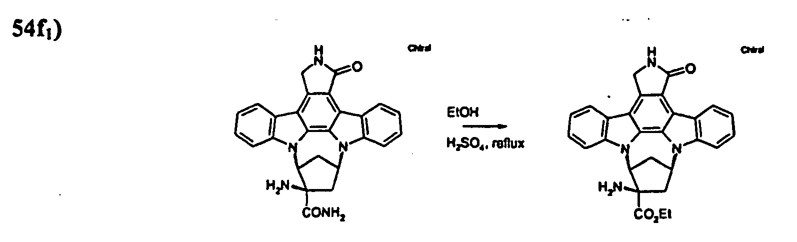

To a solution of 54e₁ (0.30 g, 0.69 mmol) in ethanol (20 mL) at rt was added dropwise concentrated sulfuric acid (2 mL). The reaction mixture was stirred under reflux for 24h then poured onto ice-water (50 mL) and neutralized by sodium carbonate. The aqueous layer was extracted 3x by ethyl acetate. The combined organic layers were washed with brine, dried over sodium sulfate and concentrated in vacuo to obtain 0.27 g of the compound 54f₁ (84%).

¹H-NMR (300 MHz, DMSO-*d*_{*6*}): δ 1.36 (3H, t, OCH₂CH₃), 1.48 (1H, d, 5-CH₂), 2.61 (1H, d, 5-CH₂), 3.15 (1H, dd, 2-CH₂), 3.23 (1H, dd, 2-CH₂), 4.31 (2H, m, OCH₂CH₃) 4.98 (2H, s, lactam-CH₂), 5.62 (1H, d, 3-CHN), 5.79 (1H, m, 1-CHN), 7.25 (1H, t, Harom), 7.35 (1H, t, Harom), 7.48 (1H, m, Harom), 7.55 (1H, m, Harom), 7.75 (1H, d, Harom), 7.81 (1H, d, Harom), 8.08 (1H, d, Harom), 8.56 (1H, s, NH), 9.26 (1H, d, Harom). MS (APCI) m/z 465 [M+H]⁺.

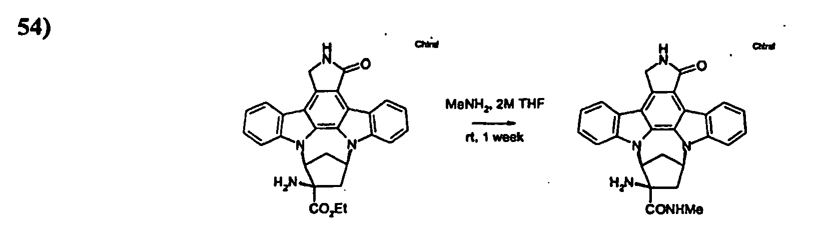

To the previously prepared amino ethylester 54f₁ (0.27 g, 0.58 mmol) was added a solution of methylamine in THF (2M; 50 mL). The reaction mixture was stirred at rt for 1 week (controlled by HPLC) then concentrated in vacuo. The crude was purified by chromatography on silica gel (eluent: dichloromethane / ethyl acetate 7/3 then 1/1 followed by methanol / ethyl acetate) to give 0.045 g of the desired compound 54 (17% yield).

¹H-NMR (300 MHz, DMSO-*d*_{*6*}): δ 1.28 (2H, s, NH₂), 1.41 (1H, d, 5-CH₂), 2.61 (1H, d, 5-CH₂), 2.73 (3H, d, NH-CH₃), 3.11 (1H, dd, 2-CH₂), 3.23 (1H, dd, 2-CH₂), 4.99 (2H, s, lactam-CH₂), 5.41 (1H, d, 3-CHN), 5.76 (1H, m, 1-CHN), 7.25 (1H, t, Harom), 7.35 (1H, t, Harom), 7.48 (1H, m, Harom), 7.54 (1H, m, Harom). 7.76 (1H, d, Harom), 7.81 (1H, d, Harom). 8.10 (2H, m, Harom, CONHMe), 8.55 (1H, s, NH), 9.26 (1H, d, Harom). MS (APCI) m/z 450 [M+H]⁺

Chiral HPLC:
Column: Daicel Chiralpak AD 10 µm, 250 x 4.6 mm
Solvent: isocratic 90 acetonitrile / 10 ethanol / 0.1 diethylamine
Flow Rate: 0.5 mL / min .
UV-detection: 290 nm
Retention Time: 14.3 min

### Example 55

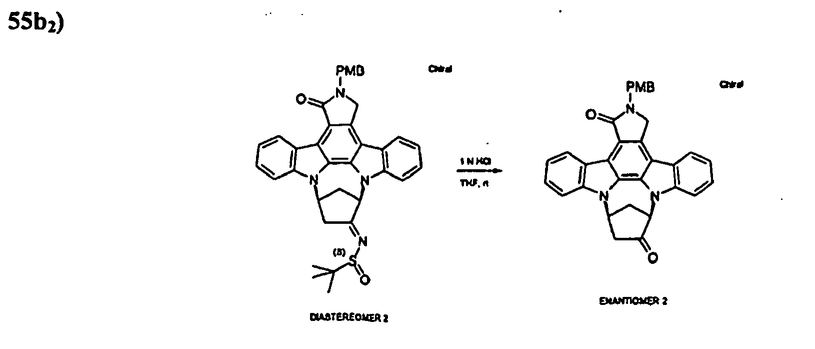

A solution of diastereomeric imine **54b**_{**1**} (0.32 g, 0.52 mmol) in THF (20 mL) was treated with 1 N HCl solution (20 mL) at rt for 3 days. After complete reaction (TLC monitoring), the reaction mixture was concentrated in vacuo. The concentrate was dissolved in ethyl acetate, washed with water and brine. The organic layer was dried over sodium sulfate and concentrated in vacuo. 0.29 g of the chiral PMB-protected ketone **55b**_{**2**} were obtained (quant. yield).

Chiral HPLC:
Column: Daicel Chiralpak AD 10 µm, 250 x 4.6 mm
Solvent: isocratic 90 acetonitrile / 10 ethanol / 0.1 diethylamine
Flow Rate: 0.5 mL / min
UV-detection: 290 nm
Retention Time: 9.87 min

¹H-NMR (300 MHz, DMSO-*d*_{*6*}): δ 2.45 (1H, dd, 5-CH₂), 2.98 (1H, dd, 5-CH₂), 3.20 (1H, dd, 2-CH₂) 3.49 (1H, m, 2-CH₂), 3.71 (3H, s, OCH3), 4.78 (1H, d, lactam-CH₂), 4.84 (1H, d, lactam-CH₂), 4.93 (2H, s, CH₂-PMB), 5.54 (1H, d, 3-CHN), 6.11 (1H, t, 1-CHN), 6.91n (2H, d, Harom), 7.32 (4H, m, Harom), 7.54 (2H, m, Harom), 7.82 (1H, d, Harom), 7.92 (1H, d, Harom), 8.00 (1H, d, Harom), 9.35 (1H, d, Harom). MS (APCI) m/z 512 [M+H]⁺.

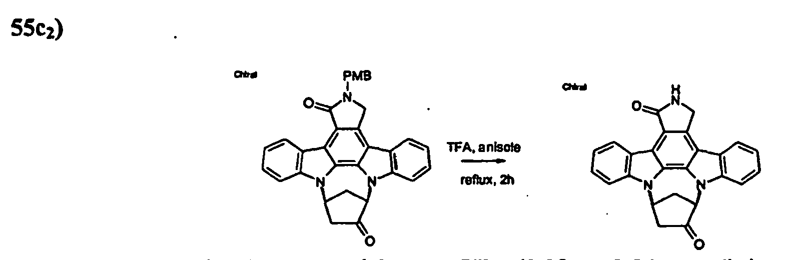

A mixture of previously prepared ketone **55b**_{**2**} (0.29 g, 0.56 mmol) in anisole (3 mL) was treated with trifluoroacetic acid (30 mL) and heated under reflux for 2h. The reaction mixture was concentrated in vacuo. The residue was diluted with ethyl acetate and THF and washed by aq. sat. NaHCO₃-solution, water and brine. The organic emulsion layer was concentrated in vacuo. The residue was diluted with ethanol, and the resulting precipitate was filtered off, washed with diethyl ether and dried in vacuo to obtain compound 55c₂ (0.195 g, 89%).

¹H-NMR (300 MHz, DMSO-*d*_{*6*}): δ 2.33 (1H, dd, 5-CH₂. 2.98 (1H, dd, 5-CH₂, 3.21 (1H, dd, 2-CH₂), 3.50 (1H, m, 2-CH₂), 4.96 (2H, s, lactam-CH₂), 5.55 (1H, d, 3-CHN), 6.12 (1H, t, 1-CHN), 7.29 (1H, t, Harom), 7.37 (1H, t, Harom), 7.52 (1H, m, Harom) , 7.58 (1H, m, Harom) , 7.81 (1H,d,Harom), 7.94 (1H,d, Harom), 8.08 (1H,d, Harom), 8.60 (1H,s,NH), 9.29 (1H,d, Harom). MS (APCI) m/z 392 [M+H]⁺.

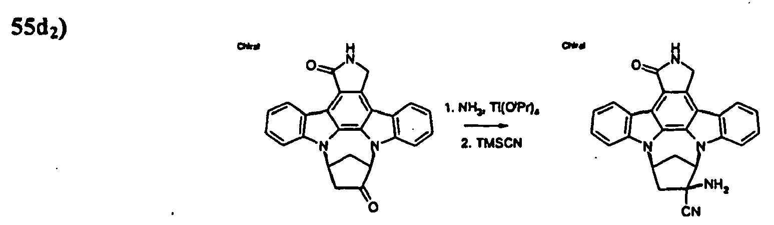

To a stirred mixture of **55c**_{**2**} (0.195 g, 0.5 mmol) in dichloroethane (20 mL) at rt was added titanium isopropylate (0.16 mL, 0.55 mmol) followed by a 7 N solution of ammonia in methanol (0.7 mL, 5.0 mmol). After 2h, trimethylsilyl cyanide (0.04 mL, 0.6 mmol) was added. The reaction mixture was stirred at rt for 18h. Water (10 mL) was added, and the mixture was extracted 3x by dichloromethane. The combined organic layers were dried over sodium sulfate and concentrated in vacuo. 0.22g of aminonitrile 55d₂ has been obtained (quant.).

¹H-NMR (300 MHz, DMSO-*d*_{*6*}): δ 1.58 (1H, m, 5-CH₂), 2.38 (2H, s, NH₂), 2.75 (1H, m, 5-CH₂), 3.25 (2H, m, 2-CH₂), 4.98 (2H, s, lactam-CH₂), 5.73 (1H, d, 3-CHN), 5.94 (1H, dd, 1-CHN), 7.26 (1H, t, Harom), 7.37 (1H, t, Harom), 7.53 (2H, m, Harom), 7.80 (1H, d, Harom), 8.05 (2H, m, Harom), 8.58 (1H, s, NH), 9.26 (1H, d, Harom). MS (ESI) m/z 418 [M+H]⁺.

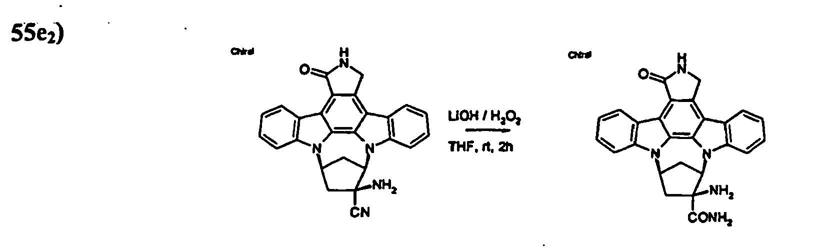

To a solution of 55d₂ (0.22 g, 0.5 mmol) in THF (50 mL) at rt was added lithium hydroxide hydrate (0.21 g, 5.0 mmol) followed by 5 ml of a 30% hydrogen peroxide solution. The reaction mixture was stirred for 2h at rt, then concentrated in vacuo. The residue was diluted with water, the formed precipitate was filtered off, washed with water and dried in vacuo at 50°C to yield 0.16 g (73 %) of the expected compound 55e₂.

¹H-NMR (300 MHz, DMSO-d₆): δ 1.33 (2H, s, NH₂), 1.45 (1H, d, 5-CH₂), 2.60 (1H, d, 5-CH₂), 3.13 (1H, dd, 2-CH₂), 3.23 (1H, dd, 2-CH₂), 5.00 (2H, s, lactam-CH₂), 5.45 (1H, d, 3-CHN), 5.76 (1H, m, 1-CHN), 7.26 (1H, t, Harom), 7.36 (1H, t, Harom), 7.51 (2H, m, Harom), 7.62 (2H, broad, CONH₂), 7.75 (1H, d, Harom), 7.80 (1H, d, Harom), 8.09 (1H, d, Harom), 8.56 (1H, s, NH), 9.27 (1H, d, Harom). MS (APCI) m/z 436 [M+H]⁺.

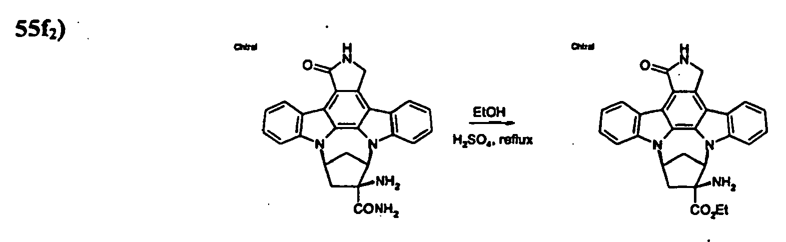

To a solution of 55e₂ (0.16 g, 0.37 mmol) in ethanol (20 mL) at rt was added dropwise concentrated sulfuric acid (2 mL). The reaction mixture was stirred under reflux for 24h then poured onto ice-water (50 mL) and neutralized by sodium carbonate. The aqueous layer was extracted 3x by ethyl acetate. The combined organic layers were washed with brine, dried over sodium sulfate and concentrated in vacuo to obtain 0.17 g of compound 55f₂ (99%).

¹H-NMR (300 MHz, DMSO-d₆): δ 1.36 (3H, t, OCH₂CH₃), 1.48 (1H, d, 5-CH₂), 2.61 (1H, d, 5-CH₂), 3.15 (1H, dd, 2-CH₂), 3.23 (1H, dd, 2-CH₂), 4.31 (2H, m, OCH₂CH₃) 4.98 (2H, s, lactam-CH₂), 5.62 (1H, d, 3-CHN), 5.79 (1H, m, 1-CHN), 7.25 (1H, t, Harom), 7.35 (1H, t, Harom), 7.48 (1H, m, Harom), 7.55 (1H, m, Harom), 7.75 (1H, d, Harom), 7.81 (1H, d, Harom), 8.08 (1H, d, Harom), 8.56 (1H, s, NH), 9.26 (1H, d, Harom), MS (APCI) m/z 465 [M+H]⁺.

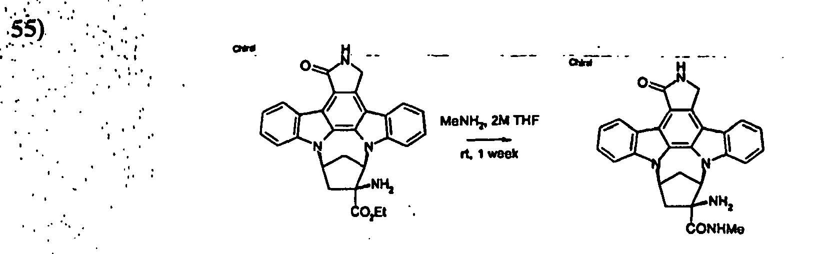

To the previously prepared amino ethyl ester (0.17 g, 0.37 mmol) was added a solution of methylamine in THF (2M; 50 mL). The reaction mixture was stirred at rt for 1 week (controlled by HPLC) then concentrated in vacuo. The crude was purified by chromatography on silica gel (eluent: dichloromethane / ethyl acetate 7/3 then 1/1 followed by methanol / ethyl acetate) to give 0.035 g of 55 (21% yield).

¹H-NMR (300 MHz, DMSO-*d*_{*6*}): δ 1.28 (2H, s, NH₂), 1.41 (1H, d, 5-CH₂), 2.61 (1H, d, 5-CH₂), 2.73 (3H, d, NH-CH₃), 3.11 (1H, dd, 2-CH₂), 3.23 (1H, dd, 2-CH₂), 4.99 (2H, s, lactam-CH₂), 5.41 (1H, d, 3-CHN), 5.76 (1H, m, 1-CHN), 7.25 (1H, t, Harom), 7.35 (1H, t, Harom), 7.48 (1H, m, Harom), 7.54 (1H, m, Harom), 7.76 (1H, d, Harom), 7.81 (1H, d, Harom), 8.10 (2H, m, Harom, CONHMe), 8.55 (1H, s, NH), 9.26 (1H, d, Harom). MS (APCI) m/z 450 [M+H]⁺.

Chiral HPLC:
Column: Daicel Chiralpak AD 10 µm, 250 x 4.6 mm
Solvent: isocratic 90 acetonitrile /10 ethanol / 0.1 diethylamine
Flow Rate: 0.5 mL / min
UV-detection: 290 nm
Retention Time: 15.9 min

A list of all compounds prepared is provided in Table 1.

**Table 1. Structures of compounds 1-55.**

| **Compound** | **R1** | **R2** | **R3** | **R4** | **R5** | **R6** | **R7** | **R8, R9** | **R10, R11** | **R12** |
|---|---|---|---|---|---|---|---|---|---|---|
| 1 | NH₂ | H | H | H | H | H | H | O | O | PMB |
| 2 | NHMe | H | H | H | H | H | H | O | O | H |
| 3 | NMor | H | H | H | H | H | H | O | O | H |
| 4 | NH₂ | CN | H | H | H | H | H | O | O | H |
| 5 | NH₂ | CONH₂ | H | H | H | H | H | O | O | H |
| 6 | NH₂ | COOEt | H | H | H | H | H | O | O | H |
| 7 | NH₂ | COOH | H | H | H | H | H | O | O | H |
| 8 | NH₂ | COOMe | H | H | H | H | H | O | O | H |
| 9 | NH₂ | COOEtNH₂ | H | H | H | H | H | O | O | H |
| 10 | NH₂ | CONHMe | H | H | H | H | H | O | O | H |
| 11 | NH₂ | CONHEtOH | H | H | H | H | H | O | O | H |
| 12 | NH₂ | CONH₂ | H | 5OH | H | 5OH | H | O | O | H |
| 13 | NHHyd | COHyd | H | H | H | H | H | O | O | PMB |
| 14 | NHHydMe | COHydMe | H | H | H | H | H | O | O | PMB |
| 15 | N₃ | H | OH | H | H | H | H | O | O | H |
| 16 | NH₂ | H | OH | H | H | H | H | O | O | H |
| 17 | NTzCOOMe | H | OH | H | H | H | H | O | O | H |
| 18 | NTzCOOH | H | OH | H | H | H | H | O | O | H |
| 19 | NHOxa | H | Oxa | H | H | H | H | O | O | H |
| 20 | NH₂ | CONH₂ | H | H | H | H | H | O | H | H |
| 21 | NHMe | H | H | H | H | H | H | O | O | PMB |
| 22 | NHBn | H | H | H | H | H | O | O | O | PMB |
| 23 | NHBn | H | H | H | H | H | H | O | O | H |
| 24 | NPip | H | H | H | H | H | H | O | O | PMB |
| 25 | NHEtOH | H | H | H | H | H | H | O | O | PMB |
| 26 | NMor | H | H | H | H | H | H | O | O | PMB |
| 27 | NHMe | CN | H | H | H | H | H | O | O | PMB |
| 28 | NH₂ | CN | H | H | H | H | H | O | O | PMB |
| 29 | NPip | H | H | H | H | H | H | O | O | H |
| 30 | NHBu | H | H | H | H | H | H | O | O | PMB |
| 31 | NMe₂ | H | H | H | H | H | H | O | O | PMB |
| 32 | NHCOCF₃ | CN | H | H | H | H | H | O | O | H |
| 33 | NH₂ | H | OH | H | H | H | H | O | O | PMB |
| 34 | NH₂ | CONH₂ | H | H | H | H | H | O | O | PMB |
| 35 | NH₂ | COOEt | H | H | H | H | H | O | O | PMB |
| 36 | NH₂ | COOH | H | H | H | H | H | O | O | PMB |
| 37 | NH₂ | CONHEtOH | H | H | H | H | H | O | O | EtOH |
| 38 | NTzOH | H | OH | H | H | H | H | O | O | H |
| 39 | NH₂ | CN | H | 50Me | H | 50Me | H | O | O | H |
| 40 | NH₂ | CONH₂ | H | 50Me | H | 50Me | H | O | O | H |
| 41 | NH₂ | CONHEt | H | H | H | H | H | O | O | H |
| 42 | NH₂ | CONHBu | H | H | H | H | H | O | O | H |
| 43 | NH₂ | COOEtNHBoc | H | H | H | H | H | O | O | H |
| 44 | NHOxa | H | Oxa | H | H | H | H | O | O | PMB |
| 45 | NHMe | CN | H | H | H | H | H | O | O | H |
| 46 | NH₂ | CN | H | H | H | H | H | O | H | H |
| 47 | NH₂ | CN | H | H | H | H | H | H | O | H |
| 48 | NH₂ | CONH₂ | H | H | H | H | H | H | O | H |
| 49 | NH₂ | CONH₂ | H | H | H | H | H | O | H | H |
| 50 | NH₂ | COOEt | H | H | H | H | H | O | H | H |
| 51 | NH₂ | COOEt | H | H | H | H | H | H | O | H |
| 52 | NH₂ | CONHMe | H | H | H | H | H | O | H | H |
| 53 | NH₂ | CONHMe | H | H | H | H | H | H | O | H |
| 54 | NH₂ | CONHMe | H | H | H | H | H | O | H | H |
| 55 | NH₂ | CONHMe | H | H | H | H | H | O | H | H |

### Example 56

### Biological characterization

The inhibition of enzymatic activity by the nitrogen-substituted, N,N-bridged carbacyclic indolocarbazoles of the present invention can be determined using, for example, the following assays:
1. Extracellular signal Regulated Kinase 2 inhibition assay (ERK2)
2. Protein Kinase A inhibition assay (PKA)
3. Protein Kinase C inhibition assay (PKC)
4. Glycogen Synthase Kinase 3β inhibition assay (GSK3β).

Description of these assays are set in the examples 56A-D below. The results are intended to be illustrative and not to be construed as limiting the scope of the disclosure. For convenience, certain abbreviations are defined as follows: "µg" for microgram, "mg" for milligram, "g" for gram, "µL" for microliter, "mL" for milliliter, "L" for liter, "nM" for nanomolar, "µM" for micromolar, "mM" for millimolar, and "M" for molar. The compounds of the present invention preferably demonstrate measurable inhibition in the assays described herein at a concentration of about 100 µM to about 10 µM. More preferably, compounds of the present invention demonstrate measurable inhibition at a concentration of about 10 µM to about 1 µM. Even more preferably, compounds of the present invention demonstrate measurable inhibition at concentrations lower than 1 µM.

A list of in vitro activities on the four above mentioned kinases, expressed as percentage inhibition at 10 µM concentration of tested compounds, is provided for most compounds prepared in **Table 2.**

**Table 2. Biological properties of compounds 1-8, 10-18, 21-38, 41.**

| | **% Inhibition**^{**a**} | | | |
|---|---|---|---|---|
| **Compound** | **ERK2** | **PKA** | **PKC** | **GSK3β** |
| 1 | 30,94 | 17,64 | 49,73 | NA^{b} |
| 2 | 65,28 | 68,40 | 90,78 | 78,21 |
| 3 | 39,90 | 26,12 | 75,80 | 19,31 |
| 4 | 94,12 | 89,62 | 78,10 | 98,49 |
| 5 | 92,28 | 96,79 | 91,62 | 100,49 |
| 6 | 61,28 | 59,38 | 83,97 | 94,16 |
| 7 | 94,67 | 62,49 | 85,71 | 98,97 |
| 8 | 50,22 | 70,78 | 59,42 | 96,87 |
| 10 | 93,55 | 95,34 | 90,75 | 99,19 |
| 11 | 93,63 | 90,46 | 95,94 | 99,21 |
| 12 | 98,06 | 99,86 | 96,63 | 100,27 |
| 13 | 24,74 | 13,23 | 13,67 | NA |
| 14 | 46,65 | 16,61 | 34,01 | NA |
| 15 | 94,59 | 79,82 | 88,68 | 96,92 |
| 16 | 98,20 | 84,76 | 93,34 | 98,48 |
| 17 | NA | NA | 96,75 | 12,08 |
| 18 | NA | 11,51 | 21,55 | 75,94 |
| 21 | 45,99 | 14,05 | 51,90 | NA |
| 22 | 37,22 | 17,87 | 42,90 | NA |
| 23 | 53,66 | 27,53 | 70,41 | 72,56 |
| 24 | 28,09 | 10,67 | 28,18 | NA |
| 25 | 33,51 | NA | 15,94 | NA |
| 26 | 67,56 | NA | 10,18 | NA |
| 27 | 63,52 | 30,19 | 32,18 | 18,28 |
| 28 | 48,74 | NA | 28,19 | 25,64 |
| 29 | 56,44 | 18,38 | 46,63 | 54,14 |
| 30 | 44,61 | NA | 29,04 | NA |
| 31 | 42,42 | NA | 21,91 | NA |
| 32 | 44,03 | 23,81 | 49,33 | 72,20 |
| 33 | 61,21 | 16,69 | 50,80 | 73,02 |
| 34 | 67,50 | 44,48 | 33,31 | 27,64 |
| 35 | 63,78 | NA | 28,47 | NA |
| 36 | 73,44 | 21,80 | 65,10 | 82,71 |
| 37 | 35,09 | 19,81 | 53,92 | 80,46 |
| 38 | 38,09 | NA | 67,40 | 90,84 |
| 41 | 72,93 | 86,43 | 83,88 | 95,80 |

| | | | | |
|---|---|---|---|---|
| ^{a}10 µM compound; ^{b} NA=<10% inhibition. | | | | |

### Example 56A - Inhibition of Extracellular Signal Regulated Kinase 2 (ERK 2) Activity

The ERK2 kinase activity assay utilizes a radioactivity-based format in a 96-well PCR plate with radioactive readout. The ERK2 activity was assayed in a 25µL assay mixture containing 25 mM HEPES (pH 7.0), 250 µM ATP, 1 mM MgCl₂, 1 mM DTT, 2% DMSO, 150 ng/mL Myelin Basic Protein (MBP, Sigma M-1891), and 13.6 ng/mL His tagged ERK2 (specific activity = 38.1 nmol/min*mg). Compounds were screened for inhibition of the ERK2 kinase activity at a concentration of 10 µM. The kinase reaction was allowed to proceed at 37°C for 30 minutes, then reaction was stopped by the addition of 5 µL of 0.5M EDTA (pH 8). 15 µL of each solution were then spotted onto the corresponding square of a filtermat (8x12 glassfibre mat 90x120 mm, PE-Wallac 1450-421). The filtermat was allowed to dry and washed once with 10% TCA, 2% PPA, 500 mM NaCl each for 30 minutes at rt. Two further washings in 10% TCA and 2% PPA for 30 minutes were performed, then a final 30 minute wash in 99% EtOH at rt was done and the filtermat was air dried. The dry filtermat was then placed in a sample bag with a Meltilex sheet (Melt-on scintillator sheets 73x109 mm, PE-Wallac 1450-441 for filtermat A) over the filtermat. The bag was trimmed to fit into the microplate heatsealer (PE-Wallac 1495-021). The heatsealer is used to melt the Meltilex on the filtermat. The bag containing the filtermat and the melted Meltilex was then placed into a filter cassette and counted using the Microbeta Jet Scintillation and Luminescence Counter PE-Wallac 1450. The results for compounds showing a >50% inhibition at the tested concentration are summarized in Table 1.

### Example 56B - Inhibition of Protein Kinase A Activity

The PKA kinase activity assay utilizes a radioactivity-based format in a 96-well PCR plate with radioactive readout. The PKA activity was assayed in a 25µL assay mixture containing 25 mM HEPES (pH 7.0), 250 µM ATP, 10 mM MgCl₂, 1 mM DTT, 1 mM EDTA (pH 8), 2% DMSO, 250 ng/mL Histone H2B (Roche 223 514), and 2 ng/mL PKA (catalytic subunit, bovine heart, Calbiochem 539486, specific activity = 1170 pmol/min*µg). Compounds were screened for inhibition of the PKA kinase activity at a concentration of 10 µM. The kinase reaction was allowed to proceed at 37°C for 30 minutes, then the reaction was stopped by addition of 5 µL, of 0.5M EDTA (pH 8). The remainder of the protocol is identical to the one reported for ERK2. the results for compounds showing a >50% inhibition at the tested concentration are summarized in Table 1.

### Example 56C - Inhibition of Protein Kinase C Activity

The PKC kinase activity assay utilizes a radioactivity-based format in a 96-well PCR plate with radioactive readout. The PKC activity was assayed in a 25µL assay mixture containing 25 mM HEPES (pH 7.0), 250 µM ATP, 10 mM MgCl₂, 1 mM DTT, 1 mM EDTA (pH 8), 2 mM CaCl₂, 2% DMSO, 250 ng/mL Histone H1 (Roche 1 004 875), and 165 ng/mL PKC (rat brain, Calbiochem 539494, specific activity = 1600 nmol/min*mg). Compounds were screened for inhibition of the PKC kinase activity at a concentration of 10 µM. The kinase reaction was allowed to proceed at 37°C for 30 minutes, then the reaction was stopped by addition of 5 µL of 0.5M EDTA (pH 8). The remainder of the protocol is identical to the one reported for ERK2. The results for compounds showing a >50% inhibition at the tested concentration are summarized in Table 1.

### Example 56D - Inhibition of Glycogen Synthase Kinase 3β Activity

The GSK3β kinase activity assay utilizes a radioactivity-based format in a 96-well PCR plate with radioactive readout. The GSK3β activity was assayed in a 25µL assay mixture containing 8 mM HEPES (pH 7.0), 250 µM ATP, 0.2 mM EDTA (pH 8), 2% DMSO, 250 ng/mL Myelin Basic Protein (MBP, Sigma M-1891), and 12 ng/mL GSK3β (Upstate Discovery, specific activity = 607 nmol/min*GS2 peptide, concentration of 6.05 mg/mL). Compounds were screened for inhibition of the GSK3β kinase activity at a concentration of 10 µM. The kinase reaction was allowed to proceed at 37°C for 30 minutes, then the reaction was stopped by addition of 5 µL of 0.5M EDTA (pH 8).

### Bioavailability characterization

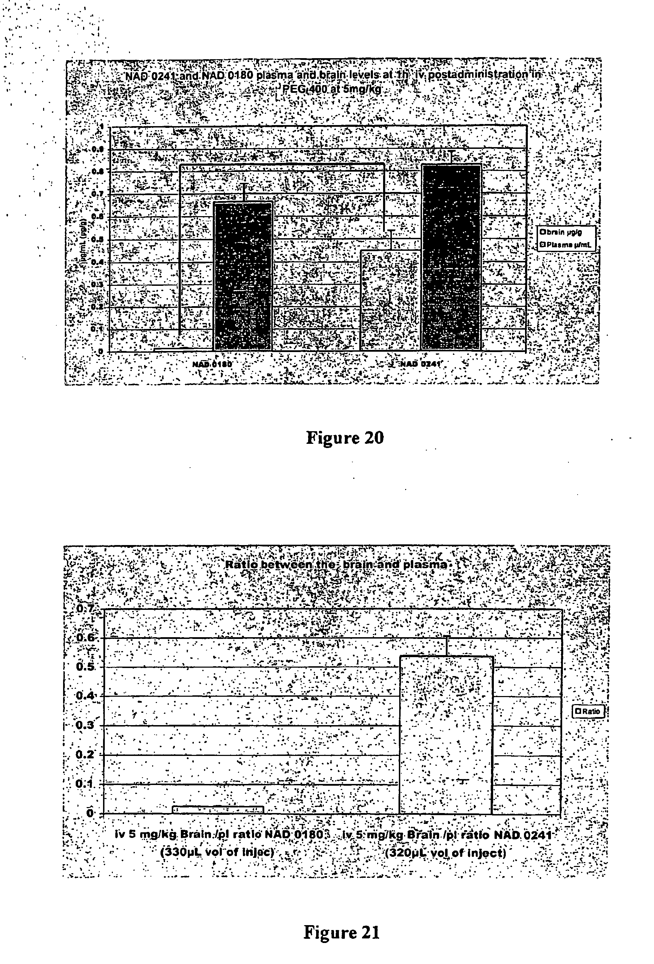

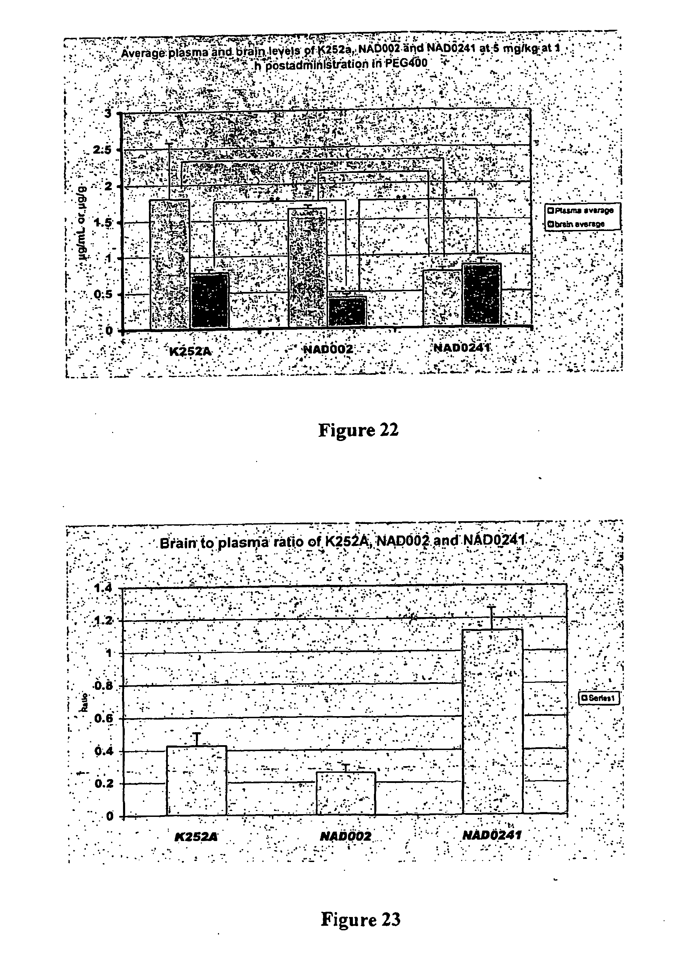

Compounds of the novel series disclosed here display superior capabilities to penetrate the blood-brain barrier in direct comparison with closely related structures of the prior art. Fig. 23 shows that with NAD0241 shortly after administration brain/plasma ratios around 1 are achieved, essentially reflecting full equilibration into the CNS compartment. This represents an improvement of several-fold over the closely related compound NAD002 disclosed in US 6,013,646, and about two-fold over the structurally related natural product K252a. It can be appreciated from Fig. 22 that at equivalent dosing with identical vehicle conditions higher levels of NAD0241 can be achieved in the brain while maintaining lower exposure of the peripheral compartment than with the reference compounds. Fig. 20 and 21 provide a reference for a structurally unrelated mono-glycosylated indolocarbazole compound virtually devoid of blood-brain barrier penetration in spite of plasma levels comparable to NAD0241. The spurious levels of NAD0180 may represent the contaminating contribution from the vascular compartment in the brain.

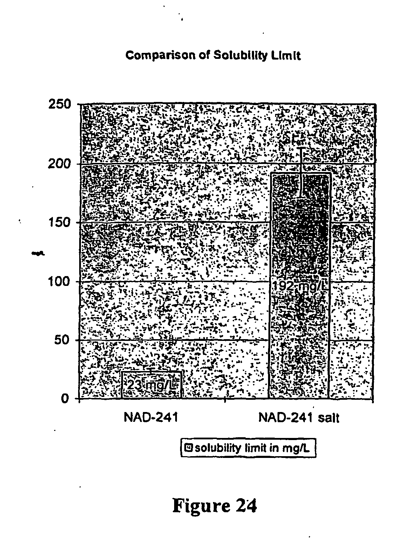

As the novel compounds are readily amenable to formulation in the form of salts, solubility characteristics in vehicles and dissolution characteristics in solid dosages can be improved considerably. Fig. 24 shows the improvement in aqueous solubility by about one order of magnitude by forming the hydrochloride salt of NAD0241.

In summary, the novel compounds provide for targeting kinase-related pathological mechanisms in the CNS with less peripheral side effects and better pharmacoeconomic parameters than comparable compounds of related structure.

The following examples illustrate the superior pharmaceutical features of the new series, represented by NAD0241.

### Example 56E - Determination of brain/plasma ratios for NAD0241 and comparison with NAD0180

*Application af Compound:* 5 mg/kg of NAD 241 and NAD0180 each in a vehicle of 1.5 mL/kg PEG 400 Macrogol Ph. Eur. were applied i.v. through the femoral vein to two groups (n =3) of Wistar Han Rats weighing between 240-270g. The application was performed under anaesthesia, initiated with 3.5 % Isofluorane/O₂, then maintained at 1% during compound application and suture of the vein. 200µL of local anaesthetic lidocaine were locally administered. 1 hour after application of the compound 200µl of blood were obtained by tail vein incision. Immediately following the blood sampling rats were terminated by decapitation after brief CO₂ anaesthesia. Brains were resected and weighed. Plasma was obtained by centrifugation of the tail vein blood at 13000 g for 20 minutes at 4°C.

*Extraction of NAD0241 from Plasma:* In a glass tube 200µl of conc. ammonia solution and 200µl of saturated NaCl solution were mixed with each plasma sample obtained from an equal volume of blood. The mixture was extracted with 2 mL EtOAc (HPLC grade) by vigorous vortexing for 1 minute to form a fine emulsion. After accelerated phase separation by centrifugation at 4000 g for 5-10 minutes at room temperature. The organic phase was collected with a glass pipette and evaporated in a glass vial in a SpeedVac at 50-60°C. The aqueous phase was extracted a second time in an identical fashion, and the organic phase combined with the first extract.

*Extraction of NAD0241 Compound from Brain:* One half of a rat brain was placed in a conical bottom glass tube and homogenized in 500µL of saturated NaCl solution by sonication (Bandelin Sonoplus UW 2070, Berlin) at 55% power for 20 seconds at 4 °C. 200µL of concentrated ammonia was added to the samples. Extraction was then performed with 2x 2 mL EtOAc (HPLC grade) as with plasma samples.

*Extraction of NAD0180 from plasma and brain:* The extraction was performed as described above for NAD0241 with the exception that concentrated ammonia was not added prior to extraction.

*Establishment of Extraction Recovery and Standard Curve for NAD0241 and NAD0180:* 100µL of plasma or 0,5g brain tissue homogenate in saturated NaCl, obtained from control rats, were spiked with NAD0241 and NAD0180, respectively, to producc concentrations ranging from 1 ng/mL to 5 µg/mL. The spiked brain or plasma samples then were then subjected to the extraction and processing as described above. Quantitative analysis of the extracts was performed by HPLC with fluorescence detection. Residues after evaporation of combined organic extracts were taken up in 300 µL of acetonitrile and 100 µL were injected onto a 5 µm RP 18 Select B 12.5x4.6mm column (Merck) at a flow rate of 0.75 mL/min for NAD0241, and 1.250 mL/min for NAD0180 on a C 18 shield symmetry 25 x 4,6 mm column (Waters and Owen). Elution was isocratic at a temperature of 40°C with acetonitrile/water 60/40 (v/v). Fluorescence detection and quantitation by peak area was performed with a G1321A (Agilent Tech 1100 series) detector at excitation and emission wavelengths of 317 nm and 542 nm, respectively, for NAD0241, and 315 nm and 565 nm for NAD0180. The peak areas were compared against those obtained after injection of several concentrations of pure standard derived from a stock solution of NAD0241 and NAD0180, respectively, in acetonitrile (HPLC grade) to determine extraction efficiencies, which were routinely 90-95%. The standard curve was obtained by plotting amounts of pure standard injected in µg vs. fluorescent peak area.

### Example 56F - Determination of brain/plasma ratios for prior art compounds K252a and NAD002 for comparison with NAD0241

Prior art compounds K252a and NAD002 and were assessed using identical conditions of application as used in example 56E to provide a direct comparison with NAD0241 for the ability to penetrate the blood-brain barrier. Volumes of injection for all compounds (including NAD0241) was fixed at 2.3mL/kg, in average about 500-600µL per animal. The other parameters were left unchanged.

Sampling of body fluids and brain tissue was performed as described in example 56E. Extraction of compounds from brain and plasma was performed as described in example 56E but no concentrated ammonia solution was added prior to extraction of K252a and NAD002.

HPLC analysis of K252a was performed on a 5 µm Merck RP 18 Select B 12.5x4.6 mm column at a flow rate of 0.75 mL/min with isocratic elution by acetonitrile/water 60/40 (v/v). Fluorescent excitation/emission was at 284 nm / 476nm. NAD002 was analyzed on a C 18 shield symmetry 25 x 4,6 mm (Waters and Owen) at 40°C using excitation/emission wavelengths of 315/565 nm. Quantitations were performed as under example 56E.

### Example 56G - Determination of solubility of the free base NAD0241 and of its hydrochloride salt in 99/1 water/DMSO mixtures.

Stock solutions of NAD0241 (100 mM) and of its hydrochloride salt (50 mM) in DMSO (UV-grade) were prepared freshly before the experiment and further diluted to a final concentration of 10 mM. These stock solutions in 100% DMSO were diluted with water (UV grade) to provide five 99/1 water/DMSO samples with varying concentrations of the compound (1000, 500, 100, 50 and 10 µM).

Each sample was thoroughly mixed by rigorous vortexing (30 seconds) followed by equilibration for 18 hours at room temperature. A 500 mL-aliquot for each sample was transferred into 1.5 mL polypropylene tubes and centrifuged for 30 minutes at room temperature at 16000 g in a fixed angle tabletop centrifuge (Heraeus Biofuge pico equipped with #3325 rotor). The supernatant was pipetted into a quartz glass cuvette and UV-absorption (322 nm) was measured in a UV spectrophotometer (Varian Cary 50). Each experiment was run in duplicate.

In the case of NAD-241 a value of 23 mgL⁻¹ was found as solubility limit in water containing 1% DMSO for both experiments. Its hydrochloride salt provided respectively a value of 170 mgL⁻¹ and 214 mgL⁻¹ for the two duplicate experiments.

## Claims

1. A compound of the general formula (I) 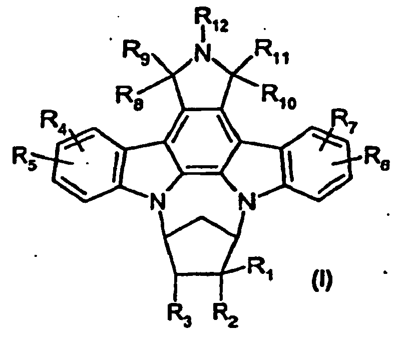 including diastereomeric and enantiomeric forms, mixtures of diastereomeric and enantiomeric forms, or pharmaceutically acceptable salt forms, wherein
R₁ is NR₁₃R₁₄ or may join together with R₂ to form an optionally substituted saturated or unsaturated N-heterocycle or may join together with R₃ to form an optionally substituted saturated or unsaturated N-heterocycle;
R₂ is selected from the group consisting of H, C₁-C₆ alkyl, aryl, heteroaryl, CN, COR₁₃, COOR₁₃, CONHR₁₃, and CONR₁₃R₁₄;
R₃ is selected from the group consisting of H, OR₁₃, OCOR₁₃, OCONHR₁₃, and OCONR₁₃R₁₄;
R₄,R₅,R₆,R₇ taken alone can be the same or different and are each independently selected from the group consisting of H, halogen, C₁-C₆ alkyl C₂-C₆ alkenyl, alkynyl, aryl or heteroaryl, CN, COR₁₃, COOR₁₃, CONHR₁₃, CONR₁₃R₁₄, CSR₁₃, CSSR₁₃, NR₁₃R₁₄, NHCOR₁₃, NHCOOR₁₃, NHSO₂R₁₃, N₃, OR₁₃, OCOR₁₃, SR₁₃, SO₂R₁₃, and SiR₁₅R₁₆R₁₇; wherein R₁₅, R₁₆ and R₁₇ can be the same or different and are independently selected from the group consisting of H, C₁-C₆ alkyl, aryl and heteroaryl;
R₈,R₉ when taken alone they are both H, or one of them is H and the other is OH, or when taken together they are the oxygen atom of a carbonyl group or the sulfur atom of a thiocarbonyl group; and with the proviso that when R₁₀,R₁₁ are different from carbonyl R₈,R₉ taken together are the oxygen atom of a carbonyl group or the sulfur atom of a thiocarbonyl group;
R₁₀,R₁₁ when taken alone they are both H, or one of them is H and the other is OH, or when taken together they are the oxygen atom of a carbonyl group or the sulfur atom of a thiocarbonyl group; and with the proviso that when R₈,R₉ are different from carbonyl R₁₀,R₁₁ taken together are the oxygen atom of a carbonyl group or the sulfur atom of a thiocarbonyl group;
R₁₂ is selected from the group consisting of H, C₁-C₆ alkyl, cycloalkyl, substituted benzyl, aryl, heteroaryl, COR₁₃, COOR₁₃, NR₁₃R₁₄, and OR₁₃,
and wherein
R₁₃ and R₁₄ can be the same or different and are independently selected from the group consisting of H, C₁-C₆ alkyl, cycloalkyl, optionally substituted acyl, aryl, optionally substituted benzyl and heteroaryl rest; or may join together to form N₃ or an optionally substituted saturated or unsaturated N-heterocyle ,

2. A compound according to claim 1 of the general formula (IA) 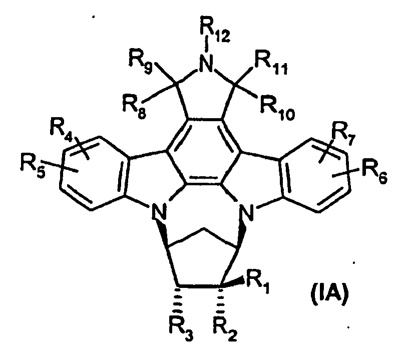 including diastereomeric and enantiomeric forms, mixtures of diastereomeric and enantiomeric forms, or pharmaceutically acceptable salt forms,
wherein R₁ to R₁₂ are as defined in claim 1.

3. A compound according to claim 1 or 2 including diastereomeric and enantiomeric forms, mixtures of diastereomeric and enantiomeric forms, or pharmaceutically acceptable salt forms,
wherein
R₁ is NR₁₃R₁₄;
R₂ is selected from the group consisting of H, CN, COOR₁₃, CONHR₁₃, and CONR₁₃R₁₄;
R₃ is selected from the group consisting of H and OH;
R₄,R₅,R₆,R₇ taken alone can be the same or different and are each independently selected from the group consisting of H, CONHR₁₃, CONR₁₃R₁₄, NR₁₃R₁₄, NHCOR₁₃, NHCOOR₁₃, NHSO₂R₁₃, and OR₁₃;
R₈,R₉ are both H, or one of them is H and the other is OH, or taken together they are the oxygen atom of a carbonyl group; and with the proviso that when R₁₀,R₁₁ are different from carbonyl R₈,R₉ taken together are the oxygen atom of a carbonyl group;
R₁₀,R₁₁ are both H, or one of them is H and the other is OH, or taken together they are the oxygen atom of a carbonyl group; and with the proviso that when R₈,R₉ are different from carbonyl R₁₀,R₁₁ taken together are the oxygen atom of a carbonyl group;
R₁₂ is selected from the group consisiting ofH, substituted C₁-C₆ alkyl, NR₁₃R₁₄, and OR₁₃,
and wherein
R₁₃ and R₁₄ can be the same or different and are independently selected from the group consisting of H and substituted C₁-C₆ alkyl.

4. A compound according to any one of claims 1 to 3 including diastereomeric and enantiomeric forms, mixtures of diastereomeric and enantiomeric forms, or pharmaceutically acceptable salt forms, wherein
R₁ is NHR₁₃, wherein R₁₃ is selected from the group consisting of H and substituted C₁-C₆ alkyl;
R₂ is selected from the group consisting of CN, COOR₁₃, and CONHR₁₃, wherein R₁₃ is selected from the group consisting ofH and substituted C₁-C₆ alkyl;
R₃ is selected from the group consisting ofH and OH;
R₄,R₅,R₆,R₇ taken alone can be the same or different and are each independently selected from the group consisting of H, NHR₁₃, and OR₁₃, wherein R₁₃ is selected from the group consisting ofH and substituted C₁-C₆ alkyl;
R₈,R₉ are both H, or taken together they are the oxygen atom of a carbonyl group; with the proviso that when R₁₀,R₁₁ are different from carbonyl R₈,R₉ taken together are the oxygen atom of a carbonyl group;
R₁₀,R₁₁ are both H, or taken together they are the oxygen atom of a carbonyl group; with the proviso that when R₈,R₉ are different from carbonyl R₁₀,R₁₁ taken together are the oxygen atom of a carbonyl group;
R₁₂ is H.

5. A compound according to any one of claims 1 to 4 of the general formula (IB) 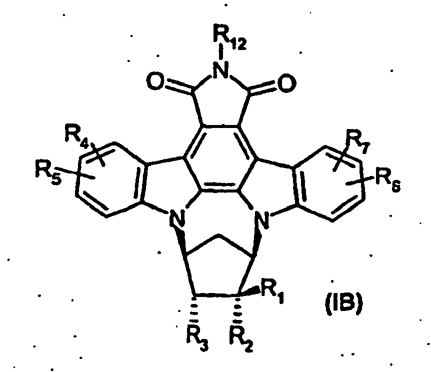 including diastereomeric and enantiomeric forms, mixtures of diastereomeric and enantiomeric forms, or pharmaceutically acceptable salt forms, wherein R₁ to R₇ and R₁₂ are as defined in any one of claims 1 to 4.

6. A compound according to any one of claims 1 to 5, wherein R₄,R₅,R₆,R₇ are all H.

7. Use of a compound according to any one of claims 1 to 6 for inhibiting the activity of one or more protein kinases.

8. The use according to claim 7, wherein the one or more protein kinases are selected from the group consisting of extracellular signal regulated kinase 2, protein kinase A, protein kinase C, and glycogen synthase kinase 3β.

9. A medicament comprising a compound according to any one of claims to 6.

10. Use of a compound according to any one of claims 1 to 6 for the preparation of a pharmaceutical composition for treating non-insulin dependent diabetes mellitus, acute stroke and other neurotraumatic injuries, for treating diabetes mellitus, as a chemotherapeutic for the treatment of various malignant diseases, for treating diseases caused by malfunctioning of specific signaling pathways, and for treating neurodegenerative diseases such as for example Alzheimer's disease.

## Patentansprüche

1. Verbindung der allgemeinen Formel (I) 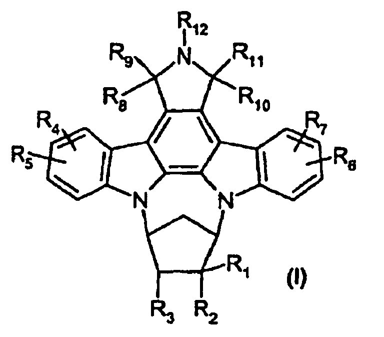 einschließlich diastereomerer und enantiomerer Formen, Gemischen von diastereomeren und enantiomeren Formen oder pharmazeutisch verträglicher Salzformen,
worin
R₁ eine NR₁₃R₁₄-Gruppe ist oder mit R₂ zusammen genommen werden kann, um einen gegebenenfalls substituierten gesättigten oder ungesättigten N-Heterozyklus zu bilden, oder mit R₃ zusammen genommen werden kann, um einen gegebenenfalls substituierten gesättigten oder ungesättigten N-Heterozyklus zu bilden,
R₂ ausgewählt ist aus der Gruppe bestehend aus H-Atom, C₁-C₆-Alkyl-, Aryl-, Heteroaryl-, CN-, COR₁₃-, COOR₁₃-, CONHR₁₃- und CONR₁₃R₁₄₋Gruppe,
R₃ ausgewählt ist aus der Gruppe bestehend aus H-Atom, OR₁₃-, OCOR₁₃-, OCONHR₁₃- und OCONR₁₃R₁₄-Gruppe,
R₄, R₅, R₆, R₇, wenn für sich genommen, gleich oder unterschiedlich sein können und jeweils unabhängig voneinander ausgewählt sind aus der Gruppe bestehend aus H-, Halogenatom, C₁-C₆-Alkyl-, C₂-C₆-Alkenyl-, C₂₋C₆-Alkinyl-, Aryl- oder Heteroaryl-, CN-, COR₁₃-, COOR₁₃-, CONHR₁₃-, CONR₁₃R₁₄-, CSR₁₃-, CSSR₁₃-, NR₁₃R₁₄-, NHCOR₁₃-, NHCOOR₁₃-, NHSO₂R₁₃-, N₃-, OR₁₃-, OCOR₁₃-, SR₁₃-, SO₂R₁₃- und SiR₁₅R₁₆R₁₇-Gruppe, worin R₁₅, R₁₆ und R₁₇ gleich oder unterschiedlich sein können und unabhängig voneinander ausgewählt sind aus der Gruppe bestehend aus H-Atom, C₁-C₆-Alkyl-, Aryl- und Heteroarylgruppe,
R₈, R₉, wenn für sich genommen, beide H-Atome sind oder eine dieser Gruppen ein H-Atom ist und die andere eine OH-Gruppe ist oder, wenn zusammen genommen, sie das Sauerstoffatom einer Carbonylgruppe oder das Schwefelatom einer Thiocarbonylgruppe sind, und mit der Maßgabe, dass, wenn R₁₀, R₁₁ keine Carbonylgruppe sind, R₈, R₉, wenn zusammen genommen, das Sauerstoffatom einer Carbonylgruppe oder das Schwefelatom einer Thiocarbonylgruppe sind,
R₁₀, R₁₁, wenn für sich genommen, beide H-Atome sind oder eine dieser Gruppen ein H-Atom ist und die andere eine OH-Gruppe ist oder, wenn zusammen genommen, sie das Sauerstoffatom einer Carbonylgruppe oder das Schwefelatom einer Thiocarbonylgruppe sind, und mit der Maßgabe, dass, wenn R₈, R₉ keine Carbonylgruppe sind, R₁₀, R₁₁, wenn zusammen genommen, das Sauerstoffatom einer Carbonylgruppe oder das Schwefelatom einer Thiocarbonylgruppe sind,
R₁₂ ausgewählt ist aus der Gruppe bestehend aus H-Atom, C₁-C₆-Alkyl-, Cycloalkyl-, substituierter Benzyl-, Aryl-, Heteroaryl-, COR₁₃-, COOR₁₃-, NR₁₃R₁₄- und OR₁₃-Gruppe,
und worin
R₁₃ und R₁₄ gleich oder unterschiedlich sein können und unabhängig voneinander ausgewählt sind aus der Gruppe bestehend aus H-Atom, C₁-C₆₋Alkyl-, Cycloalkyl-, gegebenenfalls substituierter Acyl-, Aryl-, gegebenenfalls substituierter Benzyl- und Heteroarylgruppe, oder zusammen genommen werden können, um eine N₃-Gruppe oder einen gegebenenfalls substituierten gesättigten oder ungesättigten N-Heterozyklus zu bilden.

2. Verbindung gemäß Anspruch 1 der allgemeinen Formel (IA) 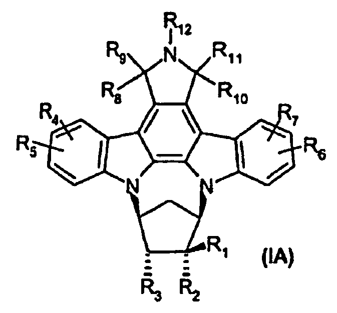 einschließlich diastereomerer und enantiomerer Formen, Gemischen von diastereomeren und enantiomeren Formen oder pharmazeutisch verträglicher Salzformen,
worin R₁ bis R₁₂ wie in Anspruch 1 definiert sind.

3. Verbindung gemäß Anspruch 1 oder 2, einschließlich diastereomerer und enantiomerer Formen, Gemischen von diastereomeren und enantiomeren Formen oder pharmazeutisch verträglicher Salzformen,
worin
R₁ eine NR₁₃R₁₄-Gruppe ist,
R₂ ausgewählt ist aus der Gruppe bestehend aus H-Atom, CN-, COOR₁₃-, CONHR₁₃- und CONR₁₃R₁₄-Gruppe,
R₃ ausgewählt ist aus der Gruppe bestehend aus H-Atom und OH-Gruppe,
R₄, R₅, R₆, R₇, wenn für sich genommen, gleich oder unterschiedlich sein können und jeweils unabhängig voneinander ausgewählt sind aus der Gruppe bestehend aus H-Atom, CONHR₁₃-, CONR₁₃R₁₄- NR₁₃R₁₄-, NHCOR₁₃-, NHCOOR₁₃-, NHSO₂R₁₃- und OR₁₃-Gruppe,
R₈, R₉ beide H-Atome sind oder eine dieser Gruppen ein H-Atom ist und die andere eine OH-Gruppe ist oder, wenn zusammen genommen, sie das Sauerstoffatom einer Carbonylgruppe sind, und mit der Maßgabe, dass, wenn R₁₀, R₁₁ keine Carbonylgruppe sind, R₈, R₉, wenn zusammen genommen, das Sauerstoffatom einer Carbonylgruppe sind,
Rio, R₁₁ beide H-Atome sind oder eine dieser Gruppen ein H-Atom ist und die andere eine OH-Gruppe ist oder, wenn zusammen genommen, sie das Sauerstoffatom einer Carbonylgruppe sind, und mit der Maßgabe, dass, wenn R₈, R₉ keine Carbonylgruppe sind, R₁₀, R₁₁, wenn zusammen genommen, das Sauerstoffatom einer Carbonylgruppe sind,
R₁₂ ausgewählt ist aus der Gruppe bestehend aus H-Atom, substituierter C₁-C₆-Alkyl-, NR₁₃R₁₄- und OR₁₃-Gruppe,
und worin
R₁₃ und R₁₄ gleich oder unterschiedlich sein können und unabhängig voneinander ausgewählt sind aus der Gruppe bestehend aus H-Atom und substituierter C₁-C₆-Alkylgruppe.

4. Verbindung gemäß einem der Ansprüche 1 bis 3, einschließlich diastereomerer und enantiomerer Formen, Gemischen von diastereomeren und enantiomeren Formen oder pharmazeutisch verträglicher Salzformen,
worin
R₁ eine NHR₁₃-Gruppe ist, worin R₁₃ ausgewählt ist aus der Gruppe bestehend aus H-Atom und substituierter C₁-C₆-Alkylgruppe,
R₂ ausgewählt ist aus der Gruppe bestehend aus CN-, COOR₁₃- und CONHR₁₃-Gruppe, worin R₁₃ ausgewählt ist aus der Gruppe bestehend aus H-Atom und substituierter C₁-C₆-Alkylgruppe,
R₃ ausgewählt ist aus der Gruppe bestehend aus H-Atom und OH-Gruppe,
R₄, R₅, R₆, R₇, wenn für sich genommen, gleich oder unterschiedlich sein können und jeweils unabhängig voneinander ausgewählt sind aus der Gruppe bestehend aus H-Atom, NHR₁₃- und OR₁₃-Gruppe, worin R₁₃ ausgewählt ist aus der Gruppe bestehend aus H-Atom und substituierter C₁₋C₆-Alkylgruppe,
R₈, R₉ beide H-Atome sind oder, wenn zusammen genommen, sie das Sauerstoffatom einer Carbonylgruppe sind, mit der Maßgabe, dass, wenn R₁₀, R₁₁ keine Carbonylgruppe sind, R₈, R₉, wenn zusammen genommen, das Sauerstoffatom einer Carbonylgruppe sind,
R₁₀, R₁₁ beide H-Atome sind oder, wenn zusammen genommen, sie das Sauerstoffatom einer Carbonylgruppe sind, mit der Maßgabe, dass, wenn R₈, R₉ keine Carbonylgruppe sind, R₁₀, R₁₁, wenn zusammen genommen, das Sauerstoffatom einer Carbonylgruppe sind,
R₁₂ ein H-Atom ist.

5. Verbindung gemäß einem der Ansprüche 1 bis 4 der allgemeinen Formel (IB) 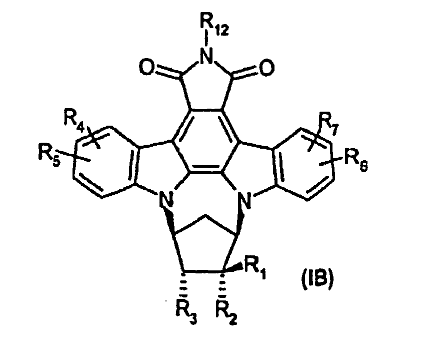 einschließlich diastereomerer und enantiomerer Formen, Gemischen von diastereomeren und enantiomeren Formen oder pharmazeutischer verträglicher Salzformen,
worin R₁ bis R₇ und R₁₂ wie in einem der Ansprüche 1 bis 4 definiert sind.

6. Verbindung gemäß einem der Ansprüche 1 bis 5, worin R₄, R₅, R₆, R₇ alle H-Atome sind.

7. Verwendung einer Verbindung gemäß einem der Ansprüche 1 bis 6 zum Hemmen der Aktivität einer oder mehrerer Proteinkinasen.

8. Verwendung gemäß Anspruch 7, wobei die eine oder mehreren Proteinkinasen ausgewählt sind aus der Gruppe bestehend aus extrazelluläres Signal-regulierter Kinase 2, Proteinkinase A, Proteinkinase C und Glykogensynthasekinase 3β.

9. Medikament, das eine Verbindung gemäß einem der Ansprüche 1 bis 6 umfasst.

10. Verwendung einer Verbindung gemäß einem der Ansprüche 1 bis 6 zur Herstellung einer pharmazeutischen Zusammensetzung zum Behandeln von nicht insulinabhängigem Diabetes mellitus, akutem Schlaganfall und anderen neurotraumatischen Verletzungen, zum Behandeln von Diabetes mellitus, als ein Chemotherapeutikum zur Behandlung von verschiedenen malignen Erkrankungen, zum Behandeln von Erkrankungen, die durch eine Störung spezifischer Signalwege verursacht werden, und zum Behandeln neurodegenerativer Erkrankungen wie Alzheimer-Krankheit.

## Revendications

1. Composé de formule générale (I) 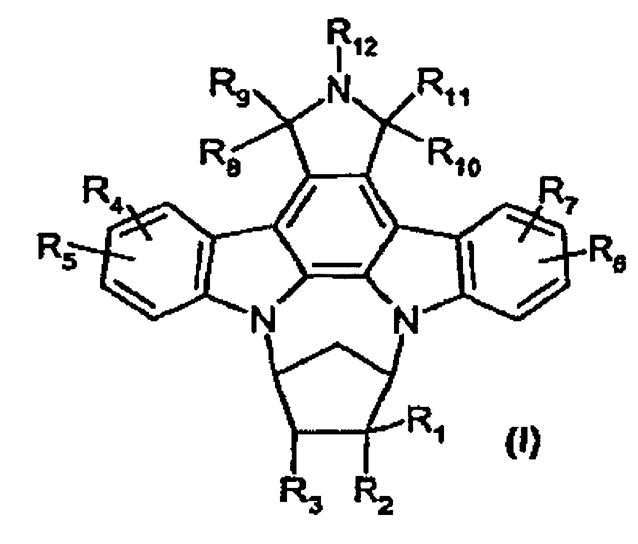 y compris les formes diastéréo-isomères et énantiomères, les mélanges de formes diastéréo-isomères et énantiomères, ou les formes sel pharmaceutiquement acceptable,
où
R₁ est NR₁₃R₁₄ ou peut être relié à R₂ pour former un N-hétérocycle saturé ou insaturé, en option substitué, ou peut être relié à R₃ pour former un N-hétérocycle saturé ou insaturé, en option substitué;
R₂ est choisi dans le groupe consistant en H, alkyle en C₁-C₆, aryle, hétéroaryle, CN, COR₁₃, COOR₁₃, CONHR₁₃ et CONR₁₃R₁₄;
R₃ est choisi dans le groupe consistant en H, OR₁₃, OCOR₁₃, OCONHR₁₃ et OCONR₁₃R₁₄;
R₄, R₅, R₆, R₇ pris individuellement peuvent être identiques ou différents et sont chacun indépendamment choisis dans le groupe consistant en H, halogéno, alkyle en C₁-C₆, alcényle en C₂-C₆, alcynyle en C₂-C₆, aryle ou hétéroaryle, CN, COR₁₃, COOR₁₃, CONHR₁₃, CONR₁₃R₁₄, CSR₁₃, CSSR₁₃, NR₁₃R₁₄, NHCOR₁₃, NHCOOR₁₃, NHSO₂R₁₃, N₃, OR₁₃, OCOR₁₃, SR₁₃, SO₂R₁₃ et SiR₁₅R₁₆R₁₇; tandis que R₁₅, R16 et R₁₇ peuvent être identiques ou différents et sont indépendamment choisis dans le groupe consistant en H, alkyle en C₁-C₆, aryle et hétéroaryle;
R₈, R₉ lorsqu'ils sont pris individuellement sont tous deux H, ou l'un d'eux est H et l'autre est OH, ou lorsqu'ils sont considérés ensemble ils sont l'atome d'oxygène d'un groupe carbonyle ou l'atome de soufre d'un groupe thiocarbonyle; et avec pour condition que quand R₁₀, R₁₁ sont différents d'un carbonyle R₈, R₉ pris ensemble sont l'atome d'oxygène d'un groupe carbonyle ou l'atome de soufre d'un groupe thiocarbonyle;
R₁₀, R₁₁ lorsqu'ils sont pris individuellement sont tous deux H, ou l'un d'eux est H et l'autre est OH, ou lorsqu'ils sont considérés ensemble ils sont l'atome d'oxygène d'un groupe carbonyle ou l'atome de soufre d'un groupe thiocarbonyle; et avec pour condition que quand R₈, R₉ sont différents d'un carbonyle R₁₀, R₁₁ pris ensemble sont l'atome d'oxygène d'un groupe carbonyle ou l'atome de soufre d'un groupe thiocarbonyle;
R₁₂ est choisi dans le groupe consistant en H, un alkyle en C₁-C₆, un cycloalkyle, un benzyle substitué, un aryle, un hétéroaryle, COR₁₃, COOR₁₃, NR₁₃R₁₄ et OR13,
et tandis que
R₁₃ et R₁₄ peuvent être identiques ou différents et sont indépendamment choisis dans le groupe consistant en les restes H, alkyle en C₁-C₆, cycloalkyle, acyle substitué en option, aryle, benzyle substitué en option et hétéroaryle; ou peuvent être reliés pour former N3 ou un N-hétérocycle saturé ou insaturé, en option substitué.

2. Composé selon la revendication 1 de formule générale (IA) 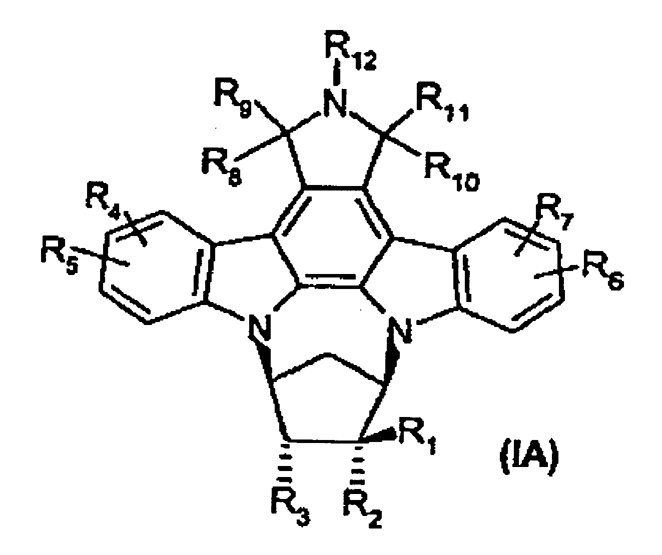 y compris les formes diastéréo-isomères et énantiomères, les mélanges de formes diastéréo-isomères et énantiomères, ou les formes sel pharmaceutiquement acceptable,
tandis que R₁ à R₁₂ sont tels que définis selon la revendication 1.

3. Composé selon la revendication 1 ou 2, y compris les formes diastéréo-isomères et énantiomères, les mélanges de formes diastéréo-isomères et énantiomères, ou les formes sel pharmaceutiquement acceptable,
où
R₁ est NR₁₃R₁₄;
R₂ est choisi dans le groupe consistant en H, CN, COOR₁₃, CONHR₁₃ et CONR₁₃R₁₄;
R₃ est choisi dans le groupe consistant en H et OH;
R₄, R₅, R₆, R₇ pris individuellement peuvent être identiques ou différents et sont chacun indépendamment choisis dans le groupe consistant en H, CONHR₁₃, CONR₁₃R₁₄, NR₁₃R₁₄, NHCOR₁₃, NHCOOR₁₃, NHSO₂R₁₃ et OR₁₃;
R₈, R₉ sont tous deux H, ou l'un d'eux est H et l'autre est OH, ou lorsqu'ils sont considérés ensemble ils sont l'atome d'oxygène d'un groupe carbonyle; et avec pour condition que quand R₁₀, R₁₁ sont différents d'un carbonyle R₈, R₉ pris ensemble sont l'atome d'oxygène d'un groupe carbonyle;
R₁₀, R₁₁ sont tous deux H, ou l'un d'eux est H et l'autre est OH, ou lorsqu'ils sont considérés ensemble ils sont l'atome d'oxygène d'un groupe carbonyle; et avec pour condition que quand R₈, R₉ sont différents d'un carbonyle R₁₀, R₁₁ pris ensemble sont l'atome d'oxygène d'un groupe carbonyle;
R₁₂ est choisi dans le groupe consistant en H, un alkyle en C₁-C₆ substitué, NR₁₃R₁₄ et OR₁₃,
et tandis que
R₁₃ et R₁₄ peuvent être identiques ou différents et sont indépendamment choisis dans le groupe consistant en H et alkyle en C₁-C₆ substitué.

4. Composé selon l'une quelconque des revendications 1 à 3, y compris les formes diastéréo-isomères et énantiomères, les mélanges de formes diastéréo-isomères et énantiomères, ou les formes sel pharmaceutiquement acceptable,
tandis que
R₁ est NHR₁₃, où R₁₃ est choisi dans le groupe consistant en H et un alkyle en C₁-C₆ substitué;
R₂ est choisi dans le groupe consistant en CN, COOR₁₃ et CONHR₁₃, tandis que R₁₃ est choisi dans le groupe consistant en H et alkyle en C₁-C₆ substitué;
R₃ est choisi dans le groupe consistant en H et OH;
R₄, R₅, R₆, R₇ pris individuellement peuvent être identiques ou différents et sont chacun indépendamment choisis dans le groupe consistant en H, NHR₁₃ et OR₁₃, tandis que R₁₃ est choisi dans le groupe consistant en H et alkyle en C₁-C₆ substitué;
R₈, R₉ sont tous deux H, ou considérés ensemble ils sont l'atome d'oxygène d'un groupe carbonyle; avec pour condition que quand R₁₀, R₁₁ sont différents d'un carbonyle R₈, R₉ pris ensemble sont l'atome d'oxygène d'un groupe carbonyle;
R₁₀, R₁₁ sont tous deux H, ou considérés ensemble ils sont l'atome d'oxygène d'un groupe carbonyle; avec pour condition que quand R₈, R₉ sont différents d'un carbonyle R₁₀, R₁₁ pris ensemble sont l'atome d'oxygène d'un groupe carbonyle;
R₁₂ est H.

5. Composé selon l'une quelconque des revendications 1 à 4 de formule générale (IB) 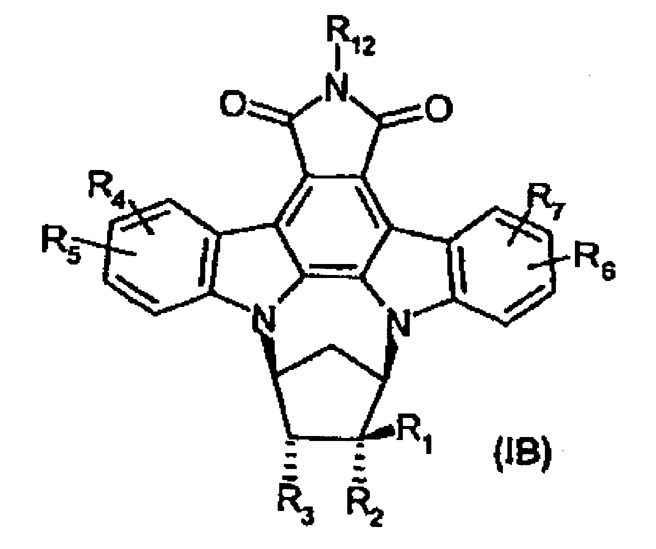 y compris les formes diastéréo-isomères et énantiomères, les mélanges de formes diastéréo-isomères et énantiomères, ou les formes sel pharmaceutiquement acceptable,
où R₁ à R₇ et R₁₂ sont tels que définis selon l'une quelconque des revendications 1 à 4.

6. Composé selon l'une quelconque des revendications 1 à 5, dans lequel R₄, R₅, R₆, R₇ sont tous H.

7. Utilisation d'un composé selon l'une quelconque des revendications 1 à 6 pour l'inhibition de l'activité d'une ou plusieurs protéine kinases.

8. Utilisation selon la revendication 7, dans laquelle lesdites une ou plusieurs protéine kinases sont choisies dans le groupe consistant en kinase 2 régulée à signal extracellulaire, protéine kinase A, protéine kinase C et glycogène synthase kinase 3β.

9. Médicament comprenant un composé selon l'une quelconque des revendications 1 à 6.

10. Utilisation d'un composé selon l'une quelconque des revendications 1 à 6 pour la préparation d'une composition pharmaceutique pour le traitement du diabète sucré non-insulinodépendant, d'un ictus aigu et d'autres atteintes neurotraumatiques, pour le traitement du diabète sucré, comme agent chimiothérapeutique pour le traitement de diverses affections malignes, pour le traitement d'affections provoquées par le mauvais fonctionnement de voies de signalisation spécifiques, et pour le traitement de maladies neurodégénératives telles que par exemple la maladie d'Alzheimer.
